# EUROPEAN PATENT APPLICATION

(11) **EP 3 683 304 A1**
(43) Date of publication of application: **22.07.2020**
(21) Application number: 18855704.5
(22) Date of filing: 14.09.2018
(51) Int. Cl.: C12N 5/071, A61K 35/30, A61L 27/38, A61P 27/02

(54) **METHOD FOR AMPLIFYING CONE PHOTORECEPTORS OR ROD PHOTORECEPTORS USING DORSALIZATION SIGNAL TRANSMITTER OR VENTRALIZATION SIGNAL TRANSMITTER**

(30) Priority: 14.09.2017 JP 2017177187
(71) Applicant: RIKEN, Wako-shi, Saitama 351-0198 (JP); Sumitomo Dainippon Pharma Co., Ltd., Osaka-shi Osaka 541-8524 (JP); SUMITOMO CHEMICAL COMPANY, LIMITED, Chuo-ku, Tokyo 104-8260 (JP)
(72) Inventor: NUKAYA, Daiki, Tokyo 104-8356 (JP); EIRAKU, Mototsugu, Wako-shi Saitama 351-0198 (JP); KINOSE, Yuiko, Wako-shi Saitama 351-0198 (JP); ONISHI, Akishi, Wako-shi Saitama 351-0198 (JP); TAKAHASHI, Masayo, Wako-shi Saitama 351-0198 (JP)
(74) Representative: J A Kemp LLP
(86) International application number: PCT/JP2018/034315
(87) International publication number: WO 2019/054515

(57) **Abstract**

The present invention aims to provide a retinal tissue rich in cone photoreceptor precursors and/or cone photoreceptors, a retinal tissue rich in rod photoreceptor precursors and/or rod photoreceptors, and a production method thereof and the like. i) A method for increasing a proportion of a cone photoreceptor precursor and a cone photoreceptor in a photoreceptor precursor and a photoreceptor contained in a retinal tissue, including a step of culturing a retinal tissue, in an initial developmental stage to a stage where an emergence rate of a cone photoreceptor precursor reaches maximum, in a medium containing a dorsalization signal transmitter at a concentration sufficient to suppress expression of a ventral marker, or ii) a method for increasing a proportion of a rod photoreceptor precursor and a rod photoreceptor in a photoreceptor precursor and a photoreceptor contained in a retinal tissue, including a step of culturing a retinal tissue, in an initial developmental stage to a stage where an emergence rate of a cone photoreceptor precursor reaches maximum, in a medium containing a ventralization signal transmitter at a concentration sufficient to promote expression of a ventral marker.

## Description

### [Technical Field]

The present invention relates to a retinal tissue rich in cone photoreceptors or rod photoreceptors, and a production method thereof.

### [Background Art]

While the retinal tissue region in all of mouse, rat, human and chicken is said to be determined by dorsalization and ventralization signals, the structure thereof is unique depending on each animal species.

For example, the retina of human and chicken has, unlike that of mouse and rat, a region called macula that has a high percentage of cone photoreceptors (Cone) in the fundus (or central retina), and only cone photoreceptors are present in the center (Rod-Free Zone) of the region. Especially in the macula of human retinal tissue, the content of LM cone photoreceptors becomes higher toward the center, and conversely, the content of S cone photoreceptors increases toward the periphery. Outside the macula, there is a region containing S cone photoreceptors and rod photoreceptors, and the rod photoreceptors densely gather at the outer edge of the macula.

As regards the retinal tissue of chicken, it is known that Rod-free zone disappears by excision of the entire dorsalized region of the retinal tissue in the optic vesicle stage of chicken embryo and that Rod-free zone disappears by overexpression of ventralization signals. Thus, it is suggested that the Rod-free zone is formed by the contribution of the dorsalized region (non-patent document 1). On the other hand, there are known some culture methods for inducing differentiation of pluripotency stem cells such as ES cell and the like into a retinal tissue (patent documents 1 - 5).

However, how the dorsalized region contributes to the formation of a Rod-free zone in the retinal tissue of human and chicken has not been known. Particularly, a method for producing a retinal tissue rich in cone photoreceptors (LM cone photoreceptors and/or S cone photoreceptors) or rod photoreceptors has not been known at all.

### [Document List]

### [Patent documents]

patent document 1: WO 2009/148170
patent document 2: WO 2011/055855
patent document 3: WO 2013/077425
patent document 4: WO 2013/065763
patent document 5: WO 2015/025967

### [non-patent document]

non-patent document 1: J Neurosci. 25(11):2823-2831 (2005)

### [SUMMARY OF THE INVENTION]

### [Problems to be Solved by the Invention]

The problem to be solved by the present invention is to provide a retinal tissue rich in cone photoreceptors or rod photoreceptors, and a production method thereof.

### [Means of Solving the Problems]

The present inventors have conducted intensive studies in an attempt to solve the above-mentioned problem and found that the proportion of a cone photoreceptor contained in photoreceptors can be increased by culturing and maturing a retinal tissue containing a retinal progenitor cell or a neural retinal progenitor cell and in a differentiation stage without emergence of a ganglion cell, namely, a retinal tissue in an initial developmental stage (Initial Developmental Stage), in the presence of a dorsalization signal transmitter at a concentration sufficient to suppress the expression of ventral markers, as compared to culturing in the absence of the aforementioned dorsalization signal transmitter.

In addition, they have found that the proportion of a rod photoreceptor contained in photoreceptors can be increased by culturing and maturing a retinal tissue in an initial developmental stage in the presence of a ventralization signal transmitter, as compared to culturing in the absence of the aforementioned ventralization signal transmitter, which resulted in the completion of the present invention.

That is, the present invention relates to the following:
[1] A method for increasing a proportion of a cone photoreceptor precursor and a cone photoreceptor in a photoreceptor precursor and a photoreceptor comprised in a retinal tissue, the method comprising a step of culturing a retinal tissue in an initial developmental stage to a stage where an emergence rate of a cone photoreceptor precursor reaches maximum in a medium comprising a dorsalization signal transmitter at a concentration sufficient to suppress expression of a ventral marker;
[2] the method of the above-mentioned [1], wherein the cone photoreceptor precursor and the cone photoreceptor are CRX-positive and RXR-γ-positive, or CRX-positive and TRβ2-positive; and NRL-negative cells;
[3] the method of the above-mentioned [1] or [2], wherein the ventral marker is ALDH1A3 and/or COUP-TF I;
[4] the method of any of the above-mentioned [1] to [3], wherein the concentration of the dorsalization signal transmitter is such that it does not induce expression of a most dorsal marker;
[5] the method of any of the above-mentioned [1] to [3], wherein the concentration of the dorsalization signal transmitter is such that it promotes expression of the dorsal marker;
[6] the method of any of the above-mentioned [1] to [3], wherein the concentration of the dorsalization signal transmitter is such that it does not induce expression of the most dorsal marker and promotes expression of other dorsal markers;
[7] the method of the above-mentioned [5] or [6], wherein the dorsal marker is CYP26A1 and/or CYP26C1;
[8] the method of the above-mentioned [4] or [6], wherein the most dorsal marker is COUP-TF II;
[9] the method of the above-mentioned [5] or [6], wherein the dorsal marker is ALDH1A1;
[10] the method of the above-mentioned [9], wherein the concentration of the dorsalization signal transmitter is sufficient to induce expression of not less than 0.1% and not more than 30% of the expression level of ALDH1A1 promoted by 1.35 nM BMP4;
[11] the method of any of the above-mentioned [1] to [10], wherein the retinal tissue in an initial developmental stage comprises a ciliary marginal zone-like structure;
[12] the method of any of the above-mentioned [1] to [10], wherein the retinal tissue in an initial developmental stage comprises a cell that can differentiate into a photoreceptor and a ganglion cell;
[13] the method of any of the above-mentioned [1] to [12], wherein the retinal tissue in an initial developmental stage is derived from a pluripotent stem cell;
[14] the method of any of the above-mentioned [1] to [12], wherein the retinal tissue in an initial developmental stage is derived from a neuroepithelial cell obtained from an adult tissue;
[15] the method of any of the above-mentioned [1] to [14], wherein the retinal tissue in an initial developmental stage comprises a PAX6-positive and RX-positive cell;
[16] the method of any of the above-mentioned [1] to [15], wherein the retinal tissue in an initial developmental stage comprises a PAX6-positive, RX-positive and CHX10-positive cell;
[17] the method of any of the above-mentioned [1] to [16], wherein the step of culturing in the presence of a dorsalization signal transmitter is continued for 4 days to 170 days;
[18] the method of the above-mentioned [17], wherein the step of culturing in the presence of a dorsalization signal transmitter is continued until a period when a rod photoreceptor precursor emerges when cultured in the absence of a dorsalization signal transmitter;
[19] the method of any of the above-mentioned [1] to [18], wherein the dorsalization signal transmitter is a BMP signal transduction pathway agonist or a Wnt signal transduction pathway agonist, or a SHH signal transduction pathway inhibitor which is capable of inducing a BMP signal corresponding to 0.01 nM - 0.90 nM of BMP4;
[20] the method of any of the above-mentioned [1] to [19], wherein the dorsalization signal transmitter is BMP4;
[21] the method of the above-mentioned [20], wherein the concentration of BMP4 is 0.05 nM - 0.45 nM;
[22] the method of any of the above-mentioned [1] to [19], wherein the dorsalization signal transmitter is Cyclopamine-KAAD;
[23] the method of the above-mentioned [22], wherein the concentration of Cyclopamine-KAAD is 0.01 µM - 5 µM;
[24] the method of the above-mentioned [23], wherein the concentration of Cyclopamine-KAAD is 0.2 µM - 1 µM;
[25] the method of any of the above-mentioned [1] to [24], wherein the method is performed in a medium free of 9-cisretinoic acid;
[26] a retinal tissue comprising a photoreceptor precursor rich in a cone photoreceptor precursor and/or a photoreceptor rich in a cone photoreceptor, wherein the retinal tissue is obtained by the method of any of the above-mentioned [1] to [25];
[27] a retinal tissue comprising a photoreceptor precursor rich in a cone photoreceptor precursor and/or a photoreceptor rich in a cone photoreceptor, and a ganglion cell, wherein the number of the cone photoreceptor precursor and cone photoreceptor is not less than 2 times, preferably not less than 4 times, the number of the rod photoreceptor precursor and rod photoreceptor, in the photoreceptor precursor and photoreceptor;
[28] the retinal tissue of the above-mentioned [26] or [27], wherein the whole photoreceptor precursor and the whole photoreceptor comprises the cone photoreceptor precursor and cone photoreceptor in not less than 70%, preferably not less than 80%;
[29] a retinal tissue that is able to mature into the retinal tissue of the above-mentioned [26] or [27] by culturing;
[30] the retinal tissue of any of the above-mentioned [26] to [29], wherein not less than 50% of the layer structure of the retinal tissue forms a continuous epithelial structure;
[31] the retinal tissue of the above-mentioned [30], wherein the retinal tissue has a diameter in the major axis direction of not less than 0.6 mm;
[32] a pharmaceutical composition for transplantation to a retinal tissue of a retina disease patient in need of transplantation, comprising a retinal tissue section cut out from the retinal tissue of any of the above-mentioned [26] to [31];
[33] the pharmaceutical composition of the above-mentioned [32], wherein the retinal tissue requiring transplantation is a tissue of a region comprising Rod-free zone;
[34] the pharmaceutical composition of the above-mentioned [33], wherein the region comprising the Rod-free zone has a macular-like structure;
[35] A method for increasing a proportion of a rod photoreceptor precursor and a rod photoreceptor in a photoreceptor precursor and a photoreceptor comprised in a retinal tissue, comprising a step of culturing a retinal tissue, in an initial developmental stage to a stage where an emergence rate of a cone photoreceptor precursor reaches maximum, for at least one day in the presence of a ventralization signal transmitter at a concentration sufficient to promote expression of a ventral marker;
[36] the method of the above-mentioned [35], wherein the rod photoreceptor precursor and rod photoreceptor are NRL-positive and CRX-positive cells;
[37] the method of the above-mentioned [35] or [36], wherein the ventral marker is ALDH1A3 and/or COUP-TF I;
[38] the method of any of the above-mentioned [35] to [37], wherein the retinal tissue in an initial developmental stage comprises a ciliary marginal zone-like structure;
[39] the method of any of the above-mentioned [35] to [38], wherein the retinal tissue in an initial developmental stage comprises a cell that can differentiate into a photoreceptor or a ganglion cell;
[40] the method of any of the above-mentioned [35] to [39], wherein the retinal tissue in an initial developmental stage is derived from a pluripotent stem cell;
[41] the method of any of the above-mentioned [35] to [39], wherein the retinal tissue in an initial developmental stage is derived from a neuroepithelial cell obtained from an adult tissue;
[42] the method of any of the above-mentioned [35] to [41], wherein the retinal tissue in an initial developmental stage comprises a PAX6-positive and RX-positive cell;
[43] the method of the above-mentioned [42], wherein the retinal tissue in an initial developmental stage comprises a PAX6-positive, RX-positive and CHX10-positive cell;
[44] the method of any of the above-mentioned [35] to [43], wherein the step of culturing in the presence of a ventralization signal transmitter is continued for 4 days to 170 days;
[45] the method of the above-mentioned [44], wherein the step of culturing in the presence of a ventralization signal transmitter is continued until a period when a rod photoreceptor precursor emerges;
[46] the method of any of the above-mentioned [35] to [45], wherein the ventralization signal transmitter is a substance having an SHH signal transduction pathway promoting activity corresponding to 1 nM - 10 µM SAG, or a substance having a BMP signal transduction pathway inhibitory activity corresponding to 0.1 nM - 20 µM LDN193189;
[47] the method of the above-mentioned [46], wherein the ventralization signal transmitter is SAG;
[48] the method of the above-mentioned [47], wherein the concentration of SAG is 1 nM - 10 µM;
[49] the method of the above-mentioned [48], wherein the concentration of SAG is 10 nM - 500 nM;
[50] the method of the above-mentioned [46], wherein the ventralization signal transmitter is LDN193189;
[51] the method of the above-mentioned [50], wherein the concentration of LDN193189 is 0.1 nM - 20 µM;
[52] the method of the above-mentioned [51], wherein the concentration of LDN193189 is 30 nM - 1 µM;
[53] the method of any of the above-mentioned [35] to [52], wherein the method is performed in a medium free of 9-cisretinoic acid;
[54] a retinal tissue comprising a photoreceptor precursor rich in a rod photoreceptor precursor and/or a photoreceptor rich in a rod photoreceptor, wherein the retinal tissue is obtained by the method of any of the above-mentioned [35] to [53];
[55] a retinal tissue comprising a photoreceptor precursor rich in a rod photoreceptor precursor and/or a photoreceptor rich in a rod photoreceptor, and a ganglion cell, wherein not less than 40%, preferably not less than 55%, of the number of the cells of the photoreceptor precursor and photoreceptor are rod photoreceptor precursors and rod photoreceptors;
[56] a retinal tissue that is able to mature into the retinal tissue of the above-mentioned [55] by culturing;
[57] the retinal tissue of any of the above-mentioned [54] to [56], wherein not less than 50% of the layer structure of the retinal tissue forms a continuous epithelial structure;
[58] the retinal tissue of the above-mentioned [57], wherein the retinal tissue has a diameter in the major axis direction of not less than 0.6 mm;
[59] a pharmaceutical composition for transplantation to a retinal tissue of a retina disease patient in need of transplantation, comprising a retinal tissue section cut out from the retinal tissue of any of the above-mentioned [54] to [58] ;
[60] the pharmaceutical composition of the above-mentioned [59], wherein the retinal tissue requiring transplantation is a region including the periphery of the macula and the outside thereof having a high proportion of rod photoreceptor precursor (Rod precursor) and/or rod photoreceptor;
[61] a method for treating a disease based on a disorder of a retinal cell or retinal tissue, comprising transplanting an effective amount of the retinal tissue of any of the above-mentioned [26] to [31], or any of the above-mentioned [54] to [58] to a subject in need of transplantation;
[62] use of the retinal tissue of any of the above-mentioned [26] to [31], or any of the above-mentioned [54] to [58] as a reagent for evaluating toxicity or efficacy;
[63] a method for producing a completely matured retinal tissue that expresses S-opsin, L-opsin and/or M-opsin, comprising a step of culturing a retinal tissue comprising a cone photoreceptor precursor and a cone photoreceptor in a serum-free medium;
[64] the method of the above-mentioned [63], wherein the retinal tissue comprising the cone photoreceptor precursor and cone photoreceptor is the retinal tissue of any of the above-mentioned [26] to [31];
[65] the method of the above-mentioned [63] or [64], wherein the serum-free medium is a medium comprising a dorsalization signal transmitter;
[66] the method of the above-mentioned [65], wherein the dorsalization signal transmitter is BMP;
[67] the method of any of the above-mentioned [63] to [66], wherein the serum-free medium further comprises a thyroid gland hormone signal transmitter.

### [Effect of the Invention]

According to the present invention, it is possible to produce a retinal tissue rich in cone photoreceptors and provide same as a retinal tissue for transplantation to the macula of the eye of patients with age-related macular degeneration and the like, and to produce a retinal tissue rich in rod photoreceptors and provide same as a retinal tissue for transplantation to the periphery of the macula or the outside thereof of the eye of patients with age-related macular degeneration and the like.

### [Brief Description of the Drawings]

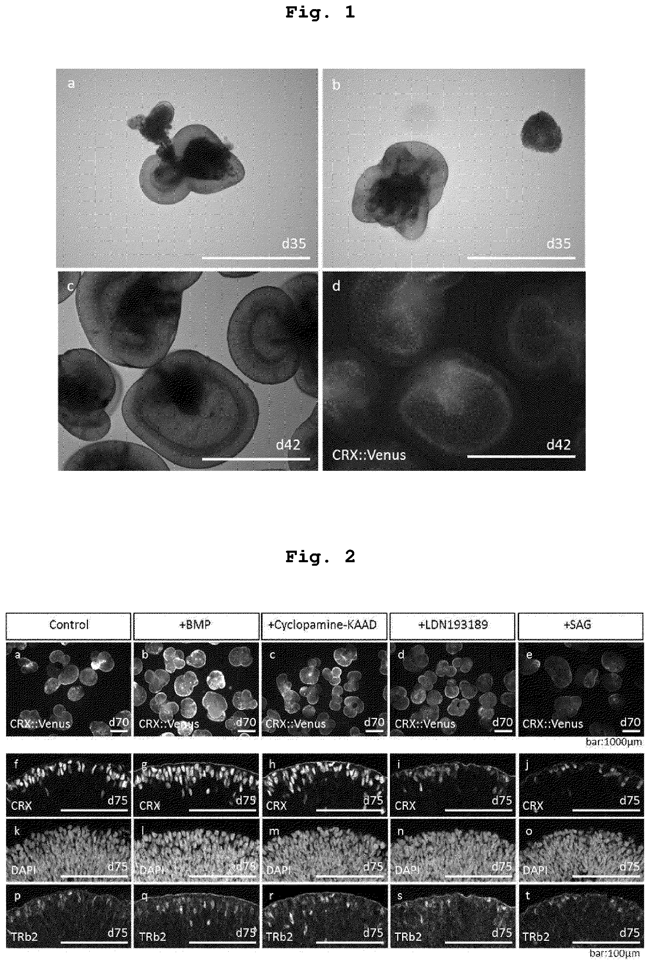

Fig. 1 shows images of cell aggregates containing a retinal tissue produced from human ES cells which were taken by a fluorescence stereo microscope on day 35 (a, b) and day 42 (c, d) from the start of suspension culture. Therein, a and b are images obtained by excising cell aggregates containing retinal tissue with tweezers on day 35 from the start of suspension culture and photographed with a fluorescence stereo microscope, and c and d are images confirming that cells with fluorescence of CRX::Venus protein, that is, photoreceptor precursors, emerged in cell aggregates containing retinal tissue on day 42 from the start of suspension culture.
Fig. 2 shows images (a - e) of cell aggregates containing a retinal tissue produced from human ES cells which were taken by a fluorescence stereo microscope after culturing up to day 70 from the start of suspension culture, and shows images (f - t) obtained by preparing a section of a cell aggregate containing retinal tissue cultured up to day 75 and collected, and performing immunostaining using an anti-CRX antibody, an anti-TRβ2 antibody, and DAPI. Compared to the non-treatment group (Control), it is clear that, when a dorsalization signal transmitter was added (+BMP, +Cyclopamine-KAAD), CRX::Venus-positive cell, CRX-positive cell and TRβ2-positive cell increased, and conversely, it decreased when a ventralization signal transmitter was added (+LDN193189, +SAG). CRX::Venus-positive cell, CRX-positive cell, and tRβ2-positive cell that emerge at this differentiation stage are all photoreceptor precursors or cone photoreceptor precursors.

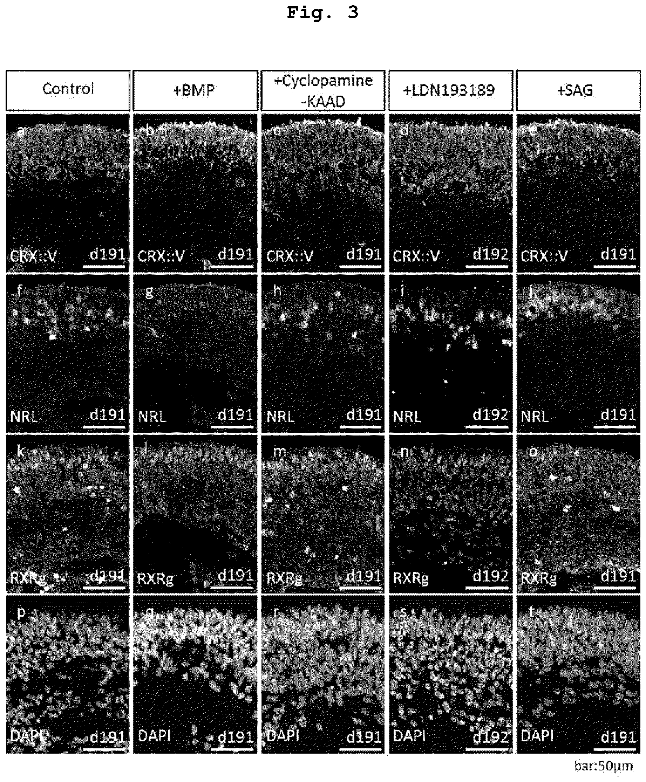

Fig. 3 shows identification (a - t) of RXR-γ-positive and NRL-negative cell (cone photoreceptor precursor) in GFP-positive cell, i.e., CRX::Venus-positive cells, or NRL-positive cell (rod photoreceptor precursor) in CRX::Venus-positive cells by culturing cell aggregates containing retinal tissue produced from human ES cells up to day 191 - day 192 from the start of suspension culture, which is the differentiation stage where Muller cells are observed, preparing a section of the recovered cell aggregates containing the retinal tissue, and performing immunostaining by a conventional method using an anti-GFP antibody (detecting CRX::Venus protein), an anti-NRL antibody, an anti-RXR-γ antibody, and DAPI. Compared to the non-treatment group (Control), it is clear that, when a dorsalization signal transmitter was added (+BMP4, +Cyclopamine-KAAD), the proportion of cone photoreceptor precursor was high, and conversely, it was low when a ventralization signal transmitter was added (+LDN193189, +SAG). Conversely, compared to the non-treatment group (Control), when a dorsalization signal transmitter was added (+BMP4, +Cyclopamine-KAAD), the proportion of rod photoreceptor precursor was low, and conversely, it was high when a ventralization signal transmitter was added (+LDN193189, +SAG).

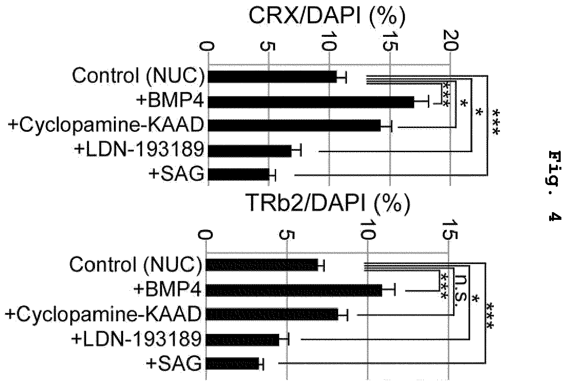

Fig. 4 shows graphs with the results obtained by culturing cell aggregates containing a retinal tissue produced from human ES cells up to about day 70-75 from the start of suspension culture, preparing a section of a cell aggregate containing retinal tissue, performing immunostaining using an anti-CRX antibody, an anti-TRβ2 antibody, and DAPI, and measuring the proportion of CRX-positive cell, and CRX-positive and TRβ2-positive cell to DAPI-positive cell by using an image analysis software. Compared to the non-treatment group (Control (NUC)), it is clear that, when a dorsalization signal transmitter was added (+BMP4, +Cyclopamine-KAAD), CRX-positive cell and CRX-positive and TRβ2-positive cell increased, and conversely, it decreased when a ventralization signal transmitter was added (+LDN193189, +SAG). In this differentiation stage, CRX-positive cell, and CRX-positive and TRβ2-positive cell are respectively photoreceptor precursor and cone photoreceptor precursor.

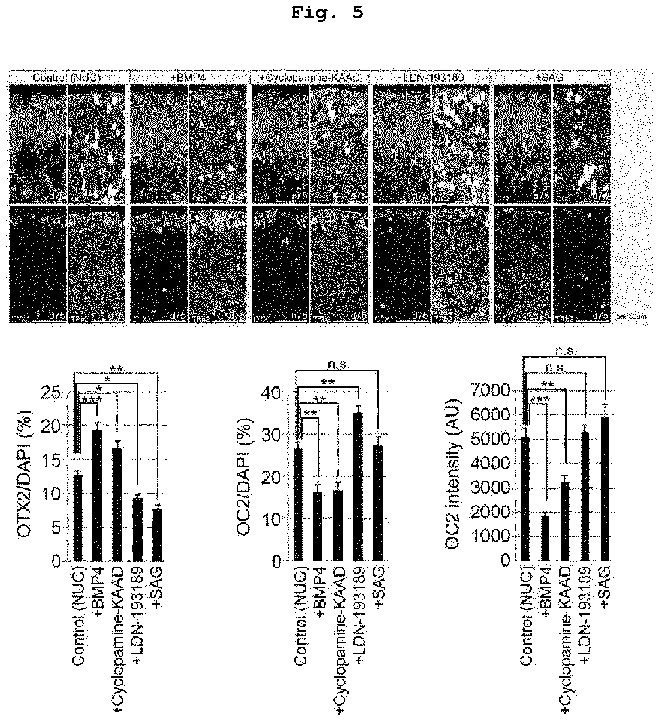

Fig. 5 shows a drawing (image) obtained by culturing cell aggregates containing a retinal tissue produced from human ES cells up to day 75 from the start of suspension culture, preparing a section of the collected cell aggregates containing retinal tissue, and performing immunostaining using an anti-OC2 antibody, an anti-OTX2 antibody, an anti-TRβ2 antibody, and DAPI and a graph showing similar immunostaining of retinal tissues on about day 70-75 from the start of suspension culture, and measurement of the proportion of OTX2-positive cell, OC2-positive cell, and fluorescence signal intensity of OC2-positive cell using an image analysis software. From these images and graphs, it is clear that, compared to the non-treatment group (Control (NUC)), both OTX2-positive cell and TRβ2-positive cell increased when a dorsalization signal transmitter was added (+BMP4, +Cyclopamine-KAAD), whereas OC2-positive cell decreased and the expression level was also low. Conversely, when a ventralization signal transmitter was added (+LDN193189, +SAG), both OTX2-positive cell and TRβ2-positive cell decreased, whereas OC2-positive cell increased with +LDN193189.

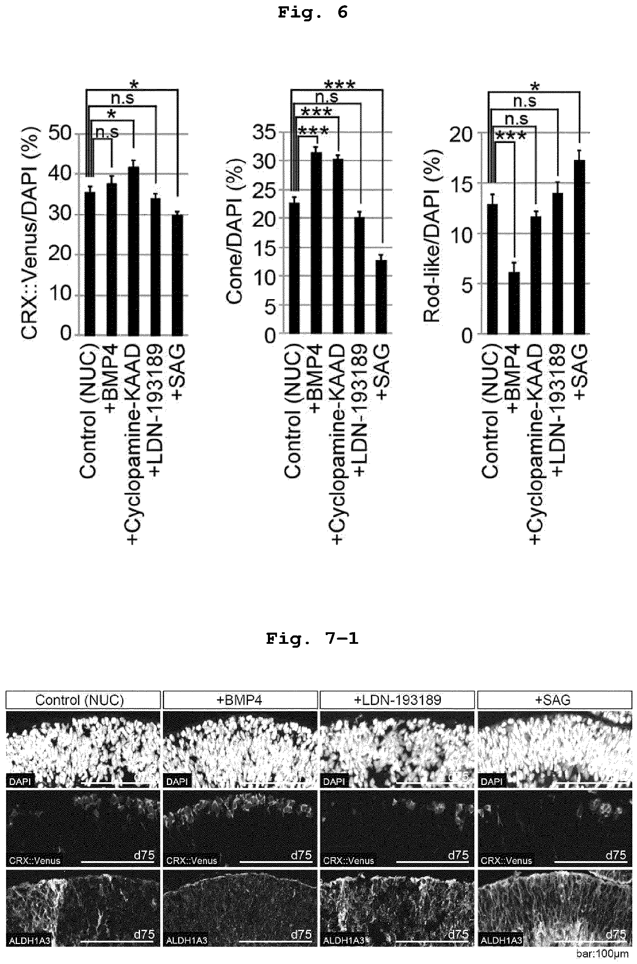

Fig. 6 shows graphs with the results obtained by culturing cell aggregates containing a retinal tissue produced from human ES cells up to about day 191-192 from the start of suspension culture, preparing a section of a cell aggregate containing retinal tissue, performing immunostaining using an anti-GFP antibody (defecting CRX::Venus protein), an anti-NRL antibody, an anti-RXR-γ antibody and DAPI, and measuring the proportion of RXR-γ-positive and NRL-negative cell (cone photoreceptor precursor) in CRX::Venus-positive cells, or NRL-positive cell (rod photoreceptor precursor) in CRX::Venus-positive cells by using an image analysis software. Compared to the non-treatment group (Control (NUC)), it is clear that, when a dorsalization signal transmitter was added (+BMP4, +Cyclopamine-KAAD), the proportion of cone photoreceptor precursor (Cone) was high, and conversely, it was low when a ventralization signal transmitter was added (+LDN193189, +SAG). Conversely, compared to the non-treatment group (Control (NUC)), it is clear that, when a dorsalization signal transmitter was added (+BMP4, +Cyclopamine-KAAD), the proportion of rod photoreceptor precursor-like cell (Rod-like) was low, and conversely, it was high when a ventralization signal transmitter was added, (+LDN193189, +SAG).
Fig. 7-1 shows a drawing (image) obtained by culturing cell aggregates containing a retinal tissue produced from human ES cells up to about day 75 from the start of suspension culture, preparing a section of the collected cell aggregates containing retinal tissue, and performing immunostaining using an anti-GFP antibody (detecting CRX::Venus protein), an anti-ALDH1A3 antibody, and DAPI. In the non-treatment group (Control (NUC)), it is clear that GFP-positive cell, i.e., CRX::Venus-positive cell is relatively less in ALDH1A3-positive region, i.e., ventralization region of retina, and differentiation of the photoreceptor precursors is suppressed. On the other hand, when a dorsalization signal transmitter was added (+BMP4), a decrease in the ALDH1A3-positive region and an accompanying increase in the CRX::Venus-positive cell are observed. On the other hand, it is clear that when a ventralization signal transmitter was added (+LDN193189, +SAG), an increase in the ALDH1A3-positive region and an accompanying decrease in the CRX::Venus-positive cell are found.

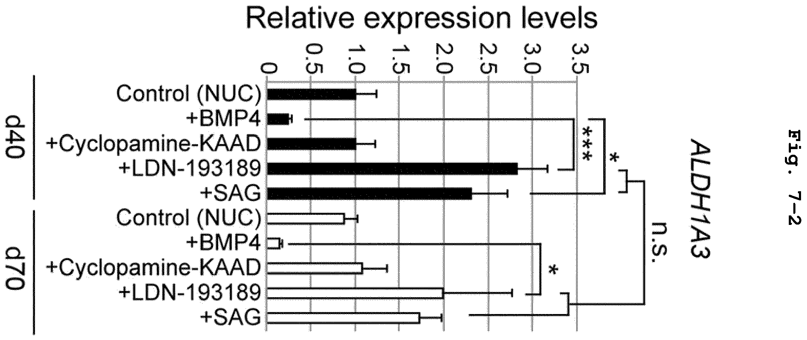

Fig. 7-2 shows a graph with the results obtained by culturing cell aggregates containing a retinal tissue produced from human ES cells up to about day 40-44 (d40) or 70-75 (d70) from the start of suspension culture, and performing quantitative PCR of the collected cell aggregates containing the retinal tissue. On day 70-75 (white bar) from the start of suspension culture, similar to the results of the immunostaining, compared to the non-treatment group (Control (NUC)), when a dorsalization signal transmitter was added (+BMP4), a decrease in the ALDH1A3 gene expression was found. On the other hand, it is clear that, when a ventralization signal transmitter was added (+LDN193189, +SAG), an increase in the ALDH1A3 gene expression was found. Such ALDH1A3 gene expression is of the same level as that on about day 40-44 from the start of suspension culture, and it was suggested that ALDH1A3 gene was expressed continuously from about day 40-44 from the start of suspension culture. From these, it is clear that the induction of differentiation into photoreceptor precursor or photoreceptor and suppression of ALDH1A3 expression are correlated, and a dorsalization signal transmitter having the level of suppressing ALDH1A3 expression or ALDH1A3 gene expression promotes differentiation of CRX::Venus cells.

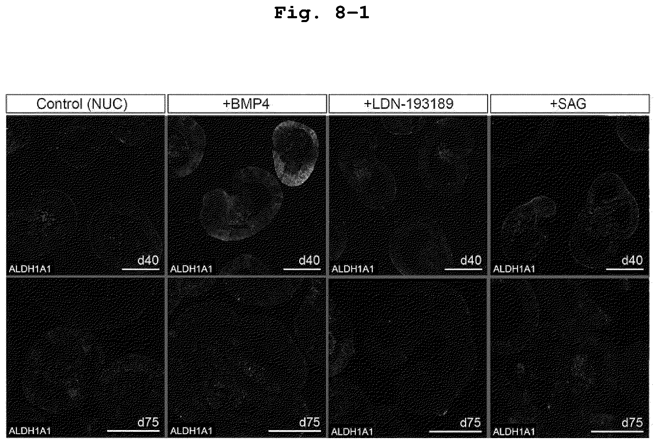

Fig. 8-1 shows a drawing (image) obtained by culturing cell aggregates containing a retinal tissue produced from human ES cells up to about day 40 or about day 75 from the start of suspension culture, preparing a section of the collected cell aggregates containing retinal tissue, and performing immunostaining using an anti-ALDHlAl antibody. In the non-treatment group (Control (NUC)) on about day 40 from the start of suspension culture, when a ventralization signal transmitter was added (+LDN193189, +SAG), an ALDH1A1-positive region was not found. On the other hand, when a dorsalization signal transmitter was added (+BMP4), an ALDH1A1-positive region on about day 40 from the start of suspension culture was found. However, it is clear that even when a dorsalization signal transmitter was added (+BMP4), such a clear ALDH1A1-positive region was no longer found on about day 75 from the start of suspension culture.

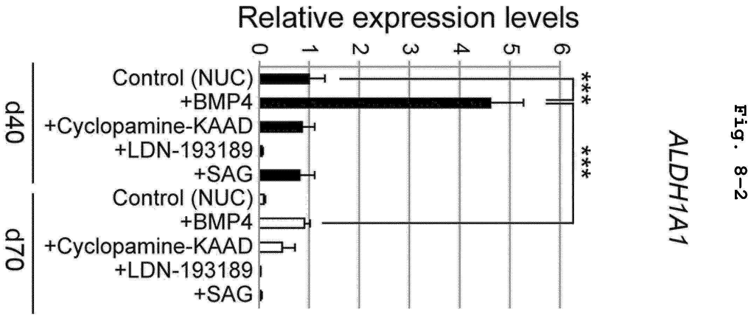

Fig. 8-2 shows a graph obtained by culturing cell aggregates containing a retinal tissue produced from human ES cells up to about day 40-44 (d40) or 70-75 (d70) from the start of suspension culture, and performing quantitative PCR of the collected cell aggregates containing the retinal tissue. It is clear that, on day 40-44 (black bar) from the start of suspension culture, similar to the results of the immunostaining, compared to the non-treatment group (Control (NUC)) and when a ventralization signal transmitter was added (+LDN193189, +SAG), an increase in the ALDH1A1 gene expression was found when a dorsalization signal transmitter was added (+BMP4). However, such ALDH1A1 gene expression decreased by about day 70-75 from the start of suspension culture and correlates to the results of immunostaining that failed to show a clear ALDH1A1 expression. From the above, it is clear that the expression of ALDH1A1 decreases in the stage where cone differentiation reaches maximum.

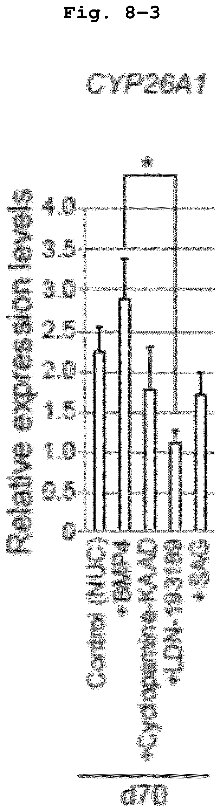

Fig. 8-3 shows a graph obtained by culturing cell aggregates containing a retinal tissue produced from human ES cells up to about day 70-75 (d70) from the start of suspension culture, and performing quantitative PCR of the collected cell aggregates containing the retinal tissue. Compared to when a ventralization signal transmitter was added (+LDN193189), it is clear that, an increase in the CYP26A1 gene expression was found when a dorsalization signal transmitter was added (+BMP4).

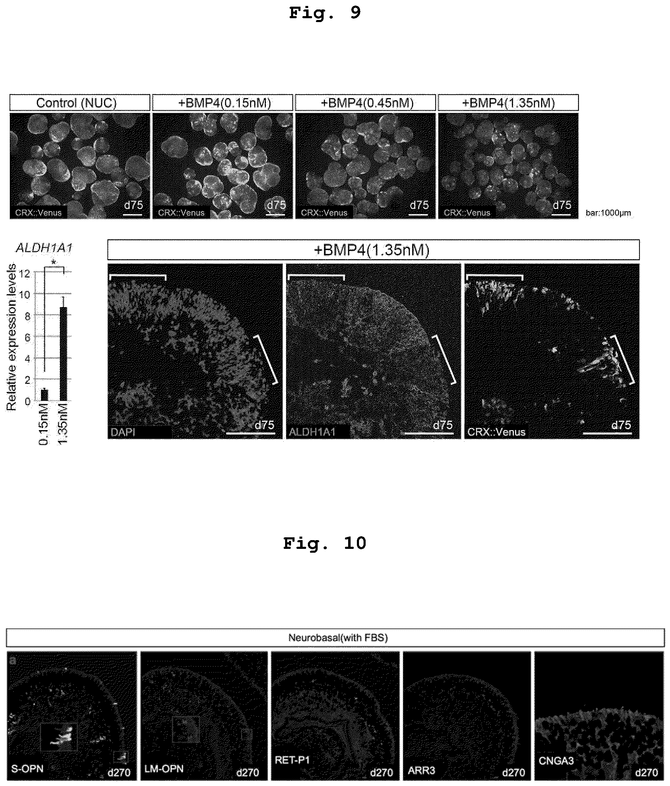

Fig. 9 shows the results obtained by culturing cell aggregates containing a retinal tissue produced from human ES cells up to about day 70-75 from the start of suspension culture, and performing observation of CRX::Venus by a fluorescence microscope (image, upper panel), quantitative PCR (graph), and immunostaining (image, lower panel), for the recovered cell aggregates containing the retinal tissue. Compared to when a 0.15 nM BMP4 was added, when 0.45 nM or 1.35 nM BMP4 was added, the fluorescence intensity of CRX::Venus became low, which indicates that CRX::Venus-positive cell decreased (image, upper panel). In addition, it is clear that addition of a high concentration (1.35 nM) of BMP4 increases expression of ALDH1A1 gene that is expressed in the dorsalization region of a retinal tissue (graph). It is clear that when cultured by adding 1.35 nM BMP4, a region strongly stained by an anti-ALDHlAl antibody contains relatively less CRX::Venus-positive cells, and relatively many CRX::Venus-positive cells are present in a region stained comparatively weakly by an anti-ALDHlAl antibody (blaket). Thus, it is clear that addition of a high concentration of BMP4 induces expression of ALDH1A1, promotion of differentiation of photoreceptor does not occur easily in a region with a high expression level of ALDH1A1, excessive induction of ALDH1A1 expression rather fails to promote differentiation of photoreceptor precursor.
Fig. 10 shows a drawing with the results obtained by culturing up to day 270 cell aggregates containing a retinal tissue produced from human ES cells through maturation to a level showing Muller cell (e.g., day 190 from the start of suspension culture), preparing a section of the recovered cell aggregate containing the retinal tissue, and performing immunostaining for S-Opsin (S-OPN), LM-Opsin (LM-OPN), Rhodopsin (RET-P1), Cone arrestin (ARR3), and cone photoreceptor-specific cyclic nucleotide-gated channel (CNGA3), which are expressed along with further maturation of photoreceptor. As a result, S-opsin expression was somewhat observed but almost all photoreceptor precursors did not express S-opsin (S-OPN), LM-Opsin (LM-OPN), Rhodopsin (RET-P1), and Cone arrestin (ARR3), which are expressed along with further maturation, and it is clear that final maturation is not found unlike in vivo.

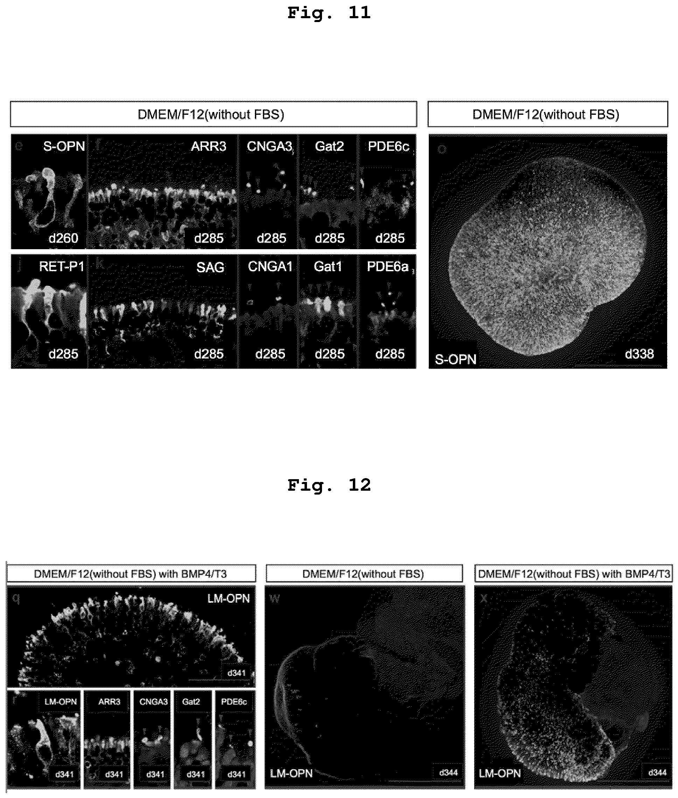

Fig. 11 shows a drawing with the results obtained by culturing cell aggregates containing a retinal tissue produced from human ES cells up to day 190 from the start of culture until it became a retinal tissue in a differentiation stage that matured to a level showing Muller cell, thereafter further culturing same up to day 260 - 338 from the start of culture in a fetal calf serum-free medium obtained by adding N2 supplement and taurine to DMEM/F12 medium containing glutamic acid as a component, preparing a section of the recovered cell aggregate containing the retinal tissue, and performing immunostaining for S-opsin (S-OPN), Rhodopsin (RET-P1), Cone arrestin (ARR3), Rod Arrestin (SAG), cone photoreceptor specific cyclic nucleotide-gated channel (CNGA3), rod-specific cyclic nucleotide-gated channel (CNGA1), cone photoreceptor-specific transducin alpha subunit (Gat2), rod photoreceptor-specific transducin alpha subunit (Gat1), cone photoreceptor-specific Phosphodiesterase 6C (PDE6c), and rod photoreceptor-specific Phosphodiesterase 6A (PDE6a), which are expressed along with further maturation of photoreceptor. As a result, these functional molecules that are expressed along with further maturation were expressed in cone photoreceptor or rod photoreceptor and it was found that final maturation was promoted. In addition, it was found that the maturated photoreceptor was observed in the entire retinal tissue.
Fig. 12 shows a drawing with the results obtained by culturing cell aggregates containing a retinal tissue produced from human ES cells up to day 190 from the start of culture until it became a retinal tissue in a differentiation stage that matured to a level showing Muller cell, thereafter further culturing same up to day 341 - 344 from the start of culture in a fetal calf serum-free medium obtained by adding N2 supplement and taurine to DMEM/F12 medium containing glutamic acid as a component and adding BMP4 and T3, preparing a section of the recovered cell aggregate containing the retinal tissue, and performing immunostaining for LM-opsin (LM-OPN), Cone arrestin (ARR3), cone photoreceptor specific cyclic nucleotide-gated channel (CNGA3), cone photoreceptor-specific transducin alpha subunit (Gat2), and cone photoreceptor-specific Phosphodiesterase 6C (PDE6c), which are expressed along with further maturation of photoreceptor. As a result, these functional molecules that are expressed along with further maturation were expressed in cone photoreceptor and it was found that final maturation was promoted. It was found that, expression of LM-opsin was promoted when BMP4 and T3 were added compared to when BMP4 and T3 were not added.

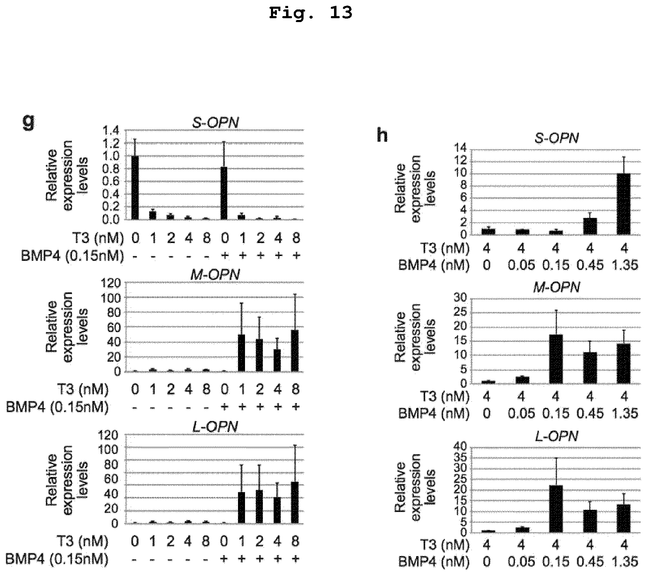

Fig. 13 shows a drawing with the results obtained by culturing cell aggregates containing a retinal tissue produced from human ES cells up to day 190 from the start of culture until it became a retinal tissue in a differentiation stage that matured to a level showing Muller cell, further culturing same for not less than 30 days from the start of culture for achieving further maturation in a fetal calf serum-free medium obtained by adding N2 supplement and taurine to DMEM/F12 medium containing glutamic acid as a component and adding BMP4 and T3, performing quantitative PCR of the recovered cell aggregate containing the retinal tissue according to a conventional method, and confirming the expression levels of S-opsin, M-opsin, L-opsin. As a result, when T3 was not contained, expression of S-opsin was promoted irrespective of the presence or absence of BMP4 addition. Thus, it was found that further maturation to S cone photoreceptor, i.e., final maturation, was promoted. On the other hand, it was found that expression of S-opsin was suppressed when T3 was added, and T3 suppressively acts on final maturation to S cone photoreceptor. When BMP4 was added in combination with T3, expression of L and M-opsins was promoted, and it was found that further maturation to L or M vertebral vision, i.e., final maturation, was promoted.

### [Description of Embodiments]

### 1. Definition

In the present specification, "stem cell" means an undifferentiated cell having differentiation potency and proliferative capacity (particularly self-renewal competence) maintaining the same differentiation potency even after cell division. The stem cell includes subpopulations such as pluripotent stem cell, multipotent stem cell, unipotent stem cell and the like according to the differentiation potency. Pluripotent stem cell refers to a stem cell capable of being cultured in vitro and having a potency to differentiate into any cell lineage belonging to three germ layers (ectoderm, mesoderm, endoderm) (pluripotency). The multipotent stem cell means a stem cell having a potency to differentiate into plural types of tissues or cells, though not all kinds. The unipotent stem cell means a stem cell having a potency to differentiate into a particular tissue or cell.

Pluripotent stem cell can be induced from fertilized egg, clone embryo, germ stem cell, stem cell in a tissue and the like. Examples of the pluripotent stem cell include embryonic stem cell (ES cell), EG cell (embryonic germ cell), induced pluripotent stem cell (iPS cell) and the like.

Embryonic stem cell was first established in 1981, and has also been applied to the generation of knockout mouse since 1989. In 1998, human embryonic stem cell was established, which is also being utilized for regenerative medicine. ES cell can be produced by culturing an inner cell mass on a feeder cell or in a medium containing LIF. The production methods of ES cell are described in, for example, WO 96/22362, WO 02/101057, US 5,843,780, US 6,200,806, US 6,280,718 and the like. Embryonic stem cells are available from given organizations, or a commercially available product can be purchased. For example, human embryonic stem cells, KhES-1, KhES-2 and KhES-3, are available from Kyoto University's Institute for Frontier Medical Sciences. EB5 cell, which is a mouse embryonic stem cell, is available from Incorporated Administrative Agency RIKEN, and D3 cell line, which is a mouse embryonic stem cell, is available from ATCC.

Nuclear transfer ES cell (ntES cell), which is one of the ES cells, can be established from a clone embryo produced by transplanting the nucleus of a somatic cell into an enucleated egg.

The "induced pluripotent stem cell" (to be also referred to as iPS cell) in the present invention is a cell induced to have pluripotency by reprogramming a somatic cell by a known method and the like. Specifically, a cell induced to have pluripotency by reprogramming differentiated somatic cells such as fibroblast, peripheral blood mononuclear cell and the like by the expression of a combination of plural genes selected from the group consisting of reprogramming genes including Oct3/4, Sox2, Klf4, Myc (c-Myc, N-Myc, L-Myc), Glis1, Nanog, Sall4, lin28, Esrrb and the like can be mentioned. Examples of preferable combination of reprogramming factors include (1) Oct3/4, Sox2, Klf4, and Myc (c-Myc or L-Myc), and (2) Oct3/4, Sox2, Klf4, Lin28 and L-Myc (Stem Cells, 2013; 31:458-466).

Induced pluripotent stem cell was established by Yamanaka et al. in mouse cell in 2006 (Cell, 2006, 126(4), pp.663-676). In 2007, induced pluripotent stem cell was also established from human fibroblast, and has pluripotency and self-renewal competence similar to those of embryonic stem cells (Cell, 2007, 131(5), pp.861-872; Science, 2007, 318(5858), pp.1917-1920; Nat. Biotechnol., 2008, 26(1), pp.101-106). The induction method of induced pluripotent stem cell has been variously improved thereafter (e.g., mouse iPS cell: Cell. 2006 Aug 25; 126(4):663-76, human iPS cell: Cell. 2007 Nov 30; 131(5):861-72) .

Besides the production method of induced pluripotent stem cell based on direct reprogramming by gene expression, induced pluripotent stem cell can also be obtained from somatic cell by the addition of a compound and the like (Science, 2013, 341, pp. 651-654).

It is also possible to obtain established induced pluripotent stem cell and, for example, human induced pluripotent cell lines established by Kyoto University such as 201B7 cell, 201B7-Ff cell, 253G1 cell, 253G4 cell, 1201C1 cell, 1205D1 cell, 1210B2 cell or, 1231A3 cell, Ff-I01 cell, QHJI01 cell and the like are available from Kyoto University.

While the somatic cell used for obtaining induced pluripotent stem cell is not particularly limited, tissue-derived fibroblast, blood-lineage cells (e.g., peripheral blood mononuclear cell, T cell), hepatocyte, pancreatic cell, intestinal epithelial cell, smooth muscle cell and the like can be mentioned. As the fibroblast, those derived from corium and the like can be mentioned.

When induced pluripotent stem cell is produced by reprogramming by the expression of several kinds of genes, the means for gene expression is not particularly limited. Examples of the aforementioned means include an infection method using a virus vector (e.g., retrovirus vector, lentivirus vector, Sendaivirus vector, adenovirus vector, adeno-associated virus vector), a gene transfer method using a plasmid vector (e.g., plasmid vector, episomal vector) (e.g., calcium phosphate method, lipofection method, RetroNectin method, electroporation method), a gene transfer method using an RNA vector (e.g., calcium phosphate method, lipofection method, electroporation method), a method with direct injection of protein and the like.

The pluripotent stem cell to be used in the present invention is preferably ES cell or induced pluripotent stem cell, more preferably induced pluripotent stem cell (iPS cell).

The pluripotent stem cell to be used in the present invention is preferably a pluripotent stem cell of primates (e.g., human, monkey), preferably a human pluripotent stem cell. Therefore, the pluripotent stem cell to be used in the present invention is preferably a human ES cell or human induced pluripotent stem cell (human iPS cell), most preferably a human induced pluripotent stem cell (human iPS cell).

Genetically-modified pluripotent stem cells can be produced by using, for example, a homologous recombination technique. Examples of the gene on the chromosome to be modified include a cell marker gene, a histocompatibility antigen gene, a gene related to a disease due to a disorder of retinal cell and so on. A target gene on the chromosome can be modified using the methods described in Manipulating the Mouse Embryo, A Laboratory Manual, Second Edition, Cold Spring Harbor Laboratory Press (1994); Gene Targeting, A Practical Approach, IRL Press at Oxford University Press (1993); Biomanual Series 8, Gene Targeting, Making of Mutant Mouse using ES cell, YODOSHA CO., LTD. (1995); and so on.

To be specific, for example, the genomic DNA containing the target gene to be modified (e.g., cell marker gene, histocompatibility antigen gene, disease-related gene and so on) is isolated, and a targeting vector used for homologous recombination of the target gene is produced using the isolated genomic DNA. The produced targeting vector is introduced into stem cells and the cells that showed homologous recombination between the target gene and the targeting vector are selected, whereby stem cells having the modified gene on the chromosome can be produced.

Examples of the method for isolating genomic DNA containing the target gene include known methods described in Molecular Cloning, A Laboratory Manual, Second Edition, Cold Spring Harbor Laboratory Press (1989), Current Protocols in Molecular Biology, John Wiley & Sons (1987-1997) and so on. The genomic DNA containing the target gene can also be isolated using genomic DNA library screening system (manufactured by Genome Systems), Universal GenomeWalker Kits (manufactured by CLONTECH) and so on. A polynucleotide encoding the target protein can also be used instead of genome DNA. The polynucleotide can be obtained by amplifying the corresponding polynucleotide by the PCR method.

Production of targeting vector used for homologous recombination of the target gene, and efficient selection of a homologous recombinant can be performed according to the methods described in Gene Targeting, A Practical Approach, IRL Press at Oxford University Press (1993); Biomanual Series 8, Gene Targeting, Making of Mutant Mouse using ES cell, YODOSHA CO., LTD. (1995); and so on. As the targeting vector, any of replacement type or insertion type can be used. As the selection method, methods such as positive selection, promoter selection, negative selection, polyA selection and so on can be used.

Examples of a method for selecting the desired homologous recombinant from the selected cell lines include Southern hybridization method, PCR method and so on for the genomic DNA.

The "suspension culture" or "suspension culture method" in the present invention refers to culturing while maintaining a state in which cells or cell aggregates are suspended in a culture medium and a method of performing the culture. That is, suspension culture is performed under conditions in which cells or cell aggregates are not adhered to a culture vessel and the like, and culturing performed under conditions permitting adhesion to a culture vessel and the like (adhesion culture or adhesion culture method) is not included in the category of suspension culture. In this case, adhesion of cell means that a strong cell-substratum junction is formed between a cell or cell aggregate and a culture vessel. More particularly, suspension culturing refers to culturing under conditions in which a strong cell-substratum junction is not formed between a cell or cell aggregate and a culture vessel, and "adhesion culture" refers to culturing under conditions in which a strong cell-substratum junction is formed between a cell or cell aggregate and a culture vessel material and the like.

In a cell aggregate in suspension culture, a planar cell-cell adhesion is formed (plane attachment). In cell aggregates in suspension culture, a cell-substratum junction is hardly formed with a culture vessel and the like and, even if it is formed, its contribution is small. In some embodiment, in a cell aggregate in suspension culture an endogenous cell-substratum junction is present inside the aggregate, but a cell-substratum junction is hardly formed with a culture vessel and the like and, even if it is formed, its contribution is small. From such aspect, one embodiment of "suspension culture" is, for example, a culture method in which a cell aggregate is fixed on a thin needle equipment or the like that works as a scaffold for cell aggregates and cultured in equipment filled with a culture medium. Examples of the culture method include a method using a bio 3D printer "Regenova (registered trade mark)" manufactured by Cyfuse Biomedical K.K., reported at 29AB-pm009, the 136th Annual Meeting of the Pharmaceutical Society of Japan.

The planar cell-cell adhesion means that a cell attaches to another cell via planes. More particularly, the planar cell-cell adhesion means that, for example, not less than 1%, preferably not less than 3%, more preferably not less than 5%, of the surface area of a cell adheres to the surface of another cell. A surface of a cell can be observed by staining with a reagent (e.g., DiI) that stains membranes, immunostaining of cell adhesion molecules (e.g., E-cadherin and N-cadherin).

The culture vessel to be used when performing suspension culture is not particularly limited as long as it enables "culturing in suspension" and those of ordinary skill in the art can appropriately determine the same. Examples of such culture vessel include flask, tissue culture flask, dish, petri dish, tissue culture dish, multidish, microplate, microwell plate, micropore, multiplate, multiwell plate, chamber slide, schale, tube, tray, culture bag, spinner flask, roller bottle and so on. To enable suspension culture, these culture vessels are preferably non-cell-adhesive. Non-cell-adhesive culture vessels include culture vessels whose surfaces have not undergone an artificial treatment for improving the cell adhesiveness (e.g., coating treatment with extracellular matrix such as basement membrane preparation, laminin, entactin, collagen, gelatin etc., and the like, or with polymer such as polylysine, polyornithine etc. and the like or surface processing such as positive electric charge treatment and the like), and the like. As a non-cell-adhesive culture vessel, culture vessels whose surfaces have been artificially treated to decrease adhesiveness to the cells (e.g., superhydrophilic treatment with MPC polymer and the like, protein low adsorption treatment etc.) and the like can be used. Rotation culture using spinner flask, roller bottle and the like may be performed. The culture surface of the culture vessel may be a flat bottom or may have concaves and convexes.

On the other hand, as an incubator used for adhesion culture, culture vessels whose surfaces have undergone an artificial treatment for improving the cell adhesiveness (e.g., surface treatment with extracellular matrix such as basement membrane preparation, laminin, entactin, collagen, gelatin, Matrigel, Synthemax, vitronectin and the like, and the like, or coating treatment with polymer such as polylysine, polyornithine and the like or positive electric charge treatment and the like), and the like can be mentioned.

In the present specification, an aggregate of cells (cell cluster or cell aggregate) is not particularly limited as long as plural cells are adhered to each other to form a cluster, and may be a cell aggregate formed by assembly of cells dispersed in a medium, or derived from a colony formed by cell culture, or a cell aggregate formed by newly budding from other cell aggregate. The cell aggregate also encompasses embryoid body, sphere and spheroid. Preferably, a planar cell-cell adhesion is formed in the aggregate of cells. In some embodiments, cells sometimes form a cell-cell junction or a cell adhesion, for example, adherence junction, in some or all of the cell aggregates. The aggregate also includes a cell population as a derivative obtained from the aforementioned cell aggregate.

The "uniformed aggregates" means that the size of each aggregate is constant when plural aggregates are cultured, and that the variance in the length of the maximum diameter is small when the size of the aggregates are evaluated by the length of the maximum diameter. More specifically, it means that not less than 75% of aggregates in the whole aggregate population are within mean ± 100%, preferably mean ± 50%, more preferably mean ± 20%, of the maximum diameter in the population of the aggregates.

To "form uniformed cell aggregates" means to "rapidly aggregate a given number of dispersed cells" to form cell aggregates uniform in size, when assembling the cells to form cell aggregates and culturing the aggregates in suspension. That is, when an aggregate of pluripotent stem cells is formed by rapidly assembling the pluripotent stem cells, an epithelium-like structure can be formed with good reproducibility in the cells induced and differentiated from the formed aggregate. To be specific, a cell aggregate having an epithelium-like structure can be formed by rapidly aggregating pluripotent stem cells in a serum-free medium (SFEBq method (Serum-free Floating culture of Embryoid Body-like aggregates with quick reaggregation)).

Examples of the experimental operation to form the aggregate include a method involving keeping cells in a small space by using a plate with small wells (e.g., plate with wells having a base area of about 0.1 - 2.0 cm² when calculated in terms of flat bottom; 96 well plate), micropore and so on, a method involving aggregating cells by centrifugation for a short time using a small centrifugation tube and the like.

As a plate with small wells, for example, 24 well plate (area of about 1.88 cm² when calculated in terms of flat bottom), 48 well plate (area of about 1.0 cm² when calculated in terms of flat bottom), 96 well plate (area of about 0.35 cm² when calculated in terms of flat bottom, inner diameter about 6 - 8 mm), and 384 well plate can be mentioned. Preferred is 96 well plate. As a shape of the plate with small wells, the shape of the bottom surface when the well is seen from above is, for example, polygon, rectangle, ellipse, true circle, preferably true circle. As a shape of the plate with small wells when the well is seen from the side well, the shape of the bottom surface is preferably a structure having high outer circumference and low inner concave. The shape includes, for example, U-bottom, V-bottom, µ-bottom, preferably U-bottom or V-bottom, most preferably V-bottom. As a plate with small wells, a cell culture dish (e.g., 60 mm - 150 mm dish, culture flask) with a concave convex, or dent on the bottom surface (e.g., EZSPHERE (AGC TECHNO GLASS CO., LTD.)) may also be used. The bottom surface of a plate with small wells is preferably a non-cell-adhesive bottom surface, preferably the aforementioned non-cell-adhesive-coated bottom surface.

"Dispersion" means that cells and tissues are separated into small cell debris (not less than 2 cells and not more than 100 cells, preferably not more than 50 cells) or single cells by dispersion treatments such as enzyme treatment, physical treatment and the like. A certain number of dispersed cells mean a collection of a certain number of cell debris or single cells. Examples of the method for dispersing pluripotent stem cells include mechanical dispersion treatment, cell dispersing solution treatment, and cell protecting agent addition treatment. These treatments may be performed in combination. It is preferable to perform a cell dispersing solution treatment and then a mechanical dispersion treatment. Examples of the method for the mechanical dispersion treatment include pipetting treatment and scraping operation with a scraper.

The "tissue" in the present specification refers to a structure of a cell population having a structure in which plural types of cells having different morphologies and properties are sterically arranged in a given pattern.

In the present specification, the "retinal tissue" means a tissue in which at least plural types of retinal cells (in the case of retinal progenitor cell, other retinal cells may not be contained), such as photoreceptors, horizontal cells, bipolar cells, amacrine cells, ganglion cells, retinal pigment epithelial cells, Muller cells, progenitor cells of these (neural retina progenitor cell or retinal progenitor cell) and the like, which constitute respective retinal layers in retina in vivo, are sterically arranged in layers. Which retinal layer is constituted by respective cells can be confirmed by a known method, for example, the presence or absence of the expression of a cell marker or the level thereof, and the like.

In the present specification, the "retinal tissue" includes retinal tissue obtained by inducing differentiation of pluripotent stem cells and retinal tissue derived from a living body. Specifically, epithelial tissues that are obtained by suspension culture of cell aggregates formed from pluripotent stem cells under appropriate differentiation-inducing conditions and contain retinal progenitor cells and/or neural retinal progenitor cells formed on the surface of the aforementioned aggregate, and a part of the cell aggregate can be mentioned.

In the present specification, the "cell aggregate containing retinal tissue" is not particularly limited as long as it is a cell aggregate containing the aforementioned retinal tissue.

In the present invention, the "retinal layer" means optional each layer constituting the retina. Specific examples thereof include retinal pigment epithelial layer and neural retinal layer, and the neural retinal layer includes outer limiting membrane, photoreceptor layer (outer nuclear layer), outer plexiform layer, inner nuclear layer, inner plexiform layer, ganglion cell layer, nerve fiber layer and inner limiting membrane. In addition, in a retinal tissue in a stage between the below-mentioned "retinal tissue in an initial developmental stage" and a matured retinal tissue, a neural retina layer contains a layer containing neural retina progenitor cells which is called a neuroblastic layer in a neural retinal tissue.

In the present invention, the "retinal progenitor cell" refers to a progenitor cell capable of differentiating into any mature retinal cells constituting a retinal tissue, including photoreceptor, horizontal cell, bipolar cell, amacrine cell, ganglion cell, retinal pigment epithelial cell and Muller cell.

In the present invention, the "neural retinal progenitor cell" refers to a cell that is destined to be the inner layer of the optic cup. It includes, for example, a progenitor cell capable of differentiating into any mature cell constituting a neural retinal layer (retinal layer containing retinal layer-specific neuron) that does not contain retinal pigment epithelium.

The photoreceptor precursor, horizontal cell precursor, bipolar cell precursor, amacrine cell precursor, ganglion cell precursor, and retinal pigment epithelial precursor refer to precursor cells committed to differentiate into photoreceptor, horizontal cell, bipolar cell, amacrine cell, ganglion cells, and retinal pigment epithelial cell, respectively. The differentiation stages are continuous and it is difficult to clearly distinguish, for example, the boundary of differentiation stages that shift from photoreceptor precursor to photoreceptor. In the present specification, therefore, photoreceptor, horizontal cell, bipolar cell, amacrine cell, ganglion cell, retinal pigment epithelial cell and the like may include respective precursor cells. Conversely, photoreceptor precursor, horizontal cell precursor, bipolar cell precursor, amacrine cell precursor, ganglion cell precursor, retinal pigment epithelial cell precursor and the like may include respective differentiated cells thereof, namely, photoreceptor, horizontal cell, bipolar cell, amacrine cell, ganglion cell, or retinal pigment epithelial cell and the like.

In the present specification, the "retinal layer-specific neuron" is a cell constituting a retinal layer and is a neuron specific to the retinal layer. Examples of the retinal layer-specific neuron include bipolar cell, ganglion cells, amacrine cell, horizontal cell and photoreceptor, and examples of the photoreceptor include rod photoreceptor (Rod photoreceptor cell) and cone photoreceptor (Cone photoreceptor cell). Examples of the cone photoreceptor include S-cone photoreceptor that expresses S-opsin and receives blue light, L-cone photoreceptor that expresses L-opsin and receives red light, and M cone photoreceptor that expresses M-opsin and receives green light.

In the present specification, the "retinal cell" is a concept encompassing the aforementioned retinal pigment epithelial cell, Muller cell, photoreceptor, horizontal cell, bipolar cell, amacrine cell, ganglion cell and precursor cell thereof, retinal progenitor cell, and neural retinal progenitor cell, and retinal layer-specific neuron and progenitor cell of the retinal layer-specific neuron and the like.

The cells constituting the aforementioned retinal tissue can be detected or identified using an expressed or non-expressed retinal cell marker as an index.

Examples of the retinal cell marker include genes and proteins that are predominantly expressed in retinal cells, and the following can be exemplified for each cell. Alternatively, a gene or protein that is predominantly expressed in cells other than retinal cell can be used as a negative marker.

Examples of the negative marker of retinal cells such as retinal progenitor cell, neural retinal progenitor cell, photoreceptor precursor and the like include Nkx2.1 expressed in precursor of hypothalamus neuron but not expressed in retinal progenitor cell, Sox1 expressed in hypothalamus neuroepithelium but not expressed in retina and the like.

Examples of the retinal progenitor cell marker include RX (also referred to as RAX) and PAX6.

Examples of the neural retinal progenitor cell marker include RX, PAX6 and CHX10.

Examples of the marker of the retinal layer-specific neuron include Chx10, PKCα, Goα, VSX1 and L7 expressed in bipolar cell, TUJ1 and BRN3 expressed in ganglion cell, Calretinin and HPC-1 expressed in amacrine cell, Calbindin expressed in horizontal cell, LIM1 and the like.

Examples of the marker expressed in photoreceptor precursor and photoreceptor include CRX, Recoverin, BLIMP1, OTX2 and the like. Furthermore, a marker expressed in a rod photoreceptor and a rod photoreceptor precursor is, for example, NRL, Rhodopsin or the like. Therefore, for example, using a CRX-positive cell being NRL-positive as an index, rod photoreceptor and rod photoreceptor precursor can be identified. As a marker expressed in cone photoreceptor, cone photoreceptor precursor and ganglion cell, RXR-γ can be mentioned. As a marker expressed in cone photoreceptor and cone photoreceptor precursor, TRβ2, or TRβ1 can be mentioned. For example, cone photoreceptor precursor can be identified using coexpression of TRβ2 and CRX, or TRβ1 and CRX as an index. A cone photoreceptor precursor coexpresses RXR-γ and CRX, and can also be identified using the absence of NRL expression as an index.

OC1 (ONECUT1/HNF6) and OC2 (ONECUT2) are necessary for differentiation of a cone photoreceptor precursor, and are factors that are transiently expressed during differentiation. They are also expressed in a part of ganglion cell, horizontal cell, and a part of amacrine cell. For example, when cone photoreceptor precursor or cone photoreceptor that expresses OC1 and OC2 and precursor of horizontal cell are differentiated into cone photoreceptor precursor or cone photoreceptor and horizontal cell, the expression of OC1 and OC2 decreases in the cone photoreceptor precursor or cone photoreceptor, whereas the expression of OC1 and OC2 increases in the horizontal cell. Therefore, the differentiation efficiency of the cone photoreceptor precursor can be determined by measuring the expression level or proportion thereof.

That a cone photoreceptor and a cone photoreceptor precursor are induced and the retinal tissue is at the stage before emergence of rod photoreceptor precursor can be confirmed using CRX-positive cell being NRL-negative and TRβ2-positive, or being NRL-negative and RXR-γ-positive as an index. OTX2 is a marker expressed in a photoreceptor precursor and photoreceptor, as well as bipolar cell, and can be utilized as a marker for cone photoreceptor precursor and cone photoreceptor when the OTX2-positive cell contained in the neural retinal tissue is CHX10-negative and NRL-negative. On the other hand, of the OTX2-positive cells contained in a neural retinal tissue, NRL-positive cell can be identified as a rod photoreceptor precursor and a rod photoreceptor.

As the S cone photoreceptor marker, S-opsin can be mentioned, as the L cone photoreceptor marker, L-opsin can be mentioned, and as the M cone photoreceptor marker, M-opsin can be mentioned.

Examples of the marker commonly expressed in horizontal cell, amacrine cell and ganglion cell include PAX6 and the like.

In addition, examples of the marker of a retinal cell contained in a retinal tissue include RPE65, MITF and PAX6 expressed in retinal pigment epithelial cell, CRABP and CRALBP expressed in Muller cell and the like.

The dorsal marker and ventral marker in a retinal tissue mean a gene and a protein that are expressed in tissues respectively corresponding to the dorsal and ventral sides in a retinal tissue.

Examples of the dorsal marker include markers such as TBX5, TBX3, TBX2, COUP-TF II, CYP26A1, CYP26C1, ALDH1A1 and the like which are expressed in the dorsalization region of a neural retinal tissue among the retinal tissues. Of these, COUP-TF II can be classified as the "most dorsal marker", and ALDH1A1 is also a factor whose expression level increases as it approaches the region. In addition, examples of the ventral marker include markers such as VAX2, COUP-TF I, ALDH1A3 and the like that are expressed in the ventral region of neural retinal tissue.

In suspension culture of a neural retinal tissue in vivo or a neural retinal tissue induced to differentiate from pluripotent stem cell, it was not known that potentiation of the dorsalization signal increases the proportion of cone photoreceptors induced to differentiate. However, the present inventors have found that
1) with a dorsalization signal transmitter, dorsal marker CYP26A1-positive region (that is, neural retinal tissue with increased expression of CYP26A1) can be induced, and a neural retinal tissue with an increased content ratio of cone photoreceptor precursor and cone photoreceptor can be obtained, and
2) in a neural retinal tissue present on a further dorsal side from the aforementioned 1), in which a most dorsal marker COUP-TF II has been induced, the proportion of cone photoreceptor precursor and cone photoreceptor is rather suppressed.

On the other hand, it is known to those of ordinary skill in the art that cone photoreceptor precursor and cone photoreceptor do not emerge in a retinal pigment epithelium present further dorsally from 2).

Therefore, a neural retina rich in the proportion of cone photoreceptor precursor and cone photoreceptor can be produced by confirming CYP26A1, COUP-TF II, RPE65 or MITF as a marker, and appropriately adjusting the intensity of dorsalization signal such that the dorsal marker is promoted and expression of the most dorsal marker is not promoted.

The "serum-free medium" in the present specification means a medium not containing an unadjusted or unpurified serum. In the present specification, a medium containing purified blood-derived components and animal tissue-derived components (e.g., growth factor) is also included in the serum-free medium unless unadjusted or unpurified serum is contained therein.

The "serum-free conditions" in the present specification means conditions free of unadjusted or unpurified serum, specifically, conditions using a serum-free medium.

The serum-free medium here may contain a serum replacement. Examples of the serum replacement include one appropriately containing albumin, transferrin, fatty acid, collagen precursor, trace element, 2-mercaptoethanol or 3' thiolglycerol, or equivalents of these etc., and so on. Such serum replacement may be prepared by, for example, the method described in WO 98/30679. The serum replacement may be a commercially available product. Examples of such commercially available serum replacement include Knockout™ Serum Replacement (Life Technologies, hereinafter sometimes to be indicated as KSR), Chemically-defined Lipid concentrated (manufactured by Life Technologies) and Glutamax™ (manufactured by Life Technologies), B27 (manufactured by Life Technologies), N2 (manufactured by Life Technologies).

The serum-free medium may appropriately contain a fatty acid or lipid, amino acid (e.g., non-essential amino acids), vitamin, growth factor, cytokine, antioxidant, 2-mercaptoethanol, pyruvic acid, buffering agent, inorganic salts and so on.

To avoid complicated preparation, a serum-free medium supplemented with an appropriate amount (e.g., about 0.5% to about 30%, preferably about 1% to about 20%) of commercially available KSR (manufactured by Life Technologies) may be used as such serum-free medium (e.g., medium of 1:1 mixture of F-12 medium and IMDM medium supplemented with 10% KSR and 450 µM 1-monothioglycerol). As a product equivalent to KSR, the medium disclosed in JP-A-2001-508302 can be mentioned.

The "serum-containing medium" in the present specification means a medium containing unadjusted or unpurified serum. The medium may contain a fatty acid, lipid, amino acid (e.g., non-essential amino acids), vitamin, growth factor, cytokine, antioxidant, 2-mercaptoethanol, 1-monothioglycerol, pyruvic acid, buffering agent, inorganic salts and so on. In addition, a serum-containing medium can be used in the step of maintaining retinal cell or retinal tissue produced by the present invention (Cell Stem Cell, 10(6), 771-775 (2012)).

Known growth factors, proteins, additives or chemical substances that promote proliferation and the like may be added to the aforementioned serum-free medium or serum-containing medium. Examples of the known growth factor and protein include EGF, FGF, IGF, insulin and the like. Examples of additives that promote proliferation include N2 supplement (N2, Invitrogen), B27 supplement (Invitrogen), and the like. Examples of chemical substances that promote proliferation include retinoids (e.g., retinoic acid or a derivative thereof), taurine, glutamine and the like.

In the present specification, "xeno-free" means conditions eliminating components derived from species different from that of the cell to be cultured.

In the present specification, the "medium containing substance X" and "in the presence of substance X" refer to a medium supplemented with an exogenous substance X or a medium containing an exogenous substance X, or in the presence of an exogenous substance X. That is, when the cells or tissues present in the medium endogenously express, secrete or produce substance X, the endogenous substance X is distinguished from the exogenous substance X, and a medium free of exogenous substance X is understood to fall outside the category of the "medium containing substance X", even when it contains endogenous substance X.

For example, the "medium containing a dorsalization signal transmitter" is a medium added with an exogeneous dorsalization signal transmitter or a medium containing an exogeneous dorsalization signal transmitter. The "in the presence of dorsalization signal transmitter" means in the presence of an exogeneous dorsalization signal transmitter. The "medium free of a BMP signal transduction pathway inhibitor" is a medium not added with an exogeneous BMP signal transduction pathway inhibitor or a medium not containing an exogeneous BMP signal transduction pathway inhibitor.

In the present specification, the "dorsalization signal transmitter" means a signal transmitter that promotes differentiation into a retinal tissue formed on the dorsal side in the development process. Examples of the dorsalization signal transmitter include BMP signal transduction pathway agonist that transmits a dorsalization signal, a Wnt signal transduction pathway agonist that induces expression of BMP, and a Sonic hedgehog (hereinafter sometimes indicated as SHH) signal transduction pathway inhibitor that inhibits a ventralization signal. A substance that inhibits a ventralization signal is also referred to as a ventralization signal transduction inhibitory substance.

The aforementioned BMP signal transduction pathway agonist is a substance capable of enhancing signal transduction mediated by BMP. Specific examples of the BMP signal transduction pathway agonist include BMP proteins such as BMP2, BMP4, BMP7 etc., GDF proteins such as GDF7 etc., anti-BMP receptor antibody (agonist antibody), BMP partial peptides and so on. The BMP signal transduction pathway agonist is preferably BMP4.

The aforementioned Wnt signal transduction pathway agonist is a substance capable of enhancing signal transduction mediated by Wnt. Examples of the Wnt signal transduction pathway agonist include proteins belonging to the Wnt family (e.g., Wnt1, Wnt3A, Wnt7A, Wnt2B), Wnt receptor, Wnt receptor agonist, anti-Wnt receptor antibody, Wnt partial peptide, β catenin signal transmitter, GSK3β inhibitor (e.g., 6-Bromoindirubin-3'-oxime (BIO), CHIR99021, Kenpaullone) and the like.

The aforementioned SHH signal transduction pathway inhibitor is a substance capable of inhibiting signal transduction mediated by SHH. Examples of the SHH signal transduction pathway inhibitor include SHH receptor antagonist, SHH dominant-negative form, antibody against SHH signal transduction pathway agonist, soluble SHH receptor and the like. Specific examples of the SHH signal transduction pathway inhibitor include GANT58, GANT61, Jervine, SANT-1, Veratramine, Cyclopamine, Cyclopamine-KAAD (GENES & DEVELOPMENT 16:2743-2748) and the like. Preferred SHH signal transduction pathway inhibitor is, for example, Cyclopamine-KAAD.

In the present specification, the "ventralization signal transmitter" means a signal transmitter that promotes differentiation into a retinal tissue formed on the ventral side in the process of development. Examples of the ventralization signal transmitter include an SHH signal transduction pathway agonist that transmits a ventralization signal, and a BMP signal transduction pathway inhibitor that inhibits a dorsalization signal. A substance that inhibits a dorsalization signal is also referred to as a dorsalization signal transduction inhibitory substance.

The aforementioned SHH signal transduction pathway agonist is a substance capable of enhancing signal transduction mediated by SHH. The SHH signal transduction pathway agonist is not particularly limited as long as it is a substance capable of enhancing signal transduction mediated by SHH. Examples thereof include proteins belonging to the Hedgehog family (e.g., SHH and Ihh), SHH receptor, SHH receptor agonist, Purmorphamine, or SAG (Smoothened Agonist; N-Methyl-N'-(3-pyridinylbenzyl)-N'-(3-chlorobenzo[b]thiophene-2-carbonyl)-1,4-diaminocyclohexane) and the like. The SHH signal transduction pathway agonist is preferably SAG.

The SHH signal transduction promoting activity of SAG can be determined by a method well known to those of ordinary skill in the art by, for example, reporter gene assay focusing on the expression of GLI1 gene (Oncogene (2007) 26, 5163-5168).

The aforementioned BMP signal transduction pathway inhibitor is a substance capable of inhibiting the signal transduction mediated by BMP. As the BMP, BMP2, BMP4, BMP7 and GDF7 can be mentioned. Examples of the BMP signal transduction pathway inhibitor include a substance that directly acts on BMP (e.g., antibody, aptamer etc.), a substance that suppresses expression of a gene encoding BMP (e.g., antisense oligonucleotide, siRNA etc.), a substance that inhibits the binding of a BMP receptor (BMPR) and BMP (e.g., BMP receptor antagonist, BMP dominant-negative form, soluble BMP receptor etc.), and a substance that inhibits a physiological activity caused by signal transduction by BMP receptor. As BMPR, ALK2 and ALK3 can be mentioned. As BMP signal transduction pathway inhibitor, specifically, Noggin, Chordin, Dorsomorphin, LDN193189 (4-[6-(4-piperazin-1-ylphenyl)pyrazolo[1,5-a]pyrimidin-3-yl]quinoline) and the like can be mentioned. As a preferable BMP signal transduction pathway inhibitor, LDN193189 can be mentioned.

### 2. Method for increasing proportion of cone photoreceptor in photoreceptor contained in retinal tissue (production method of retinal tissue rich in cone photoreceptors)

When a retinal tissue in an initial developmental stage is cultured in a medium substantially free of a dorsalization signal transmitter, in some cases, expression of ventral marker increases, and emergence of photoreceptor precursor and/or cone photoreceptor precursor is suppressed in a ventral marker ALDH1A3-positive region. On the contrary, when cultured in a medium added with a dorsalization signal transmitter, the above-mentioned ALDH1A3-positive region sometimes decreases and emergence of photoreceptor precursor and/or cone photoreceptor precursor is promoted along therewith. On the other hand, when cultured in a medium added with a highly active dorsalization signal transmitter or a dorsalization signal transmitter at a high concentration, the strong dorsalization signal causes induction of a most dorsal marker COUP-TF II and ALDH1A1 that shows higher expression toward the dorsalization region of a neural retinal tissue, and differentiation induction into a cone photoreceptor precursor or cone photoreceptor may be suppressed. Therefore, induction of differentiation into a retinal tissue with a high proportion of cone photoreceptor precursor or cone photoreceptor requires appropriate adjustment of the intensity of the dorsalization signal, namely, the degree of dorsalization.

That is, one embodiment of the present invention is a method for increasing a proportion of a cone photoreceptor precursor and a cone photoreceptor in a photoreceptor precursor and a photoreceptor comprised in a retinal tissue, comprising a step of culturing a retinal tissue, in an initial developmental stage to a stage where an emergence rate of a cone photoreceptor precursor reaches maximum, in a medium comprising a dorsalization signal transmitter at a concentration sufficient to suppress expression of a ventral marker. The concentration of the dorsalization signal transmitter is preferably a concentration that does not induce expression of the most dorsal marker.

As the aforementioned "ventral marker", VAX2, COUP-TF I and ALDH1A3 can be specifically mentioned.

The above-mentioned "most dorsal marker" means a marker that is expressed in a cell present in the most dorsal region of a retinal tissue in the stage of development, and is also a marker that shows an increased expression by an excessive or relatively strong dorsalization signal. Specific examples of the most dorsal marker in a retinal tissue include RPE65, MITF or COUP-TF II expressed in a cell differentiated into a retinal pigment epithelial cell or a precursor thereof due to an excessive dorsalization signal, preferably COUP-TF II expressed in a neural retinal tissue due to a comparatively strong dorsalization signal and the like.

Here, the "concentration sufficient to suppress expression of a ventral marker" can be easily determined by those of ordinary skill in the art. For example, using a method such as immunostaining and the like using an antibody against the above-mentioned ventral marker and the like, a region showing expression of the above-mentioned ventral marker as compared to when a dorsalization signal transmitter is added is analyzed using image analysis software Image J and the like in a retinal tissue in any stage between a stage where a photoreceptor precursor first emerges and a stage where emergence rate of cone photoreceptor precursor reaches maximum, for example, a retinal tissue in a stage where emergence rate of cone photoreceptor precursor reaches maximum, and a concentration of the dorsalization signal transmitter at which a region showing suppression of the expression increases can be appropriately determined as a concentration appropriate for increasing the proportion of the cone photoreceptor. That is, the concentration of the dorsalization signal transmitter to be added can be determined such that a region showing expression of the above-mentioned ventral marker is not more than 50%, preferably not more than 20%, more preferably not more than 1%, further more preferably not more than 0.01%, in a neural retinal tissue containing photoreceptor precursor and/or photoreceptor. Alternatively, using as an index a retinal tissue ventralized by a ventralization signal transmitter BMP signal transduction pathway inhibitor and showing high expression of a ventral marker such as ALDH1A3 and the like, the concentration of the dorsalization signal transmitter to be added can be determined such that the gene expression level of ALDH1A3 is not more than 50%, preferably not more than 20%, more preferably not more than 5%, compared to the index.

The "concentration that does not induce most dorsal marker" can be appropriately determined by those of ordinary skill in the art. For example, using a method such as immunostaining and the like using an antibody against the above-mentioned most dorsal marker and the like, a region showing the expression of the above-mentioned most dorsal marker in a retinal tissue in a stage where the emergence rate of cone photoreceptor precursor reaches maximum is analyzed using image analysis software such as Image J and the like, and the concentration of the dorsalization signal transmitter is appropriately determined such that a region not showing induction of the expression of the most dorsal marker (wherein "not showing induction of expression" means not more than 1/5, preferably not more than 1/10, further preferably not more than 1/50, as compared to the expression level of the most dorsal neural retinal tissue in vivo) is not less than 50%, preferably not less than 80%, more preferably not less than 90%, further more preferably not less than 99%, of the neural retinal tissue.

One embodiment of the present invention is a method for increasing a proportion of a cone photoreceptor precursor and a cone photoreceptor in a photoreceptor precursor and a photoreceptor comprised in a retinal tissue, comprising a step of culturing a retinal tissue, in an initial developmental stage to a stage where an emergence rate of a cone photoreceptor precursor reaches maximum, in a medium comprising a dorsalization signal transmitter at a concentration sufficient to promote expression of a dorsal marker.

The "concentration sufficient to promote expression of a dorsal marker" can be easily determined by those of ordinary skill in the art. For example, it can be easily determined by analyzing the expression level of the above-mentioned dorsal marker protein or gene (mRNA). To be specific, various concentrations of a dorsalization signal transmitter is added to a medium, and a concentration at which the dorsal expression level or gene expression level becomes the highest can be determined by immunostaining or quantitative PCR.

For example, the concentration of the dorsalization signal transmitter can be determined to be a concentration at which the expression of CYP26A1 and/or CYP26C1 is most strongly induced. To be specific, it is a concentration at which the expression level of CYP26A1 is not less than 1.2 times, preferably not less than 1.5 times, more preferably not less than 2 times, compared to a retinal tissue in which ventralization is performed by the aforementioned BMP signal transduction pathway inhibitor and the expression of CYP26A1 is suppressed. When the concentration of the dorsalization signal transmitter is determined based on the expression level of CYP26A1 and/or CYP26C1, retinoic acids such as all trans retinoic acids, 9-cis retinoic acid and the like excessively induce expression of these genes irrespective of the degree of dorsalization, and make it difficult to determine the concentration of the dorsalization signal transmitter. Therefore, determination using a medium substantially free of retinoic acids is preferable.

On the other hand, in the case of ALDH1A1, ALDH1A1 is weakly expressed in the CYP26A1-positive region, and the expression level becomes continuously higher as it becomes closer to the dorsal side. That is, it is a marker showing a higher expression level in the COUP-TF II-positive region, which is most dorsal in the neural retinal tissues, as compared to CYP26A1 or CYP26C1-positive region. Accordingly, expression of ALDH1A1 is desirably not too high. To be specific, in a stage where emergence rate of cone photoreceptor precursor reaches maximum, the frequency of ALDH1A1-positive cell is not more than about 1%. That is, it is desirable to adjust the concentration of the dorsalization signal transmitter to a concentration that renders the expression of ALDH1A1 sufficiently low in this stage.

Here, the "concentration that renders the expression of ALDH1A1 sufficiently low" can be determined by those of ordinary skill in the art. For example, using a method such as immunostaining and the like conventionally performed using an antibody against ALDH1A1 and the like, a region showing the expression of ALDH1A1 in a retinal tissue in a stage where the emergence rate of cone photoreceptor precursor reaches maximum is analyzed using image analysis software such as Image J and the like, and a concentration at which the region is not more than 50%, preferably not more than 20%, more preferably not more than 10%, further more preferably not more than 1%, of a neural retinal tissue in the retinal tissue can be determined. Alternatively, such gene expression level of the retinal tissue can be measured by quantitative PCR and the like. That is, for example, when BMP4 at a comparatively high concentration of 0.45 nM - 1.35 nM is added, expression of ALDH1A1 is excessively induced. Accordingly, a concentration at which the expression level of ALDH1A1 is preferably 30%, more preferably 15%, further more preferably not more than 10%, of that when differentiation induction is performed by adding 1.35 nM BMP4 can be determined.

In one embodiment of the present invention, the "dorsalization signal transmitter at a concentration that suppresses expression of a ventral marker, and does not induce expression of the most dorsal marker" is a dorsalization signal transmitter at a concentration such that, in a retinal tissue in a stage where an emergence rate of cone photoreceptor precursor relative to the cells contained in the retinal tissue reaches maximum, the proportion of the number of cells that express OC2 relative to the total number of cells contained in the neural retinal tissue is suppressed to about 20% - 70%, preferably about 30% - 70%, more preferably about 50% - 65% (e.g., when 0.15 nM BMP4 or 500 nM Cyclopamine-KAAD is added, about 60% - 65% of cells express OC2) compared to that when a dorsalization signal transmitter is not added, or about 30% - 60%, preferably about 40% - 50% (e.g., when 0.15 nM BMP4 or 500 nM Cyclopamine-KAAD is added, about 45% - 47% of cells express OC2) compared to that when the aforementioned BMP signal transduction pathway inhibitor is added. Alternatively, a dorsalization signal transmitter at a concentration such that a OC2 protein expression level of OC2-positive cell is suppressed to about 20% - 70%, preferably about 30% - 70%, more preferably about 30% - 40% (e.g., about 34% - 36% when 0.15 nM BMP4 or 500 nM Cyclopamine-KAAD is added) compared to that when a dorsalization signal transmitter is not added and/or the aforementioned BMP signal transduction pathway inhibitor is added can be mentioned.

Here, the proportion of the number of OC2-positive cells contained in the neural retinal tissue can be determined by those of ordinary skill in the art by performing conventionally possible immunostaining and the like by using an anti OC2 antibody, DAPI and the like, measuring the number of OC2-positive cells, the number of DAPI-positive cells and the like, and identifying the ratio thereof contained in a neural retinal tissue. The expression level of OC2 protein can be determined by those of ordinary skill in the art by performing conventionally possible immunostaining and the like by using an anti OC2 antibody, DAPI and the like, and analyzing the region stained by an anti OC2 antibody by using image analysis software such as Image J and the like to identify the signal intensity of OC2-positive cells. Alternatively, the proportion of the number of OC2-positive cells contained in a neural retinal tissue and/or the OC2 protein expression level of OC2-positive cell may be identified by analysis using conventional flow cytometry using an antibody against OC2 protein.

A person skilled in the art can specify the above-mentioned "stage where photoreceptor precursor first emerges" by immunostaining with photoreceptor precursor marker and/or cone photoreceptor precursor marker, nuclear staining with DAPI and the like. Specifically, for example, the retinal tissue undergoing differentiation is fixed with para-formaldehyde and the like, and frozen sections are prepared. The frozen sections are stained with, for example, CRX antibody, TKβ2 antibody, RXR-γ antibody and the like, the nucleus is simultaneously stained with DAPI and the like, and the stage where photoreceptor precursor and/or cone photoreceptor precursor first emerge/emerges in the retinal tissue is identified.

A person skilled in the art can specify the above-mentioned "stage where emergence rate of cone photoreceptor precursor reaches maximum" by immunostaining with photoreceptor precursor marker and/or cone photoreceptor precursor marker, nuclear staining with DAPI and the like.

Specifically, for example, the retinal tissue undergoing differentiation is collected at certain intervals (e.g., 1-20 days) (e.g., 40 days, 50 days, 60 days, 70 days, 80 days after the start of culture), and fixed with para-formaldehyde and the like and frozen sections are prepared. The frozen sections are stained with, for example, CRX antibody, TRβ2 antibody, RXR-γ antibody and the like, the nucleus is simultaneously stained with DAPI and the like, and the proportion of the number of cone photoreceptor precursors (i.e., the number of CRX and RXR-γ, or cells expressing CRX and TRβ2) based on the total number of cells contained in the neural retinal tissue is determined. At this time, the ratio of the number of cone photoreceptor precursor marker-positive cells that emerge in the above-mentioned certain period to the total number of cells, that is, the emergence ratio, is determined at plural times (timing). As a result, the period when the proportion of emergence of cone photoreceptor precursor marker-positive cells is the highest can be specified as "a stage where emergence rate of cone photoreceptor precursor reaches maximum".

In addition, BrdU, EdU and the like which are incorporated into the cells in a proliferation period (here, retinal progenitor cell or neural retinal progenitor cell having proliferation ability) are added to a culture medium for a particular period (e.g., 1 - 7 days), the proportion of the cells that incorporated BrdU, EdU and the like and differentiated into cells that express the aforementioned cone photoreceptor precursor marker is measured by immunostaining and the like well known to those skilled in the art, and the relationship between the proportion and the differentiation stage (e.g., period (stage) when the proportion is the highest etc.) is determined, whereby "a stage where emergence rate of cone photoreceptor precursor reaches maximum" can be identified.

Specifically, for example, it can be identified by the following procedures:
1) a step of culturing by adding BrdU or Edu for any one day to a culture medium for cultivating a retinal tissue in any differentiation stage (e.g., adding BrdU every other day such as at 40 days - 41 days, 41 days - 42 days, 42 days - 43 days, from the start of culture, and culturing for 1 day is repeated for 80 days from the start of culture), recovering the retinal tissue immediately thereafter and measuring the proportion of CRX and RXR-γ-positive cells or the proportion of CRX and TRβ2-positive cells in BrdU or EdU-positive cells;
2) a step of comparing the measurement results, and identifying a retinal tissue in which a ratio of increase in CRX and RXR-γ-positive cells, or a ratio of increase in CRX and TRβ2-positive cells, in BrdU or EdU-positive cells is the highest; and
3) a step of identifying the period of addition of BrdU or EdU to the culture medium (e.g., 1 day) when culturing the retinal tissue in which a ratio of increase in CRX and RXR-γ-positive cells, or a ratio of increase in CRX and TRβ2-positive cells, in BrdU or EdU-positive cells is the highest as a "stage where emergence rate of cone photoreceptor precursor reaches maximum".

Specifically, the "stage where emergence rate of cone photoreceptor precursor reaches maximum" corresponds to 30 to 50 days, preferably 30 to 40 days, after emergence of cone photoreceptor precursor is observed.

In the present specification, the "initial developmental stage" means a stage where retinal progenitor cells have emerged but ganglion cells have not emerged. Here, neural retinal progenitor cells may have emerged. That is, in this stage, RX (RAX)-positive and PAX6-positive cells (and may further be CHX10-positive cells) are included, and TUJ1-positive cells, BRN3-positive cells and cells positive for at least two kinds of markers from tUJ1, BRN3 and PAX6 are not included. The "retinal tissue in an initial developmental stage" is not particularly limited as long as it includes retinal progenitor cells and/or neural retinal progenitor cell, that is, cells that can differentiate into photoreceptors and ganglion cells, and ganglion cells are not included. It may include a ciliary marginal zone structure.

When the retinal tissue in an initial developmental stage is produced in accordance with, for example, the starting material production methods 5 to 7 described later, it corresponds to day 22 (d22) to day 33 (d33) after the start of suspension culture. When the tissue is produced in accordance with the starting material production methods 1 to 4, it corresponds to day 12 (d12) to day 27 (d27) after the start of suspension culture.

The "retinal tissue in an initial developmental stage" can be identified by confirming the expression state of a retinal progenitor cell marker, a neural retinal progenitor cell, and a ganglion cell marker.

The retinal tissue in an initial developmental stage encompasses one corresponding to an "optic vesicle", or an retinal tissue at "the initial stage of an optic cup" containing an RX-positive, PAX6-positive and CHX10-positive neural retinal progenitor cell and free of a ganglion cell, which tissue shows differentiation somewhat progressed from optic vesicle.

In the present invention, the step of culturing in a medium containing a dorsalization signal transmitter (step (1)) may be performed in any period between the "initial developmental stage" and the "stage where emergence rate of cone photoreceptor precursor reaches maximum", and the timing of start of step (1) and period thereof are not limited as long as they are within this period. Step (1) is preferably started within about 40 days, more preferably 20 days, from the initial developmental stage, further more preferably in the initial developmental stage.

Examples of the retinal tissue in the initial developmental stage include a retinal tissue that is differentiated from pluripotent stem cells, contains retinal progenitor cells or neural retinal progenitor cells, and is in a differentiation stage in which ganglion cells have not emerged. Furthermore, a retinal tissue in an initial developmental stage may contain a cell that can differentiate into a photoreceptor or nerve cell.

A method for producing a retinal tissue in an initial developmental stage is not particularly limited, and may be a culture method by either suspension culture or adhesion culture. As this method, the methods described in WO 2013/077425 (& US2014/341864), WO 2015/025967 (& US2016/251616), WO 2016/063985 (& US2017/313976), WO 2016/063986 (& US2017/313981) and WO 2017/183732 and the like can be mentioned. Also, examples of this method include the methods described in non-patent document: Proc Natl Acad Sci USA. 111(23): 8518-8523(2014), Nat Commun. 5:4047(2014), Stem Cells. (2017):35(5), 1176-1188 and the like, and the like.

Specifically, an aggregate containing retinal progenitor cell or neural retinal progenitor cell, which is obtained by suspension culturing a cell aggregate (cell cluster) prepared from pluripotent stem cells such as ES cell, iPS cell and the like by the SFEBq method (see Nat Commun. 6:6286 (2015)) in the presence of, for example, a differentiation-inducer such as BMP4 and the like can be mentioned.

The retinal tissue in an initial developmental stage may be a cell induced from a cell population containing a neuroepithelial cell. The aforementioned cell population can also be obtained by inducing differentiation from pluripotent stem cells such as ES cell, iPS cell and the like, or by collecting stem cells present in an adult retina and inducing differentiation of same.

A retinal tissue in an initial developmental stage is specifically a retinal tissue containing retinal progenitor cell marker-positive (preferably, RX-positive and PAX6-positive) retinal progenitor cell, or a neural retinal progenitor cell marker-positive (preferably, CHX10-positive and PAX6-positive and RX-positive) neural retinal progenitor cell in a proportion of not less than 30%, preferably not less than 50%, more preferably not less than 80%, further preferably not less than 90%, further more preferably not less than 99%, of the total number of cells contained in the retinal tissue, and is a retinal tissue containing a ganglion cell marker-positive (preferably, BRN3-positive) ganglion cell in a proportion of not more than 40%, preferably not more than 20%, not more than 10%, not more than 5%, more preferably not more than 1%, further preferably not more than 0.1%, further more preferably not more than 0.01%, of the total number of cells.

The medium used when culturing the retinal tissue in an initial developmental state in the presence of the dorsalization signal transmitter is not particularly limited as long as it does not contain a substance that inhibits the effect of the dorsalization signal transmitter in an amount capable of inhibiting the aforementioned effect, and a medium obtained by appropriately adding an additive as necessary to a commercially available medium for cell culture can be used. The medium is preferably a medium capable of maintaining a continuous epithelial structure of a retinal tissue. A specifically usable medium includes, for example, a medium added with Wnt2b, Neurobasal medium, a medium containing Neurobasal medium and the like, and may be a Neurobasal medium added with Wnt2b. A medium for maintaining a continuous epithelial tissue explained below can be used.

In the present specification, the medium for maintaining a continuous epithelial tissue contains at least one of a methyl group donor or a substrate of the methyl group donor at a concentration at which cell differentiation of a retinal progenitor cell or a neural retinal progenitor cell can be suppressed, and a neurite extension inhibitor at a concentration at which neurite extension can be suppressed. Preferably, the medium for maintaining a continuous epithelial tissue contains a methyl group donor or a substrate of the methyl group donor at a concentration at which cell differentiation of a retinal progenitor cell or a neural retinal progenitor cell is suppressed, and a neurite extension inhibitor at a concentration at which neurite extension is suppressed.

In vivo, a methyl group is transferred from a methyl donor to a DNA or a protein including histone by methyltransferase. In the present specification, a methyl group donor is a substance (methyl donor) that can donate a methyl group to be transferred to a DNA or a protein including histone. In the present specification, the substrate of the methyl group donor is a substrate necessary for biosynthesis of the aforementioned methyl donor. Specifically, the methyl group donor is, for example, S-adenosylmethionine (to be also referred to as SAM), and the substrate of the methyl group donor is, for example, methionine, S-methyl 5'-thioadenosine (sometimes to be abbreviated as MTA), homocysteine (sometimes to be abbreviated as Hcy) or the like. In the present invention, methionine is preferably used.

To determine whether cell differentiation of retinal progenitor cell or neural retinal progenitor cell is suppressed, for example, an evaluation target substance is reacted with a retinal tissue in an initial developmental stage and containing retinal progenitor cell or neural retinal progenitor cell and, after culturing for a period of time, the proportion of retinal progenitor cells or neural retinal progenitor cells in the retinal tissue may be identified, or the proportion of differentiated cells or differentiated cells that have ceased growing in the retinal tissue may be identified. Specifically, for example, after a certain period of time (e.g., 5 days - 50 days) from the emergence of ganglion cells, a decrease rate of retinal progenitor cells or neural retinal progenitor cell; an increase rate of differentiated cells, cells that have stopped proliferating, or cells that express bHLH transcription factor necessary for terminal differentiation, each in the retinal tissue; or an expression level of bHLH transcription factor may be identified by immunostaining, quantitative PCR and the like. As a retinal progenitor cell or neural retinal progenitor marker, for example, a combination of RX and PAX6, a combination of RX, PAX6 and CHX10 or the like can be used. In addition, as a marker of differentiated cells contained in the retinal tissue, a marker of differentiated progenitor cells, or a marker of a bHLH transcription factor necessary for terminal differentiation, for example, BRN3, CRX, HuNu, Cath5, NeuroM, NGN1, NGN2, ISLET-1 (also referred to as ISL1), OLIG2 and the like can be used. As a marker for cells that have ceased cell proliferation due to cell differentiation, for example, p53, p27, p21 and the like can be used.

When methionine is used as a substrate of a methyl group donor, the concentration of methionine in the medium for maintaining a continuous epithelial tissue is generally more than 17.24 mg/L (preferably not less than 23.62 mg/L, not less than 25 mg/L, not less than 25.75 mg/L, not less than 26 mg/L, not less than 26.81 mg/L, not less than 27 mg/L, not less than 30 mg/L). The upper limit of the concentration of methionine in a medium for maintaining a continuous epithelial tissue is not particularly limited as long as maintenance of continuous epithelial tissue is achieved. It is generally not more than 100 mg/L (preferably not more than 75 mg/L). The range of methionine concentration in a medium for maintaining a continuous epithelial tissue is, for example, more than 17.24 mg/L and not more than 100 mg/L, preferably not less than 23.62 mg/L and not more than 75 mg/L, not less than 25 mg/L and not more than 75 mg/L, not less than 25.75 mg/L and not more than 75 mg/L, not less than 26 mg/L and not more than 75 mg/L, not less than 26.81 mg/L and not more than 75 mg/L, not less than 27 mg/L and not more than 75 mg/L, not less than 30 mg/L and not more than 75 mg/L. When a methyl group donor other than methionine or a substrate thereof is used, it is preferably used at a concentration affording an equivalent level of suppression of cell differentiation of retinal progenitor cell or neural retinal progenitor cell to that by the above-mentioned concentration of methionine.

In the present specification, the neurite extension inhibitor is a substance that suppresses neurite extension of ganglion cells. Specifically, neurite extension suppresses hormones such as glucocorticoid and the like, neurite extension suppressive proteins such as Semaphorin, Nogo, Mag, OMgp protein, chondroitin sulfate proteoglycan and the like, and the like can be mentioned. Examples of the glucocorticoid include corticosterone, cortisol, cortisone and the like. The neurite extension inhibitor is preferably glucocorticoid, more preferably corticosterone.

The neurite extension suppressive action can be evaluated by, for example, adhesion culture of retinal tissues or ganglion cells contained in the retinal tissue in the presence of an evaluation target substance, followed by measurement of the length of the extended neurite by using image analysis software (e.g., Image J) and the like.

When corticosterone is used as a neurite extension inhibitor, the concentration of corticosterone in a medium for maintaining a continuous epithelial tissue is a concentration that suppresses neurite extension of ganglion cell and the like contained in the retinal tissue. It is generally not less than 0.1 nM (preferably, not less than 1 nM, not less than 5 nM, not less than 10 nM, not less than 50 nM, not less than 100 nM). The upper limit of the concentration of corticosterone in a medium for maintaining a continuous epithelial tissue is not particularly limited as long as maintenance of continuous epithelial tissue is achieved. It is generally not more than 10 µM (preferably not more than 5 µM, not more than 1 µM). The range of corticosterone concentration in a medium for maintaining a continuous epithelial tissue is, for example, 0.1 nM - 10 µM, preferably, 1 nM - 5 µM. When the above-mentioned neurite extension inhibitor other than corticosterone is used, it is preferably used at a concentration affording an equivalent level of neurite extension suppressive action to that by the above-mentioned concentration of corticosterone.

In one embodiment, a medium for maintaining a continuous epithelial tissue contains methionine and corticosterone at the above-mentioned concentrations. In one embodiment, a medium for maintaining a continuous epithelial tissue contains more than 17.24 mg/L (preferably not less than 23.62 mg/L, not less than 25 mg/L, not less than 25.75 mg/L, not less than 26 mg/L, not less than 26.81 mg/L, not less than 27 mg/L, not less than 30 mg/L) of methionine and not less than 0.1 nM (preferably not less than 1 nM, more preferably not less than 5 nM, further preferably not less than 10 nM, further preferably not less than 50 nM, further preferably not less than 100 nM) of corticosterone.

The medium for maintaining a continuous epithelial tissue may further contain acidic amino acid, antioxidant and retinal neuron protection substance. The concentration of each of them is preferably lower.

In the present specification, specific examples of acidic amino acid include glutamic acid and aspartic acid and each encompasses L form and D form. In the present specification, glutamic acid in an L form is indicated as L-glutamic acid, aspartic acid in an L form is indicated as L-aspartic acid, glutamic acid in a D form is indicated as D-glutamic acid, and aspartic acid in a D form is indicated as D-aspartic acid. In the present specification, when the D form and L form are not distinguished, they are indicated as "glutamic acid" and "aspartic acid".

The concentration of L-glutamic acid in a medium for maintaining a continuous epithelial tissue is preferably less than 50 µM (more preferably not more than 25 µM, further preferably not more than 12.5 µM, further more preferably not more than 1 µM, particularly preferably not more than 0.1 µM). In one embodiment, the concentration of glutamic acid in a medium for maintaining a continuous epithelial tissue is preferably less than 50 µM (more preferably not more than 25 µM, further preferably not more than 12.5 µM, further more preferably not more than 1 µM, particularly preferably not more than 0.1 µM).

The concentration of L-aspartic acid in a medium for maintaining a continuous epithelial tissue is preferably less than 50 µM (more preferably not more than 25 µM, further preferably not more than 12.5 µM, further more preferably not more than 1 µM, particularly preferably not more than 0.1 µM). In one embodiment, the concentration of aspartic acid contained in a medium for maintaining a continuous epithelial tissue is preferably less than 50 µM (more preferably not more than 10 µM, further preferably not more than 1 µM, further preferably not more than 0.1 µM).

In one preferable embodiment, a medium for maintaining a continuous epithelial tissue contains at least one (preferably both) of more than 17.24 mg/L (preferably not less than 23.62 mg/L, not less than 25 mg/L, not less than 25.75 mg/L, not less than 26 mg/L, not less than 26.81 mg/L, not less than 27 mg/L, not less than 30 mg/L) of methionine and not less than 0.1 nM (preferably not less than 1 nM, more preferably not less than 5 nM, further preferably not less than 10 nM, further preferably not less than 50 nM, further preferably not less than 100 nM) of corticosterone, and the concentration of L-glutamic acid is less than 50 µM (more preferably not more than 25 µM, further preferably not more than 12.5 µM, further more preferably not more than 1 µM, particularly preferably not more than 0.1 µM).

In a more preferable embodiment, a medium for maintaining a continuous epithelial tissue contains at least one (preferably both) of more than 17.24 mg/L (preferably not less than 23.62 mg/L, not less than 25 mg/L, not less than 25.75 mg/L, not less than 26 mg/L, not less than 26.81 mg/L, not less than 27 mg/L, not less than 30 mg/L) of methionine and not less than 0.1 nM (preferably not less than 1 nM, not less than 5 nM, not less than 10 nM, not less than 50 nM, not less than 100 nM) of corticosterone, and the concentration of L-glutamic acid is less than 50 µM (more preferably not more than 25 µM, further preferably not more than 12.5 µM, further more preferably not more than 1 µM, particularly preferably not more than 0.1 µM) and the concentration of L-aspartic acid is preferably less than 50 µM (more preferably not more than 25 µM, further preferably not more than 12.5 µM, further more preferably not more than 1 µM, particularly preferably not more than 0.1 µM).

In the present specification, examples of the antioxidant include glutathione, catalase, Superoxide dismutase, alpha-tocopherol, cysteine and the like. In one embodiment, the concentration of at least one, preferably plural, more preferably all antioxidants selected from the group consisting of glutathione, catalase, Superoxide dismutase, alpha-tocopherol, and cysteine in a medium for maintaining a continuous epithelial tissue is within the following ranges:
glutathione: not more than 100 ng/mL (preferably not more than 10 ng/mL, more preferably not more than 1 ng/mL, further preferably not more than 0.1 ng/mL);
catalase: not more than 100 U/mL (preferably not more than 10 U/mL, more preferably not more than 1 U/mL, further preferably not more than 0.1 U/mL);
Superoxide dismutase: not more than 100 U/mL (preferably not more than 10 U/mL, more preferably not more than 1 U/mL, further preferably not more than 0.1 U/mL);
alpha-tocopherol: not more than 50 nM (preferably not more than 5 nM, more preferably not more than 0.5 nM, further preferably not more than 0.05 nM); and
cysteine: not more than 0.26 mM (preferably not more than 0.22 mM, more preferably not more than 0.18 mM, further preferably not more than 0.1 mM).

In one embodiment, the concentration of at least one, preferably plural, more preferably all antioxidants selected from the group consisting of glutathione, catalase, Superoxide dismutase, and alpha-tocopherol in a medium for maintaining a continuous epithelial tissue is a concentration that does not have an antioxidative action that influences the continuous epithelial structure, and the concentration of cysteine is not more than 0.26 mM (preferably not more than 0.22 mM, more preferably not more than 0.18 mM, further preferably not more than 0.1 mM). When other antioxidant is contained, the concentration thereof is preferably not more than a concentration that affords an antioxidative action equivalent to that of the above-mentioned antioxidant at the above-mentioned concentration, or a concentration that does not have an antioxidative action. The antioxidative action can be evaluated by directly measuring a part of active oxygen belonging to free radical by, for example, electron Spin resonance apparatus (Electron Spin Resonance, to be also referred to as ESR) in the presence of a spin trap agent. The antioxidative action can also be evaluated by other various methods for measuring active oxygen (e.g., measuring the amount of lipoperoxide produced due to active oxygen, the amount of 8-hydroxydeoxyguanosine or 8-nitroguanosine utilizable as oxidation stress markers and the like). For the measurement of the amount of active oxygen and the like, commercially available measurement kits (COSMO BIO, DOJINDO LABORATORIES, Thermo Fisher Scientific etc.) may be used.

In the present specification, examples of the retinal neuron protection substance include progesterone and the like. In one embodiment, the concentration of progesterone in a medium for maintaining a continuous epithelial tissue is not more than 100 nM, preferably not more than 50 nM, more preferably not more than 20 nM (or 6.3 µg/mL (20.033708 nM)), further preferably not more than 10 nM, further preferably not more than 3 nM. In one embodiment, the concentration of progesterone in a medium for maintaining a continuous epithelial tissue is a concentration that does not show a ganglion cell protective action. When other retinal neuron protection substance is contained, the concentration thereof is preferably not more than a concentration that affords a retinal neuron protective action equivalent to that of progesterone at the above-mentioned concentration, or a concentration that does not show a retinal neuron protective action. The retinal neuron protective action can be confirmed by, for example, identifying the proportion of ganglion cell contained in a retinal tissue, or the proportion of cleaved caspase-3 (known as an apoptosis marker)-positive ganglion cell, and identifying increase and decrease thereof. When the evaluation target substance shows a ganglion cell protective action, the proportion of the ganglion cells contained in a retinal tissue reacted with the substance for a given period increases and conversely, the proportion of the cleaved caspase-3-positive ganglion cells decreases compared to that without reaction with the substance. The proportion of the ganglion cells can be identified using immunohistostaining with antibody against the aforementioned ganglion cell markers (e.g., BRN3), DAPI staining, PI staining, Hoechst staining and the like.

In one preferable embodiment, a medium for maintaining a continuous epithelial tissue contains at least one (preferably both) of more than 17.24 mg/L (preferably not less than 23.62 mg/L, not less than 25 mg/L, not less than 25.75 mg/L, not less than 26 mg/L, not less than 26.81 mg/L, not less than 27 mg/L, not less than 30 mg/L) of methionine and not less than 0.1 nM (preferably not less than 1 nM, more preferably not less than 5 nM, further preferably not less than 10 nM, further preferably not less than 50 nM, further preferably not less than 100 nM) of corticosterone, the concentration of L-glutamic acid is less than 50 µM (more preferably not more than 25 µM, further preferably not more than 12.5 µM, further more preferably not more than 1 µM, particularly preferably not more than 0.1 µM), and further, the concentration of at least one, preferably plural, more preferably all compounds selected from the group consisting of L-aspartic acid, glutathione, catalase, Superoxide dismutase, alpha-tocopherol, cysteine and progesterone is within the following ranges:
L-aspartic acid: less than 50 µM (more preferably not more than 25 µM, further preferably not more than 12.5 µM, further more preferably not more than 1 µM, particularly preferably not more than 0.1 µM);
glutathione: not more than 100 ng/mL (preferably not more than 10 ng/mL, more preferably not more than 1 ng/mL, further preferably not more than 0.1 ng/mL);
catalase: not more than 100 U/mL (preferably not more than 10 U/mL, more preferably not more than 1 U/mL, further preferably not more than 0.1 U/mL);
Superoxide dismutase: not more than 100 U/mL (preferably not more than 10 U/mL, more preferably not more than 1 U/mL, further preferably not more than 0.1 U/mL);
alpha-tocopherol: not more than 50 nM (preferably not more than 5 nM, more preferably not more than 0.5 nM, further preferably not more than 0.05 nM);
cysteine: not more than 0.26 mM (preferably not more than 0.22 mM, more preferably not more than 0.18 mM, further preferably not more than 0.1 mM); and
progesterone: not more than 100 nM (preferably not more than 50 nM, more preferably not more than 20 nM (or 6.3 µg/mL (20.033708 nM)), further preferably not more than 10 nM, further preferably not more than 3 nM).

The medium for maintaining a continuous epithelial tissue may further contain hypoxanthine, thymidine and vitamin B12. The concentration of each of them is preferably lower.

In one embodiment, the concentration of hypoxanthine in a medium for maintaining a continuous epithelial tissue is, for example, less than 15 µM (preferably not more than 7.5 µM, more preferably not more than 3.75 µM, further preferably not more than 1 µM, further more preferably not more than 0.1 µM (e.g., 0 µM)).

In one embodiment, the concentration of thymidine in a medium for maintaining a continuous epithelial tissue is less than 1.5 µM (preferably not more than 0.75 µM, more preferably not more than 0.375 µM, further preferably not more than 0.1 µM, further more preferably not more than 0.01 µM (e.g., 0 µM)).

In one embodiment, the concentration of vitamin B12 in a medium for maintaining a continuous epithelial tissue is less than 0.5 µM (preferably not more than 0.253 µM, more preferably not more than 0.129 µM, further preferably not more than 0.005 µM).

In a preferable embodiment, the concentration of 2 or 3 compounds selected from the group consisting of hypoxanthine, thymidine and vitamin B12 in a medium for maintaining a continuous epithelial tissue is within the aforementioned ranges.

In one preferable embodiment, a medium for maintaining a continuous epithelial tissue contains at least one (preferably both) of more than 17.24 mg/L (preferably not less than 23.62 mg/L, not less than 25 mg/L, not less than 25.75 mg/L, not less than 26 mg/L, not less than 26.81 mg/L, not less than 27 mg/L, not less than 30 mg/L) of methionine and not less than 0.1 nM (preferably not less than 1 nM, more preferably not less than 5 nM, further preferably not less than 10 nM, further preferably not less than 50 nM, further preferably not less than 100 nM) of corticosterone, the concentration of L-glutamic acid is less than 50 µM (more preferably not more than 25 µM, further preferably not more than 12.5 µM, further more preferably not more than 1 µM, particularly preferably not more than 0.1 µM), and the concentration of at least one, preferably plural, more preferably all compounds selected from the group consisting of L-aspartic acid, glutathione, catalase, Superoxide dismutase, alpha-tocopherol, cysteine, progesterone, hypoxantine, thymidine and vitamin B12 is within the following ranges:
L-aspartic acid: less than 50 µM (more preferably not more than 25 µM, further preferably not more than 12.5 µM, further more preferably not more than 1 µM, particularly preferably not more than 0.1 µM);
glutathione: not more than 100 ng/mL (preferably not more than 10 ng/mL, more preferably not more than 1 ng/mL, further preferably not more than 0.1 ng/mL);
catalase: not more than 100 U/mL (preferably not more than 10 U/mL, more preferably not more than 1 U/mL, further preferably not more than 0.1 U/mL);
Superoxide dismutase: not more than 100 U/mL (preferably not more than 10 U/mL, more preferably not more than 1 U/mL, further preferably not more than 0.1 U/mL);
alpha-tocopherol: not more than 50 nM (preferably not more than 5 nM, more preferably not more than 0.5 nM, further preferably not more than 0.05 nM);
cysteine: not more than 0.26 mM (preferably not more than 0.22 mM, more preferably not more than 0.18 mM, further preferably not more than 0.1 mM);
progesterone: not more than 100 nM (preferably not more than 50 nM, more preferably not more than 20 nM (or 6.3 µg/mL (20.033708 nM)), further preferably not more than 10 nM, further preferably not more than 3 nM);
hypoxanthine: less than 15 µM (preferably not more than 7.5 µM, more preferably not more than 3.75 µM, further preferably not more than 1 µM, further more preferably not more than 0.1 µM (e.g., 0 µM));
thymidine: less than 1.5 µM (preferably not more than 0.75 µM, more preferably not more than 0.375 µM, further preferably not more than 0.1 µM, further more preferably not more than 0.01 µM (e.g., 0 µM)); and
vitamin B12: less than 0.5 µM (preferably not more than 0.253 µM, more preferably not more than 0.129 µM, further preferably not more than 0.005 µM).

In one preferable embodiment, a medium for maintaining a continuous epithelial tissue has the following composition:
methionine: more than 17.24 mg/L (preferably not less than 23.62 mg/L, not less than 25 mg/L, not less than 25.75 mg/L, not less than 26 mg/L, not less than 26.81 mg/L, not less than 27 mg/L, not less than 30 mg/L);
corticosterone: not less than 0.1 nM (preferably not less than 1 nM, more preferably not less than 5 nM, further preferably not less than 10 nM, further preferably not less than 50 nM, further preferably not less than 100 nM);
L-glutamic acid: less than 50 µM (more preferably not more than 25 µM, further preferably not more than 12.5 µM, further more preferably not more than 1 µM, particularly preferably not more than 0.1 µM);
L-aspartic acid: less than 50 µM (more preferably not more than 25 µM, further preferably not more than 12.5 µM, further more preferably not more than 1 µM, particularly preferably not more than 0.1 µM);
hypoxanthine: less than 15 µM (preferably not more than 7.5 µM, more preferably not more than 3.75 µM, further preferably not more than 1 µM, further more preferably not more than 0.1 µM (e.g., 0 µM));
thymidine: less than 1.5 µM (preferably not more than 0.75 µM, more preferably not more than 0.375 µM, further preferably not more than 0.1 µM, further more preferably not more than 0.01 µM (e.g., 0 µM)); and
vitamin B12: less than 0.5 µM (preferably not more than 0.253 µM, more preferably not more than 0.129 µM, further preferably not more than 0.005 µM).

In the embodiment, the concentration of at least one, preferably plural, more preferably all compounds selected from the group consisting of glutathione, catalase, Superoxide dismutase, alpha-tocopherol, L-cysteine and progesterone is within the following ranges:
glutathione: not more than 100 ng/mL (preferably not more than 10 ng/mL, more preferably not more than 1 ng/mL, further preferably not more than 0.1 ng/mL);
catalase: not more than 100 U/mL (preferably not more than 10 U/mL, more preferably not more than 1 U/mL, further preferably not more than 0.1 U/mL);
Superoxide dismutase: not more than 100 U/mL (preferably not more than 10 U/mL, more preferably not more than 1 U/mL, further preferably not more than 0.1 U/mL);
alpha-tocopherol: not more than 50 nM (preferably not more than 5 nM, more preferably not more than 0.5 nM, further preferably not more than 0.05 nM);
cysteine: not more than 0.26 mM (preferably not more than 0.22 mM, more preferably not more than 0.18 mM, further preferably not more than 0.1 mM); and
progesterone: not more than 100 nM (preferably not more than 50 nM, more preferably not more than 20 nM (6.3 µg/mL), further preferably not more than 10 nM, further preferably not more than 3 nM).

A medium for maintaining a continuous epithelial tissue can be prepared by appropriately blending a commercially available medium.

Examples of the basal medium that can be used for preparing a medium for maintaining a continuous epithelial tissue include a medium not containing at least one of the aforementioned acidic amino acid, antioxidant and retinal neuron protection substances such as progesterone and the like (e.g., acidic amino acid), preferably two or more (e.g., acidic amino acid and any other substance), more preferably not containing all, or within the aforementioned concentration ranges. In one embodiment of the basal medium, one, preferably two, more preferably three, of hypoxanthine, thymidine and vitamin B12 are within the aforementioned concentration ranges. In one embodiment of the basal medium, hypoxanthine and thymidine are not contained, and the concentration of vitamin B12 is within the aforementioned concentration range.

As a basal medium that can be used for the preparation of a medium for maintaining a continuous epithelial tissue, a medium in which the concentration of at least one of methyl group donor (e.g., S-adenosylmethionine), substrate of methyl group donor (e.g., methionine, MTA, Hcy) and neurite extension inhibitor (e.g., corticosterone) is in the aforementioned concentration range is preferable. A medium for maintaining a continuous epithelial tissue can be prepared by appropriately supplementing necessary substances to the basal medium that fall within the above-mentioned concentration ranges.

The basal medium that can be used for the preparation of a medium for maintaining a continuous epithelial tissue may be appropriately selected from commercially available basal media according to the above-mentioned selection criteria and based on the ingredient table published by the manufacturer. Examples of the basal medium that can be used for the preparation of a medium for maintaining a continuous epithelial tissue include commercially available Neurobasal medium (including Neurobasal-A medium, phenol red-free Neurobasal medium and the like), Improved MEM Zinc Option medium, MEM, DMEM, Leibovitz's L-15, E-MEM, G-MEM and the like. In addition, it is also possible to order and purchase a medium with individual customized components to and from a medium manufacturer. Also, a medium customized according to the aforementioned description to be a basal medium usable for the preparation of a medium for maintaining a continuous epithelial tissue may be used.

A supplemental medium may be appropriately blended to supplement corticosterone. Specific examples of the supplemental medium include B27 supplement and the like. As one embodiment of the medium for maintaining a continuous epithelial tissue, specifically, a medium in which B27 supplement is blended with Neurobasal medium can be mentioned. The medium may contain L-glutamine, taurine, serum and the like as appropriate

Neurobasal medium is a known basal medium developed for nerve cell culture (J. Neurosci. Res., vol. 35, p. 567-576, 1993). While Neurobasal medium has been partially modified from the report, it is available as a commercially available Neurobasal medium from a medium manufacturer (e.g., manufactured by Thermo Fisher Scientific, 21103049). The composition of Neurobasal medium (21103049) available from Thermo Fisher Scientific does not contain acidic amino acids (L-glutamic acid and L-aspartic acid), progesterone, hypoxanthine or thymidine, has high methionine concentration (30 mg/L), high cysteine concentration (0.26 mM), and low vitamin B12 concentration (0.005 µM) compared to DMEM/F12. The specific composition is as follows.

**[Table 1-1]**

| **Components** | **Molecular Weight** | **Concentration (mg/L)** | **mM** |
|---|---|---|---|
| Amino Acids | | | |
| Glycine | 75 | 30 | 0.4 |
| L-Alanine | 89 | 2 | 0.02247191 |
| L-Arginine hydrochloride | 211 | 84 | 0.39810428 |
| L-Asparagine-H₂O | 150 | 0.83 | 0.005533333 |
| L-Cysteine | 121 | 31.5 | 0.2603306 |
| L-Histidine hydrochloride-H₂O | 210 | 42 | 0.2 |
| L-Isoleucine | 131 | 105 | 0.8015267 |
| L-Leucine | 131 | 105 | 0.8015267 |
| L-Lysine hydrochloride | 183 | 146 | 0.7978142 |
| L-Methionine | 149 | 30 | 0.20134228 |
| L-Phenylalanine | 165 | 66 | 0.4 |
| L-Proline | 115 | 7.76 | 0.06747826 |
| L-Serine | 105 | 42 | 0.4 |
| L-Threonine | 119 | 95 | 0.79831934 |
| L-Tryptophan | 204 | 16 | 0.078431375 |
| L-Tyrosine | 181 | 72 | 0.39779004 |
| L-Valine | 117 | 94 | 0.8034188 |

| Vitamins | | | |
|---|---|---|---|
| Choline chloride | 140 | 4 | 0.028571429 |
| D-Calcium pantothenate | 477 | 4 | 0.008385744 |
| Folic Acid | 441 | 4 | 0.009070295 |
| Niacinamide | 122 | 4 | 0.032786883 |
| Pyridoxal hydrochloride | 204 | 4 | 0.019607844 |
| Riboflavin | 376 | 0.4 | 0.00106383 |
| Thiamine hydrochloride | 337 | 4 | 0.011869436 |
| Vitamin B12 | 1355 | 0.0068 | 0.00000502 |
| i-Inositol | 180 | 7.2 | 0.04 |

**[Table 1-2]**

| **Inorganic Salts** | | | |
|---|---|---|---|
| Calcium Chloride (CaCl₂) (anhyd.) | 111 | 200 | 1.8018018 |
| Ferric Nitrate (Fe(NO₃)₃-9H₂O) | 404 | 0.1 | 0.000248 |
| Magnesium Chloride (anhydrous) | 95 | 77.3 | 0.8136842 |
| Potassium Chloride (KCl) | 75 | 400 | 5.3333335 |
| Sodium Bicarbonate (NaHCO₃) | 84 | 2200 | 26.190475 |
| Sodium Chloride (NaCl) | 58 | 3000 | 51.724136 |
| Sodium Phosphate monobasic (NaH₂PO₄-H₂O) | 138 | 125 | 0.9057971 |
| Zinc sulfate (ZnSO₄-7H₂O) | 288 | 0.194 | 0.000674 |

| **Other Components** | | | |
|---|---|---|---|
| D-Glucose (Dextrose) | 180 | 4500 | 25 |
| HEPES | 238 | 2600 | 10.92437 |
| Phenol Red | 376.4 | 8.1 | 0.021519661 |
| Sodium Pyruvate | 110 | 25 | 0.22727273 |

B27 supplement is a known supplemental medium developed for nerve cell culture (J. Neurosci. Res., vol. 35, p. 567-576, 1993). B27 supplement is generally used by adding to a basal medium such as Neurobasal medium and the like at a volume ratio of about 2%. B27 supplement containing corticosterone and combined with Neurobasal medium can be used as the medium for maintaining a continuous epithelial tissue. For example, B27 supplement containing corticosterone is added to a basal medium containing methionine (Neurobasal medium etc.) such that the aforementioned methionine concentration and corticosterone concentration would be achieved, whereby the medium for maintaining a continuous epithelial tissue can be prepared.

B27 supplement (J. Neurosci. Res., vol. 35, p. 567-576, 1993) can be purchased from, for example, a medium manufacturer (e.g., Thermo Fisher Scientific, 12587010), and the composition is as follows.

**[Table 2]**

| **Components** | **mM** |
|---|---|
| **Vitamins** | |
| Biotin | n/a |
| DL Alpha Tocopherol Acetate | n/a |
| DL Alpha-Tocopherol | n/a |

| **Proteins** | |
|---|---|
| BSA, fatty acid free Fraction V | n/a |
| Catalase | n/a |
| Human Recombinant Insulin | n/a |
| Human Transferrin | n/a |
| Superoxide Dismutase | n/a |

| **Other Components** | |
|---|---|
| Corticosterone | n/a |
| D-Galactose | n/a |
| Ethanolamine HCl | n/a |
| Glutathione (reduced) | n/a |
| L-Carnitine HCl | n/a |
| Linoleic Acid | n/a |
| Linolenic Acid | n/a |
| Progesterone | n/a |
| Putrescine 2HCl | n/a |
| Sodium Selenite | n/a |
| T3 (triodo-I-thyronine) | n/a |

In another embodiment, the medium for maintaining a continuous epithelial tissue includes a medium containing Neurobasal medium supplemented with B27 supplement at a volume ratio of one or more (preferably two or more, more preferably three or more) based on the cell proliferation basal medium (e.g., DMEM/F12 mixed medium (DMEM:F12=1:1)).

The cell proliferation basal medium is not particularly limited and a commercially available basal medium can be used alone or in an appropriate mixture. The cell proliferation basal medium may contain an appropriately additive (supplement), and a specific supplement is, for example, N2 supplement.

The concentration of L-methionine, L-glutamic acid, L-aspartic acid, L-cysteine, hypoxanthine, thymidine, and vitamin B12 in a medium obtained by adding B27 supplement (Thermo Fisher Scientific, 12587010) to the above-mentioned commercially available Neurobasal medium (Thermo Fisher Scientific, 21103049), and a medium obtained by mixing a medium obtained by adding B27 supplement to Neurobasal medium and a medium obtained by adding N2 supplement to DMEM/F12 mixed medium (DMEM:F12=1:1) at a ratio of 3:1, 2:1 or 1:1 is, for example, as shown below.

**[Table 3]**

| | Neurobasal + B27 | (1) Neurobasal + B27 (2) DMEM/F12 + N2 (1) : (2) =3:1 | (1) Neurobasal + B27 (2) DMEM/F12 + N2 (1) : (2) =2:1 | (1) Neurobasal + B27 (2) DMEM/F12 + N2 (1) : (2) =1:1 |
|---|---|---|---|---|
| L-methionine | 30 mg/L | 26.81 mg/L | 25.75 mg/L | 23.62 mg/L |
| L-glutamic acid | 0 µM | 12.5 µM | 16.67 µM | 25 µM |
| L-aspartic acid | 0 µM | 12.5 µM | 16.67 µM | 25 µM |
| L-cysteine | 31.5 mg/L (0.26 mM) | 28.015 mg/L (0.22 mM) | 26.853 mg/L (0.207 mM) | 24.53 mg/L (0.135 mM) |
| hypoxanthine | 0 µM | 3.75 µM | 5 µM | 7.5 µM |
| thymidine | 0 µM | 0.375 µM | 0.5 µM | 0.75 µM |
| vitamin B12 | 6.8 µg/L (0.005 µM) | 175.1 µg/L (0.12875 µM) | 231.2 µg/L (0.17 µM) | 343.4 µg/L (0.275 µM) |

In one embodiment, a medium having a composition of L-methionine, L-glutamic acid, L-aspartic acid, L-cysteine, hypoxanthine, thymidine, and vitamin B12 at concentrations equivalent to those shown in Table 3 can be used as a medium for maintaining a continuous epithelial tissue. Here, the "equivalent concentration" independently means that it includes the range of ±20% (preferably ±10%, more preferably ±5%, further preferably ±2.5%, and further more preferably ±1%) for the concentration of each factor.

In one embodiment, a medium having a composition of L-methionine, L-glutamic acid, L-aspartic acid, hypoxanthine, thymidine, and vitamin B12 at concentrations equivalent to those shown in Table 3, wherein the concentration of at least one, preferably plural, more preferably all compounds selected from the group consisting of corticosterone, glutathione, catalase, Superoxide dismutase, alpha-tocopherol, L-cysteine and progesterone is within the following ranges can be used as a medium for maintaining a continuous epithelial tissue:
corticosterone: not less than 0.1 nM (preferably not less than 1 nM, more preferably not less than 5 nM, further preferably not less than 10 nM, further preferably not less than 50 nM, further preferably not less than 100 nM);
glutathione: not more than 100 ng/mL (preferably not more than 10 ng/mL, more preferably not more than 1 ng/mL, further preferably not more than 0.1 ng/mL);
catalase: not more than 100 U/mL (preferably not more than 10 U/mL, more preferably not more than 1 U/mL, further preferably not more than 0.1 U/mL);
Superoxide dismutase: not more than 100 U/mL (preferably not more than 10 U/mL, more preferably not more than 1 U/mL, further preferably not more than 0.1 U/mL);
alpha-tocopherol: not more than 50 nM (preferably not more than 5 nM, more preferably not more than 0.5 nM, further preferably not more than 0.05 nM);
cysteine: not more than 0.26 mM (preferably not more than 0.22 mM, more preferably not more than 0.18 mM, further preferably not more than 0.1 mM); and
progesterone: not more than 100 nM (preferably not more than 50 nM, more preferably not more than 20 nM (or not more than 6.3 µg/mL (20.033708 nM)), further preferably not more than 10 nM, further preferably not more than 3 nM).

The medium for maintaining a continuous epithelial tissue may contain L-glutamine, taurine, N2 and the like as appropriate. The concentration of taurine is generally 1 µM - 1000 µM, preferably 10 µM - 500 µM. When N2 is contained, it is more preferable to add glucocorticoid such as corticosterone and the like at the aforementioned concentration without adding B27.

The medium for maintaining a continuous epithelial tissue may contain, as long as continuous epithelial tissues can be maintained, one or more additives appropriately selected from, though not limited to, adjusting agents such as buffering agent (e.g., HEPES), salt (e.g., inorganic salt such as sodium chloride, sodium hydrogen carbonate and the like) or antioxidant (e.g., 2-mercaptoethanol, antioxidant is not contained in one embodiment) and the like, nutritional supplements such as amino acid (e.g., non-essential amino acid, acidic amino acid is not contained in one embodiment), fatty acid, sugar, vitamin, lipid, pyruvic acid and the like, antibiotic (e.g., penicillin, streptomycin), extracellular matrix (e.g., Matrigel, laminin, laminin fragment, laminin511-E8 fragment), dye (e.g., phenol red) and the like, which are generally contained in a medium.

The medium for maintaining a continuous epithelial tissue may be either a serum-containing medium or a serum-free medium. It is preferably a serum-containing medium. The concentration of serum in the serum-containing medium is generally 0.1 - 20%(v/v), preferably 0.1 - 12% (v/v) (e.g., 100(v/v)). In the present specification, the concentration change of the composition contained in the medium due to the addition of serum at a concentration of 0.1 - 20% (v/v) is not to be considered.

The aforementioned medium may or may not contain retinoids (e.g., retinoic acid or a derivative thereof), but in one embodiment, it may contain 9-cis retinoic acid. A more preferred embodiment is a medium that substantially does not contain retinoids, preferably retinoids biosynthesized by ALDH1A3 and which inhibits differentiation of cone photoreceptor precursor. Specific examples of the retinoids that are biosynthesized by ALDH1A3 and inhibit differentiation of cone photoreceptor precursor include all-trans retinoic acid (to be also referred to as atRA) and the like.

The "continuous epithelial structure" in the retinal tissue means a structure in which the retinal tissue has an apical surface unique to the epithelial tissue and the apical surface is formed on the surface of the retinal tissue continuously and generally parallel to at least photoreceptor layer (outer nuclear layer) or neuroblastic layer among the respective layers forming the neural retinal layer. That is, the continuous epithelial structure does not have a structure seen in a rosette-like structure in which the apical surface is divided. For example, in the case of a cell aggregate containing a retinal tissue prepared from pluripotent stem cells, the apical surface is formed on the surface of the aggregate, and not less than 10, preferably not less than 30, more preferably not less than 100, further preferably not less than 400 photoreceptor or photoreceptor precursors are regularly and continuously arranged in the tangential direction with respect to the surface. The number of photoreceptors or photoreceptor precursors arranged continuously correlates with the size of the neural retinal tissue contained in the cell aggregate. In the present specification, the tangential direction with respect to the epithelial tissue refers to a direction in which every single cell forming the apical surface in the epithelial tissue is arranged and it is a parallel direction or transverse direction relative to the epithelial tissue (or epithelial sheet).

In one embodiment, an apical surface is formed on the surface of the neural retinal tissue, and photoreceptors or photoreceptor precursors are regularly and continuously arranged along the apical surface.

In the case of a retinal tissue in a stage where the emergence rate of photoreceptors or photoreceptor precursors is low (e.g., retinal tissue in an initial developmental stage), it is known to those of ordinary skill in the art that the layer containing proliferating neural retinal progenitor cell is called a "neuroblastic layer". In addition, the surface of a retinal tissue in such stage may sometimes contain, in addition to photoreceptors and photoreceptor precursors, a neural retinal progenitor cell that has polarity and can form the apical surface, a cell that divides and proliferates from the neural retinal progenitor cell and/or a cell in a stage of differentiation from a neural retinal progenitor cell into a cell constituting the neural retina. By culturing the retinal tissue in such a state under the conditions that maintain the "continuous epithelial structure", a retinal tissue in which photoreceptors or photoreceptor precursors are regularly and continuously arranged along the apical surface formed on the surface of the neural retinal tissue is obtained.

In one embodiment, the area of the apical surface present on the surface of the retinal tissue is not less than 30%, preferably not less than 50%, more preferably not less than 80%, further more preferably not less than 95%, on average relative to the surface area of the retinal tissue. The area of the apical surface present on the surface of the retinal tissue can be measured as described below by staining an apical surface marker.

In the present specification, the "rosette-like structure" in the retinal tissue refers to a structure in which cells are arranged radially or spirally while surrounding a central lumen. In the retinal tissue in which the rosette-like structure is formed, the apical surface and photoreceptors or photoreceptor precursors exist along the center (lumen), and the apical surface is divided for each rosette-like structure.

In the present specification, the "apical surface" refers to a 50-100 nm surface (surface layer) formed on the side opposite from the basement membrane side where the layer (basement membrane) produced by epithelial cell is present in an epithelial tissue. The surface is rich in mucopolysaccharide (positive for PAS staining) and contains a large amount of laminin and type IV collagen. In one embodiment, in a retinal tissue whose developmental stage has progressed to the extent that photoreceptors or photoreceptor precursors are observed, an outer limiting membrane formed and it refers to a surface in contact with a photoreceptor layer (outer nuclear layer) in which photoreceptors and photoreceptor precursors are present. Such apical surface can be identified by an immunostaining method or the like well known to those skilled in the art and by using an antibody against an apical surface marker (e.g., atypical-PKC (hereinafter sometimes to be abbreviated as aPKC), E-cadherin, N-cadherin). In an initial developmental stage, even when photoreceptor or photoreceptor precursor has not emerged or even when photoreceptor or photoreceptor precursor has not emerged to sufficiently cover the surface of retinal tissue, the epithelial tissue has polarity, and the apical surface expresses the above-mentioned apical surface marker.

Whether or not the retinal tissue has a continuous epithelial structure can be confirmed by the continuity of the apical surface of the retinal tissue (that is, an undivided form). The continuity of the apical surface is determined by, for example, immunostaining a marker on the apical surface (e.g., aPKC, E-cadherin, N-cadherin), a marker for photoreceptor or photoreceptor precursor located on the apical surface side (e.g., Crx or recoverin) and analyzing the obtained images and the like for positional relationship between the apical surface, the photoreceptor layer, and each retinal layer. As for retinal layers other than the apical surface and photoreceptor layer (outer nuclear layer), the continuity can be identified by DAPI staining PI staining, Hoechst staining, each including staining of the cell nucleus, or immunostaining with a marker protein (Rx, Chx10, Ki67, Crx etc.) or the like localized in the cell nucleus.

Whether or not a rosette-like structure was generated can be determined by fixing cell aggregates with 4% paraformaldehyde and so on, preparing frozen sections, and observing dysplasia (e.g., divided apical surface or invasion of apical surface into cell aggregate) of rosette-like structure by immunostaining or the like that is generally performed using antibodies against apical surface markers aPKC, E-cadherin, N-cadherin, and DAPI that specifically stains nucleus and the like.

In one embodiment of the present invention, an aggregate containing a retinal tissue having a continuous epithelial structure, namely, an aggregate containing a retinal tissue having a photoreceptor or a progenitor cell thereof continuously present in not less than at least 50% (preferably not less than 60%, not less than 70%, not less than 80%, not less than 85%, not less than 90%) of a surface of the retinal tissue can be mentioned. In addition, a preparation containing the aggregate and a medium for maintaining a continuous epithelial tissue is also within the scope of the present invention.

As one embodiment of the retinal tissue having a continuous epithelial structure of the present invention, an aggregate containing a retinal tissue in which the area of the apical surface present on the surface of the retinal tissue is not less than at least 50% (preferably not less than 60%, not less than 70%, not less than 80%, not less than 85%, not less than 90%) based on the surface area of the retinal tissue can be mentioned. In one embodiment of the present invention, the diameter in the major axis direction is not less than 0.5 mm (preferably not less than 0.6 mm, not less than 0.8 mm, not less than 1.0 mm, not less than 1.2 mm, not less than 1.4 mm, not less than 1.6 mm, not less than 1.8 mm, not less than 2.0 mm). An aggregate containing a retinal tissue having a continuous epithelial structure can be mentioned. A retinal tissue to be transplanted is preferably large since a wide area of a retinal tissue of a recipient with a disorder can be covered. Generally, a retinal tissue exceeding 0.5 mm - 1.0 mm easily produces a rosette structure during transplantation. However, the present invention can provide an aggregate containing a retinal tissue having a continuous epithelial structure, without producing a rosette structure even with a retinal tissue exceeding 0.5 mm - 1.0 mm. The structure of the retinal tissue is as described in the below-mentioned "5. Retinal tissue with high proportion of cone photoreceptor" or "6. Retinal tissue with high proportion of rod photoreceptor".

Here, the diameter in the major axis direction of a retinal tissue means, for example, when measuring based on images taken with a stereomicroscope, the length of the longest straight line connecting any two points in the outer circumference (contour, surface) of the retinal tissue. Some aggregates containing a retinal tissue contain multiple retinal tissues overlapping with one another (e.g., clover type). A person skilled in the art can easily determine whether multiple retinal tissues are present. In this case, the diameter in the major axis direction of the retinal tissue means the diameter in the major axis direction of each retinal tissue in the aggregate, and the diameter in the major axis direction of at least one retinal tissue only needs to be not less than 0.5 mm. Preferably, the diameters in the major axis direction of all retinal tissues in the aggregate are not less than 0.5 mm. More specifically, the length of the longest straight line connecting any two points in the outer circumference separated by two overlapping circles or ellipses in terms of shape (more specifically, when the continuous positional information of the outer circumference of the aggregate containing a retinal tissue is hypothetically determined, the point at which continuity of the curve is lost in the curve obtained when the positional information is plotted on the horizontal axis and the slope of the tangent line at the position is plotted on the vertical axis) is measured. Furthermore, some aggregates containing a retinal tissue contain a retinal pigment epithelial cell and/or a ciliary marginal zone. Also in this case, similar to when multiple retinal tissues are present in an aggregate, the length of the longest straight line connecting any two points in the outer circumference separated by a contact point between a retinal pigment epithelium and/or a ciliary marginal zone and a retinal tissue is measured.

The proportion of the cells expressing RAX, CHX10 and/or CRX in a retinal tissue is preferably not less than 50%, not less than 60%, not less than 70%, not less than 80%, not less than 85%, not less than 90%, not less than 95%.

When the aggregate contains multiple retinal tissues, the proportion of the number of aggregates containing a retinal tissue satisfying the above-mentioned conditions based on the whole number is preferably at least not less than 50% (preferably not less than 60%, not less than 70%, not less than 80%, not less than 85%, not less than 90%, not less than 95%).

An aggregate containing the aforementioned any retinal tissue having a continuous epithelial structure can be formed such that the apical surface of the surface of the aggregate is in contact with the medium side.

The medium for maintaining a continuous epithelial tissue contained in the preparation containing the aggregate and the medium is a medium for maintaining a continuous epithelial tissue defined in the present specification. It may contain, for example, antibiotic, antiseptic, stabilizer, preservative and the like as long as a continuous epithelial tissue can be maintained.

The concentration of the dorsalization signal transmitter contained in the medium may be a concentration affording a BMP signal transduction pathway activating action that (preferably) does not suppress emergence of a cone photoreceptor precursor. The concentration of a dorsalization signal transmitter that does not suppress emergence of a cone photoreceptor precursor can be appropriately set by the proportion of a cone photoreceptor precursor that emerges in a neural retinal tissue and the like. To be specific, it can be set such that not less than 10%, preferably not less than 13%, more preferably not less than 16%, on average of the total number of cells are photoreceptor precursors in a neural retinal tissue 30 - 50 days, preferably 30 - 40 days, after emergence of a photoreceptor precursor. Specifically, when a BMP signal transduction pathway agonist, particularly BMP4, is used as a dorsalization signal transmitter, BMP4 is used at a concentration of preferably 0.01 nM - 0.90 nM, further preferably 0.05 nM - 0.45 nM.

The concentration of the dorsalization signal transmitter contained in the medium may also be a concentration affording a Wnt signal transduction pathway activating action that does not prohibits emergence of a retinal pigment epithelial cell. The concentration of the dorsalization signal transmitter that prohibits emergence of a retinal pigment epithelial cell is specifically, for example, a concentration affording a Wnt signal pathway activating action equivalent to that of 0.01 nM - 0.90 nM, preferably 0.05 nM - 0.45 nM, BMP4.

When the medium is substantially free of exogeneous retinoids such as 9-cis retinoic acid and the like, a dorsalization signal transmitter contained in the medium is preferably 0.05 nM - 0.15 nM, further preferably 0.1 nM - 0.15 nM, BMP4. In addition, Wnt signal transduction pathway agonist may be allowed to act at a concentration that affords a BMP signal transduction pathway activating action equivalent to BMP4 at preferably 0.05 nM - 0.15 nM, further preferably 0.1 nM - 0.15 nM.

When the medium contains retinoids such as 9-cis retinoic acid and the like, a dorsalization signal transmitter to be contained in the medium is, for example, BMP4 at preferably 0.15 nM - 0.90 nM, further preferably 0.15 nM - 0.45 nM. In addition, a Wnt signal transduction pathway agonist may be allowed to act at a concentration that affords a BMP signal transduction pathway activating action equivalent to BMP4 at preferably 0.15 nM - 0.90 nM, further preferably 0.15 nM - 0.45 nM.

As the dorsalization signal transmitter, an SHH signal transduction pathway inhibitor that inhibits ventralization signal transduction, specifically Cyclopamine-KAAD, can be mentioned. The concentration of Cyclopamine-KAAD contained in the medium is specifically 0.01 µM - 5 µM, further preferably 0.2 µM - 1 µM. When a retinal tissue in an initial developmental stage is produced, a medium containing a Wnt signal transduction pathway agonist is used as in the starting material production methods 5 - 7, or a medium containing a Wnt signal transduction pathway agonist is not used. When a ventralization signal transduction inhibitor is used as a dorsalization signal transmitter, preferably, a retinal tissue in an initial developmental stage prepared using the aforementioned medium containing a Wnt signal transduction pathway agonist as in starting material production methods 5 - 7 is more preferably used.

In addition, as the dorsalization signal transmitter, the above-mentioned BMP signal transduction pathway agonist, the above-mentioned Wnt signal transduction pathway agonist and/or the above-mentioned SHH signal transduction pathway inhibitor that inhibits ventralization signal transduction may be used in combination. When an SHH signal transduction pathway inhibitor that inhibits the above-mentioned ventralization signal transduction is used as the dorsalization signal transmitter, it is preferably used in combination with the above-mentioned BMP signal transduction pathway agonist and/or the above-mentioned Wnt signal transduction pathway agonist to prepare a retinal tissue containing a cone photoreceptor precursor at a higher ratio.

The period of culturing in a medium containing a dorsalization signal transmitter may be any as long as the effect of the dorsalization signal transmitter continues until the period when a rod photoreceptor precursor emerges when cultured in the absence of the dorsalization signal transmitter, and can be appropriately determined to be typically not less than 4 days, preferably not less than 20 days, more preferably not less than 70 days. Specifically, culturing for, for example, 50 days - 170 days is possible. Further specifically, culturing for 70 days - 100 days is possible; however, a longer period of addition is preferable to suppress emergence of a rod photoreceptor precursor.

The cone photoreceptor precursor or cone photoreceptor can be matured into L cone photoreceptor, M cone photoreceptor or S cone photoreceptor. Preferably, a neural retinal tissue rich in L cone photoreceptor and M cone photoreceptor can be obtained by further culturing cone photoreceptor precursor or cone photoreceptor in the presence of a dorsalization signal transmitter, and a neural retinal tissue rich in S cone photoreceptor can be obtained by culturing in the presence or absence of a dorsalization signal transmitter. At this time, to mature into L cone photoreceptor and M cone photoreceptor, it is more preferable to differentiate by simultaneously combining dorsalization signal transmitter and thyroid gland hormone signal transmitter, and to mature into S cone photoreceptor, it is preferable to differentiate in a medium substantially free of a thyroid gland hormone signal transmitter. It is further preferable to differentiate in a serum-free medium.

To induce selective differentiation into L cone photoreceptor or M cone photoreceptor, it is preferable to use a BMP4 signal transmitter as a dorsalization signal transmitter, and it is added to the culture medium at a final concentration of not less than 0.01 nM and not more than 100 nM, preferably not less than 0.05 nM and not more than 10 nM, more preferably not less than 0.1 nM and not more than 1.5 nM. When T3 is used as a thyroid gland hormone signal transmitter, it is allowed to act at a concentration of not less than 0.01 nM and not more than 100 nM, preferably not less than 0.5 nM and not more than 10 nM, more preferably not less than 2 nM and not more than 10 nM. When T4 is used as a thyroid gland hormone signal transmitter, it is allowed to act at a concentration of T4 showing an action equivalent to the above-mentioned T3. The period of culture by simultaneously combining a dorsalization signal transmitter and a thyroid gland hormone signal transmitter is not particularly limited, and it is typically not less than 50 days, preferably not less than 70 days, more preferably not less than 100 days, further more preferably not less than 150 days. As the time of starting culturing by simultaneously combining a dorsalization signal transmitter and a thyroid gland hormone signal transmitter, culturing is started after 100 days, preferably after 150 days, from the first emergence of photoreceptor precursor, and culturing is performed by simultaneously combining a dorsalization signal transmitter and a thyroid gland hormone signal transmitter until usually after 250 days, preferably after 300 days, more preferably after 400 days, after the first emergence of a photoreceptor precursor. To mature a cone photoreceptor precursor or cone photoreceptor into an L cone photoreceptor or M cone photoreceptor, it is preferably cocultured with retinal pigment epithelium or an conditioned medium (condition medium) after culturing the retinal pigment epithelium is used.

Conversely, not less than 90%, preferably not less than 99% of a photoreceptor precursor or photoreceptor (cone photoreceptor precursor or cone photoreceptor, rod photoreceptor precursor or rod photoreceptor) in the retinal tissue can also be maintained in a state before differentiation into L cone photoreceptor, M cone photoreceptor and/or S cone photoreceptor and before final maturation and a state in which a molecule necessary for light response such as visual pigment and the like is not expressed or produced. It is known that a photoreceptor precursor is connected to a bipolar cell along with maturation. Thus, in this way, the connection between the photoreceptor precursor and the bipolar cell in the retinal tissue to be transplanted is suppressed, and when the retinal tissue is transplanted, the connection efficiency of the photoreceptor precursor in the prepared retinal tissue and the recipient bipolar cell can be increased. To be specific, the state before the final maturation can be maintained by culturing in a medium free of glutamic acid, more preferably a medium free of glutamic acid or aspartic acid, further preferably a medium free of a neurotransmitter such as glutamic acid, aspartic acid and the like. Further more preferably, a medium containing a serum, specifically, a medium containing not less than 5%, preferably not less than 10% of a serum. As said medium, the above-mentioned medium for maintaining a continuous epithelial tissue can be specifically mentioned. While the serum used here is not particularly limited, specifically, fetal bovine serum (FBS) can be mentioned.

That is, a method for suppressing final maturation of photoreceptor precursor, including a step of culturing a retinal tissue containing a photoreceptor precursor or photoreceptor in a medium not containing a neurotransmitter and containing a serum is also within the scope of the present invention.

The "final maturation" here means a state where a part, preferably not less than 15%, more preferably not less than 30%, further preferably not less than 60%, of a photoreceptor precursor or photoreceptor forms a synapse, or expresses a functional molecule such as visual pigment and the like. That is, the neural retinal tissue that has matured to a differentiation stage where Muller cells are observed but has not yet reached final maturation is preferably a neural retinal tissue in a stage where synapse formation has not occurred or a functional molecule such as a visual pigment and the like has not been expressed. Here, whether or not photoreceptor precursor is finally matured can be identified using a photoreceptor specific visual pigment such as S-opsin, L-opsin, M-opsin and the like, or an antibody against a functional factor necessary for light stimulus response. That is, a cell in which a functional molecule such as S-opsin, L-opsin, M-opsin, Rhodopsin, Cone-arrestin, arrestin, CNGA3, CNGA1, G alpha t2, G alpha t1, PDE6c, PDE6a, PDE6b and the like is expressed can be identified as a finally matured photoreceptor. Whether the photoreceptor precursor and a bipolar cell were contacted in the retinal tissue and formed a neural circuit can be identified by multiple staining using a combination of antibodies against for RIBEYE, CtBP2, which are expressed in a region where a photoreceptor and a bipolar cell formed the neural circuit, mGluR6 expressed in a bipolar cell in the region, Arrestin, Recoverin, cone specific Arrestin (Gene symbol; ARR3) which are expressed in the synaptic ending of the photoreceptor in the region, PNA said to be cone photoreceptor specific or ELFN1 said to be rod photoreceptor specific. Alternatively, formation of a neural circuit may also be identified by observing the synaptic ending of photoreceptor and the synapse structure of the bipolar cell using an electron microscope.

On the other hand, by culturing a retinal tissue containing a photoreceptor precursor or photoreceptor in a serum-free medium, a completely matured retinal tissue containing L cone photoreceptor, M cone photoreceptor and/or S cone photoreceptor can be obtained. That is, a method of promoting complete maturation of a photoreceptor precursor or photoreceptor, which includes a step of culturing a retinal tissue containing a photoreceptor precursor or photoreceptor in a serum-free medium is also within the scope of the present invention.

Here, cells in which functional molecules such as S-opsin, L-opsin, M-opsin, Rhodopsin, Cone-arrestin, arrestin, CNGA3, CNGA1, G alpha t2, G alpha t1, PDE6c, PDE6a, or PDE6b and the like are expressed can be identified as a finally matured photoreceptor. That is, a completely matured retinal tissue is a retinal tissue that expresses one or plural, preferably not less than 3, of these functional molecules, similar to normal retinal tissue in vivo.

When final maturation of L cone photoreceptor or M cone photoreceptor is promoted, the aforementioned serum-free medium is preferably a medium containing a dorsalization signal transmitter. As the dorsalization signal transmitter, a BMP signal transduction pathway promoter, specifically, BMP such as BMP2, BMP4, BMP7 and GDF7 and the like can be mentioned. On the other hand, when final maturation of S cone photoreceptor is promoted, similarly, a medium containing a dorsalization signal transmitter can be preferably used. When final maturation of a S cone photoreceptor is specifically promoted, a medium not containing a dorsalization signal transmitter can be preferably used.

When final maturation of L cone photoreceptor, M cone photoreceptor is promoted, the aforementioned serum-free medium is preferably a medium containing a dorsalization signal transmitter, as well as a thyroid gland hormone signal transmitter. As the thyroid gland hormone signal transmitter, T3 or T4 can be specifically mentioned. On the other hand, since a thyroid gland hormone signal transmitter inhibits final maturation of a S cone photoreceptor, when final maturation of a S cone photoreceptor is promoted, a medium substantially free of a thyroid gland hormone signal transmitter is preferably used.

Here, the period of culturing a retinal tissue containing a photoreceptor precursor or photoreceptor in a serum-free medium can be appropriately determined by monitoring the degree of maturation. To be specific, a retinal tissue in a differentiation stage with maturation permitting observation of Muller cell can be cultured in a serum-free medium for not less than about 20 days, preferably not less than 30 days, more preferably not less than 60 days, further preferably not less than 100 days. When final maturation of L cone photoreceptor, M cone photoreceptor is promoted, they can be cultured in a serum-free medium containing a dorsalization signal transmitter at a concentration corresponding to BMP4 at 0.5 nM - 3 nM, preferably 1 nM - 2 nM, and, in some cases, a thyroid gland hormone signal transmitter corresponding to T3 at 0 - 10 nM, for not less than about 20 days, preferably not less than 30 days, preferably not less than 30 days, more preferably not less than 60 days, further preferably not less than 100 days.

As the dorsalization signal transmitter, the above-mentioned Wnt signal transduction pathway agonist can also be used. In this case, specifically, the following steps (1) and (2) are repeated and the intensity of the Wnt signal may be adjusted:
(1) culturing in the medium added the above-mentioned Wnt signal transduction pathway agonist for 1 - 5 days, preferably 1 - 3 days;
(2) culturing in the medium substantially free of above-mentioned Wnt signal transduction pathway agonist for 1 - 15 days, 1 - 10 days, preferably 1 - 7 days or 5 - 10 days.

That is, BMP4 of the same level as when BMP was added at 0.1 - 0.45 nM can be induced by repeating the aforementioned (1) and (2) .

### 3. Method for increasing proportion of rod photoreceptor in photoreceptor contained in retinal tissue (production method of retinal tissue rich in rod photoreceptor)

One embodiment of the present invention is a method for increasing a proportion of a rod photoreceptor precursor and a rod photoreceptor in a photoreceptor precursor and a photoreceptor comprised in a retinal tissue, comprising a step of culturing a retinal tissue, in an initial developmental stage to a stage where an emergence rate of a cone photoreceptor precursor reaches maximum, in the presence of a ventralization signal transmitter at a concentration sufficient to suppress expression of a dorsal marker.

The medium used when culturing a retinal tissue, in an initial developmental stage to a stage where an emergence rate of a cone photoreceptor precursor reaches maximum, in the presence of a ventralization signal transmitter is not particularly limited as long as it does not contain a substance that inhibits the effect of the ventralization signal transmitter in an amount capable of inhibiting the aforementioned effect, and can be prepared using a medium typically used for cell culture as in the aforementioned 2. Examples of the basal medium include a medium usable for culturing animal cell such as Neurobasal medium, BME medium, BGJb medium, CMRL 1066 medium, Glasgow MEM (GMEM) medium, Improved MEM Zinc Option medium, IMDM medium, Medium 199 medium, MEM medium, Eagle MEM medium, αMEM medium, DMEM medium, F-12 medium, DMEM/F12 medium, IMDM/F12 medium, Ham's medium, RPMI1640 medium, Fischer's medium, Leibovitz's L-15 medium or a mixed medium of these and the like. A specifically usable medium includes, for example, a medium added with Wnt2b, Neurobasal medium, a medium containing Neurobasal medium and the like, and may be a Neurobasal medium added with Wnt2b.

The aforementioned medium may or may not contain retinoids (e.g., retinoic acid or a derivative thereof) and, in one embodiment, it may or may not contain all trans retinoic acid or 9-cis retinoic acid. In addition, a medium substantially free of retinoids can also be mentioned as a preferable embodiment.

The concentration of a ventralization signal transmitter contained in the medium may be a concentration affording an SHH signal transduction pathway activating action or a concentration affording a BMP signal transduction pathway inhibitory activity, that can suppress emergence of a photoreceptor precursor in a period when a cone photoreceptor precursor in the retinal tissue emerges along with the development differentiation. Such concentration can be easily set by those of ordinary skill in the art. Specifically, a concentration of a ventralization signal transmitter that promotes expression of ALDH1A3 may be set, and the concentration is determined using a method conventionally performed, such as immunostaining and the like such that the region expressing ventral marker COUP-TF I, and/or ALDH1A3 relative to the cells contained in the retinal tissue is not less than 60%, preferably not less than 80%, more preferably not less than 90%, further more preferably not less than 99%, of the whole in a neural retinal tissue in a stage where emergence rate of cone photoreceptor precursor reaches maximum. Specifically, it is, for example, a concentration affording an SHH signal transduction pathway activating action equivalent to that of SAG at 1 nM - 10 µM, preferably 10 nM - 500 nM, or a concentration affording a BMP signal transduction pathway inhibitory activity equivalent to that of LDN193189 at 0.1 nM - 20 µM, preferably 30 nM - 1 µM. As the ventralization signal transmitter, SHH signal transduction pathway agonist, preferably SAG at 1 nM - 10 µM, more preferably 10 nM - 500 nM, can be mentioned.

As the ventralization signal transmitter, a BMP signal transduction pathway inhibitor that inhibits dorsalization signal transduction, specifically LDN193189 and the like can be mentioned. The concentration of LDN193189 contained in the medium is specifically 0.1 nM - 20 µM, preferably 30 nM - 1 µM.

When a BMP signal transduction pathway inhibitor such as LDN193189 and the like is used as a ventralization signal transmitter, differentiation efficiency of rod photoreceptor precursor and rod photoreceptor, as well as S cone photoreceptor precursor and/or S cone photoreceptor is improved. That is, a method for increasing the proportion of S cone photoreceptor precursor and S cone photoreceptor in photoreceptor precursor and photoreceptor contained in a retinal tissue, including a step of culturing a retinal tissue from an initial developmental stage to a stage where emergence rate of cone photoreceptor precursor reaches maximum in the presence of a BMP signal transduction pathway inhibitor, specifically, for example, LDN-193189, at a concentration that suppresses expression of a dorsal marker is also one embodiment the present invention.

Here, to decrease the proportion of the cone photoreceptor precursor, an SHH signal transduction pathway agonist is more preferably used than a BMP4 signal transduction pathway inhibitor.

In addition, one or plural ventralization signal transmitters mentioned above may be appropriately combined and used as a ventralization signal transmitter. For example, SHH signal transduction pathway agonist and/or BMP signal transduction pathway inhibitor that inhibits dorsalization signal transduction may be combined and used.

The period of culturing in the presence of a ventralization signal transmitter may be any as long as the effect of the ventralization signal transmitter continues until the period when a rod photoreceptor precursor emerges when cultured in the absence of the ventralization signal transmitter, and can be appropriately determined to be typically not less than 4 days, preferably not less than 20 days, more preferably not less than 70 days. Specifically, culturing for, for example, 50 days - 170 days is possible. Further specifically, culturing for 70 days - 100 days is possible; however, a longer period of addition is preferable to promote emergence of a rod photoreceptor precursor.

### 4. Production of retinal tissue in initial developmental stage

A method for producing a retinal tissue in an initial developmental stage which is a starting material used in the methods of the above-mentioned 2 and the above-mentioned 3 from a pluripotent stem cell such as human iPS cell and the like is explained.

Pluripotent stem cells such as human iPS cell and the like can be obtained or produced by a method well known to those of ordinary skill in the art as mentioned above and subjected to maintenance culture and expansion culture. While the maintenance culture and expansion culture of pluripotent stem cells can be performed by suspension culture or adhesion culture, it is preferably performed by adhesion culture. While the maintenance culture and expansion culture of pluripotent stem cells may be performed in the presence of feeder cells or in the absence of feeder cells (feeder free), it is preferably performed in the absence of feeder cells.

A retinal tissue in an initial developmental stage can be produced using pluripotent stem cells subjected to maintenance culture and by a method well known to those of ordinary skill in the art. As this method, the methods described in WO 2013/077425 (& US2014/341864), WO 2015/025967 (& US2016/251616), WO 2016/063985, WO 2016/063986 and WO 2017/183732 and the like can be mentioned. Also, as this method, the methods described in non-patent documents: Proc Natl Acad Sci U S A. 111(23): 8518-8523(2014), Nat Commun. 5:4047(2014), Stem Cells.(2017):35(5), 1176-1188 and the like, and the like can be mentioned.

### 4-1. Starting material production method 1

As one preferable embodiment of production of a retinal tissue in an initial developmental stage, the method described in WO 2015/025967 including the following steps can be mentioned:
(1) the first step of forming a cell aggregate by culturing pluripotent stem cells in suspension in a serum-free medium, and
(2) the second step of obtaining an aggregate containing a retinal progenitor cell or a neural retinal progenitor cell by culturing in suspension the aggregate formed in the first step in a serum-free medium or serum-containing medium not containing an SHH signal transduction pathway agonist and containing a BMP signal transduction pathway agonist.

An aggregate containing a retinal progenitor cell or a neural retinal progenitor cell which is obtained by the method can be used as a retinal tissue in an initial developmental stage which is a starting material used in the methods of the above-mentioned 2 and the above-mentioned 3.

### [First step]

The first step can be performed according to the method described in WO2015/025967 (& US2014/341864). That is, in the first step, a cell aggregate is formed by culturing pluripotent stem cells in suspension in a serum-free medium.

The serum-free medium to be used in the first step is not particularly limited as long as it is as described above. For example, a serum-free medium free of both a BMP signal transduction pathway agonist and a Wnt signal transduction pathway inhibitor can be used. To avoid complicated preparation, for example, a serum-free medium supplemented with an appropriate amount of a commercially available serum replacement such as KSR and so on (e.g., medium of 1:1 mixture of IMDM and F-12, which is supplemented with 10% KSR, 450 µM 1-monothioglycerol and 1x Chemically Defined Lipid Concentrate) is preferably used. As a serum replacement, bovine serum albumin (BSA) can also be added at a concentration of 0.1 mg/mL - 20 mg/mL, preferably about 4 mg/mL - 6 mg/mL, to a serum-free medium. The amount of KSR to be added to a serum-free medium in the case of human ES cell or human iPS cell is generally about 1% to about 20%, preferably about 2% to about 20%.

The culture conditions such as culture temperature, CO₂ concentration and so on in the first step can be appropriately determined. The culture temperature is, for example, about 30°C to about 40°C, preferably about 37°C. The CO₂ concentration is, for example, about 1% to about 10%, preferably about 5%.

The concentration of the pluripotent stem cells usable in the first step can be appropriately set so that an aggregate of the pluripotent stem cells can be more uniformly and efficiently formed. For example, when human ES cells are cultured in suspension using a 96-well plate, a liquid prepared to achieve about 1 x 10³ to about 1 x 10⁵ cells, preferably about 3 x 10³ to about 5 x 10⁴ cells, more preferably about 5 x 10³ to about 3 x 10⁴ cells, further more preferably about 0.9 x 10⁴ to 1.2 x 10⁴ cells, per well is added to the wells, and the plate is left to stand to form aggregates.

The period for suspension culture necessary for forming an aggregate can be determined as appropriate according to the pluripotent stem cell to be used. To form uniformed cell aggregates, it is desirably as short as possible (e.g., SFEBq method). The steps for the dispersed cells to form cell aggregates can be divided into a step for assembling cells, and a step forming cell aggregates from the assembled cells. The period from the time point of seeding the dispersed cells (i.e., at the time of the start of suspension culture) to assemble cells in case of human ES cell or human iPS cell is, for example, preferably within about 24 hr, more preferably to form aggregate within about 12 hr. The period from the time point of seeding the dispersed cells (i.e., at the time of the start of suspension culture) to form an aggregate in case of pluripotent stem cell (e.g., human iPS cell) is, for example, preferably within about 72 hr, more preferably within about 48 hr. The period for cell aggregate formation can be appropriately adjusted by controlling the tools for aggregating the cells, centrifugation conditions and so on.

Formation of cell aggregates can be determined based on the size and cell number of the aggregates, macroscopic morphology, microscopic morphology by tissue staining analysis and uniformity thereof, expression of differentiation- and undifferentiation-markers and uniformity thereof, control of expression of differentiation marker and synchrony thereof, reproducibility of differentiation efficiency between the aggregates, and so on.

### [Second step]

A second step of obtaining an aggregate containing a retinal progenitor cell or a neural retinal progenitor cell as a retinal tissue in an initial developmental stage by culturing in suspension the aggregate formed in the aforementioned first step in a serum-free medium or serum-containing medium that does not contain an SHH signal transduction pathway agonist and contains a BMP signal transduction pathway agonist is explained.

The medium to be used in the second step is, for example, a serum-free medium or a serum-containing medium not supplemented with an SHH signal transduction pathway agonist but supplemented with a BMP signal transduction pathway agonist. It is not necessary to add a basement membrane preparation.

A serum-free medium or serum-containing medium to be used for such medium is not particularly limited as long as it is as mentioned above. To avoid complicated preparation, for example, a serum-free medium supplemented with an appropriate amount of a commercially available serum replacement such as KSR and so on (e.g., medium of 1:1 mixture of IMDM and F-12 supplemented with 10% KSR, 450 µM 1-monothioglycerol and 1x Chemically Defined Lipid Concentrate) is preferably used. As a serum replacement, BSA can also be added at a concentration of 0.1 mg/mL - 20 mg/mL, preferably about 4 mg/mL - 6 mg/mL, to a serum-free medium. The amount of KSR to be added to a serum-free medium in the case of human ES cell is generally about 1% to about 20%, preferably about 2% to about 20%.

As the serum-free medium to be used in the second step, the serum-free medium used in the first step may be continuously used as it is as long as it is free of a SHH signal transduction pathway agonist, or may be that replaced with a fresh medium. When the serum-free medium free of a BMP signal transduction pathway substance used in the first step is directly used for the second step, a BMP signal transduction pathway agonist may be added to the medium.

The medium "free of a SHH signal transduction pathway agonist" also includes a medium substantially free of a SHH signal transduction pathway agonist, for example, a medium free of a SHH signal transduction pathway agonist at a concentration imparting an adverse influence on selective differentiation into a retinal progenitor cell or a retinal tissue.

The medium "not supplemented with a SHH signal transduction pathway agonist" also includes a medium substantially not supplemented with a SHH signal transduction pathway agonist, for example, a medium not supplemented with a SHH signal transduction pathway agonist at a concentration imparting an adverse effect on selective differentiation into a retinal progenitor cell or a retinal tissue.

Examples of BMP signal transduction pathway agonist used in the second step include BMP proteins such as BMP2, BMP4, BMP7 etc., GDF proteins such as GDF7 etc., anti-BMP receptor antibody, BMP partial peptides and so on. BMP2, BMP4 and BMP7 are available from, for example, R&D Systems, and GDF7 is available from, for example, Wako Pure Chemical Industries, Ltd. The BMP signal transduction pathway agonist is preferably BMP4.

The concentration of the BMP signal transduction pathway agonist used in the second step only need to be a concentration at which differentiation of the cells contained in the aggregates obtained in the aforementioned the first step into retinal cells can be induced. For example, BMP4 is added to the medium such that the concentration is about 0.01 nM to about 1 µM, preferably about 0.1 nM to about 100 nM, more preferably about 1 nM - about 10 nM, further preferably about 1.5 nM (55 ng/mL). When a BMP signal transduction pathway agonist other than BMP4 is used, it is desirably used at a concentration at which a BMP signal transduction pathway activation action equivalent to that of BMP4 at the above-mentioned concentration is exerted.

A BMP signal transduction pathway agonist may be added after about 24 hr or later from the start of the suspension culture in the first step, and may also be added to the medium within several days (e.g., within 15 days) from the start of the suspension culture in the first step. Preferably, a BMP signal transduction pathway agonist is added to the medium at day 1 to day 15, more preferably day 1 to day 9, further preferably day 2 to day 9, further preferably day 3 to day 8, still more preferably day 3 to day 6, further more preferably day 6, from the start of the suspension culture.

After the addition of a BMP signal transduction pathway agonist to the medium and the start of the differentiation induction of cells contained in the aggregate obtained in the first step to a retinal cell, further addition of the BMP signal transduction pathway agonist to the medium is not necessary, and the medium may be exchanged with a serum-free medium or serum-containing medium each free of a BMP signal transduction pathway agonist.

Alternatively, the concentration of the BMP signal transduction pathway agonist in the medium may be varied during the period of the second step. For example, the BMP signal transduction pathway agonist is provided to fall within the above-mentioned range of a concentration at the time of the start of the second step, and the concentration may be gradually or stepwisely decreased at a ratio of 40 - 60% per 2 - 4 days.

In a specific embodiment, the medium is partly or entirely exchanged with a medium containing BMP4 on the 1st - 9th day, preferably the 2nd - 9th day, further preferably the 3rd - 8th day, further more preferably the 3rd - 6th day, after the start of suspension culture (namely, after the start of the aforementioned first step), the final concentration of BMP4 is adjusted to about 1 - 10 nM, and the cells can be cultured in the presence of BMP4 for, for example, 1 - 16 days, preferably 2 - 9 days, further preferably 6 - 9 days. It is also possible to culture cells for a longer term, specifically for not less than 20 days, not less than 30 days. That is, in the second step, culture performed in the presence of a BMP signal transduction pathway agonist is appropriately continued for a period until the aggregate obtained in the first step is induced to differentiate into a retinal tissue in an initial developmental stage. Specifically, the retinal tissue in an initial developmental stage can be obtained in 6-12 days after addition of the BMP signal transduction pathway agonist.

Here, the medium can be partly or entirely exchanged about 1 or 2 times with a medium containing BMP4 to maintain the concentration of BMP4 at the same concentration. Alternatively, as mentioned above, the concentration of BMP4 can also be reduced step by step.

In one embodiment, after the start of culturing in a medium containing a BMP signal transduction pathway agonist, the concentration of the BMP signal transduction pathway agonist in the medium can be gradually or stepwisely decreased at a ratio of 40 - 60% per 2 - 4 days by exchanging the medium with a serum-free medium or a serum-containing medium, each free of a BMP signal transduction pathway agonist.

Differentiation induction of a retinal tissue in an initial developmental stage can be confirmed by, for example, detecting expression of a retinal progenitor cell marker and a neural retinal progenitor cell marker in the cells in the tissue. The aggregate formed in the first step by using pluripotent stem cells in which a fluorescence reporter protein gene such as GFP was knocked-in into the Rx gene locus is cultured in suspension in the presence of a BMP signal transduction pathway agonist at a concentration necessary for differentiation induction into retinal cell, and fluorescence emitted from the expressed fluorescence reporter protein is detected, whereby the time point when differentiation induction into retinal cell was started can be confirmed.

As one embodiment of the second step, a step of culturing the aggregate formed in the first step in suspension in a serum-free medium or serum-containing medium containing a BMP signal transduction pathway agonist at a concentration necessary for differentiation induction into retinal cell and not containing an SHH signal transduction pathway agonist, until a cell expressing retinal progenitor cell marker or neural retinal progenitor marker (e.g., Rx, Pax6, Chx10) begins emerging, thereby obtaining an aggregate containing retinal progenitor cells or neural retinal progenitor cells as retinal tissues in an initial developmental stage can be mentioned.

In the second step, when a medium exchange operation is performed, for example, an operation to add a fresh medium without discarding the existing medium (medium addition operation), an operation to discard about a half amount of the existing medium (about 40 - 80% of the volume of the existing medium) and add about a half amount of a fresh medium (40 - 80% of the volume of the existing medium) (half-medium exchange operation), and an operation to discard about the whole amount of the existing medium (not less than 90% of the amount of the existing medium) and add about the whole amount of a fresh medium (not less than 90% of the amount of the existing medium) (full-medium exchange operation) can be mentioned.

When a particular component (e.g., BMP4) is added at a certain time point, for example, an operation to calculate the final concentration, to discard about a half amount of the existing medium, and to add about a half amount of a fresh medium containing a particular component at a concentration higher than the final concentration (specifically 1.5 times - 3.0 times the final concentration, for example, about 2 times the final concentration, (half-medium exchange operation, half-medium exchange) may be performed.

When the concentration of a particular component contained in the existing medium is maintained at a certain time point, for example, an operation to discard about a half amount of the existing medium and to add about a half amount of a fresh medium containing the particular component at a concentration same as that in the existing medium may be performed.

When the concentration of a component contained in the existing medium is to be decreased by dilution at a certain time point, for example, the medium exchange operation may be performed plural times per day, preferably plural times (e.g., 2 - 3 times) within 1 hr. Also, when the concentration of a component contained in the existing medium is to be decreased by dilution at a certain time point, the cells or aggregates may be transferred to another culture container.

While the tool used for the medium exchange operation is not particularly limited, for example, pipetter, micropipette, multichannel micropipette, continuous dispenser, and the like can be mentioned. For example, when a 96 well plate is used as a culture vessel, a multichannel micropipette may be used.

In a preferable embodiment, the concentration of a SHH signal transduction pathway agonist in the medium to be used in the second step is, when calculated in terms of SHH signal transduction promoting activity of SAG, not more than 700 nM, preferably not more than 300 nM, more preferably not more than 10 nM, further preferably not more than 0.1 nM. Further preferably, it is free of a SHH signal transduction pathway agonist. The medium "free of a SHH signal transduction pathway agonist" also includes a medium substantially free of a SHH signal transduction pathway agonist, for example, a medium free of a SHH signal transduction pathway agonist at a concentration imparting an adverse influence on selective differentiation into a retinal progenitor cell or a retinal tissue. The medium "not supplemented with a SHH signal transduction pathway agonist" also includes a medium substantially not supplemented with a SHH signal transduction pathway agonist, for example, a medium not supplemented with a SHH signal transduction pathway agonist at a concentration imparting an adverse effect on selective differentiation into a retinal progenitor cell or a retinal tissue.

The culture conditions such as culture temperature, CO₂ concentration and so on in the second step can be appropriately determined. The culture temperature is, for example, about 30°C to about 40°C, preferably about 37°C. The CO₂ concentration is, for example, about 1% to about 10%, preferably about 5%.

By such culture differentiation of the cells forming the aggregates obtained in the first step to a retinal tissue in an initial developmental stage can be induced. That an aggregate containing retinal progenitor cells or neural retinal progenitor cell was obtained as a retinal tissue in an initial developmental stage can be confirmed by, for example, detecting the presence of cells expressing Rx, PAX6, which is a retinal progenitor cell marker, or RX, PAX6 or CHX10, which is a neural retinal progenitor cell marker, in the aggregate.

One embodiment of the second step can be a step of culturing the aggregate formed in the first step in suspension in a serum-free medium or serum-containing medium containing a BMP signal transduction pathway agonist at a concentration necessary for differentiation induction into a retinal cell, until a cell expressing Rx gene begins emerging, whereby obtaining an aggregate containing retinal progenitor cells or neural retinal progenitor cells. In one embodiment, the culturing of the second step is performed until not less than 20% (preferably, not less than 30%, not less than 40%, not less than 50%, not less than 60%) of the cells contained in the aggregate express Rx.

The aggregate obtained by the above-mentioned method can be used as a retinal tissue in an initial developmental stage that is the starting material in the production method of the present invention after culturing in a suspension in a serum-free medium or serum-containing medium not containing any of SHH signal transduction pathway agonist, BMP signal transduction pathway agonist and Wnt signal transduction pathway agonist. The period of the suspension culture is not particularly limited as long as it is a period until emergence of a ganglion cell. It is, for example, 1 day - 50 days, preferably 1 day - 15 days, more preferably 1 day - 7 days.

The medium used in the aforementioned suspension culture is, for example, a serum-free medium or serum-containing medium not supplemented with any of SHH signal transduction pathway agonist, BMP signal transduction pathway agonist and Wnt signal transduction pathway agonist.

The medium "not containing any of SHH signal transduction pathway agonist, BMP signal transduction pathway agonist and Wnt signal transduction pathway agonist" also includes a medium substantially not containing any of SHH signal transduction pathway agonist, BMP signal transduction pathway agonist and Wnt signal transduction pathway agonist, for example, a medium not containing SHH signal transduction pathway agonist, BMP signal transduction pathway agonist or Wnt signal transduction pathway agonist at a concentration that adversely affects selective differentiation into retinal tissue.

The medium "not supplemented with any of SHH signal transduction pathway agonist, BMP signal transduction pathway agonist and Wnt signal transduction pathway agonist" also includes a medium substantially not supplemented with any of SHH signal transduction pathway agonist, BMP signal transduction pathway agonist and Wnt signal transduction pathway agonist, for example, a medium not supplemented with SHH signal transduction pathway agonist, BMP signal transduction pathway agonist or Wnt signal transduction pathway agonist at a concentration that adversely affects selective differentiation into retinal tissue.

A serum-free medium or serum-containing medium to be used for such medium is not particularly limited as long as it is as mentioned above. To avoid complicated preparation, for example, a serum-free medium supplemented with an appropriate amount of a commercially available serum replacement such as KSR and so on (e.g., medium of 1:1 mixture of IMDM and F-12 supplemented with 10% KSR, 450 µM 1-monothioglycerol and 1x Chemically Defined Lipid Concentrate) is preferably used. Bovine serum albumin (BSA) can also be added at a concentration of 0.1 mg/mL - 20 mg/mL, preferably about 4 mg/mL - 6 mg/mL, to a serum-free medium. The amount of KSR to be added to a serum-free medium in the case of, for example, human ES cell is generally about 1% to about 20%, preferably about 2% to about 20%. To avoid complicated preparation of serum-containing medium, for example, a serum-containing medium supplemented with an appropriate amount of a commercially available serum (e.g., medium of 1:1 mixture of DMDM and F-12, which is supplemented with serum, and N2 supplement) is more preferably used. The amount of serum to be added to a serum-containing medium in the case of human ES cell is generally about 1% to about 20%, preferably about 2% to about 20%. All the above-mentioned media may be used after addition of taurine and the like.

The culture conditions such as culture temperature, CO₂ concentration, O₂ concentration and the like can be appropriately determined. The culture temperature is, for example, about 30°C to about 40°C, preferably about 37°C. The CO₂ concentration is, for example, about 1% to about 10%, preferably about 5%. The O₂ concentration is not less than about 5%, for example, about 20% to about 70%, preferably about 20% to about 60%, more preferably about 20% to about 40%, particularly preferably about 20%.

As described above, that the retinal tissue obtained by the starting material production method 1 is in an initial developmental stage, that is, in a differentiation stage in the initial stage of development in which a retinal progenitor cell or a neural retinal progenitor cell is contained and a ganglion cell has not emerged can be identified by measuring the expression state of at least one of retinal progenitor cell markers such as RX, PAX6 and the like, neural retinal progenitor markers such as CHX10, RX, PAX6 and the like, and ganglion cell markers such as BRN3 and the like. That is, it can be confirmed that, in this differentiation stage, not less than 30%, preferably not less than 50%, more preferably not less than 80%, further preferably not less than 90%, further more preferably not less than 99%, of the whole cells contained in the retinal tissue express retinal progenitor cell marker and/or neural retinal progenitor cell marker, and not more than 40%, preferably not more than 20%, not more than 10%, not more than 5%, not more than 1%, further preferably not more than 0.1%, further more preferably not more than 0.01%, of the whole cells contained in the retinal tissue express ganglion cell marker.

At this time, expression of ventral marker and/or most dorsal marker (e.g., ALDH1A3 and/or ALDH1A1) does not pose any problem, and both may be in a differentiation stage that can be suppressed or promoted by a dorsalization signal transduction pathway agonist.

### 4-2. Starting material production method 2

As one preferable embodiment of production of retinal tissue in an initial developmental stage, the method described in WO 2016/063985 and WO 2017/183732 and containing the following steps can be mentioned:
(1) a first step of culturing pluripotent stem cells in the absence of feeder cells and in a medium containing 1) a TGFβ family signal transduction pathway inhibitor and/or an SHH signal transduction pathway agonist, and 2) a factor for maintaining undifferentiated state,
(2) a second step of culturing the cells obtained in the first step in suspension to form a cell aggregate, and
(3) a third step of culturing the aggregate obtained in the second step in suspension in the presence of a BMP signal transduction pathway agonist to obtain an aggregate containing a retinal progenitor cell or a neural retinal progenitor cell.

An aggregate obtained by this method and containing retinal progenitor cell or neural retinal progenitor cell can be used as a retinal tissue in an initial developmental stage and as a starting material used in the methods of the above-mentioned 2 and the above-mentioned 3.

### [First step]

The first step can be performed according to the method described in WO 2016/063985. That is, the absence of feeder cells (hereinafter to be also referred to as feeder-free) in the first step means a condition substantially free of feeder cells (e.g., the ratio of the number of feeder cells relative to the total number of cells is not more than 3%). Preferably, the first step is performed under a condition free of feeder cells. The medium to be used in the first step is not particularly limited as long as it is a medium enabling culturing of pluripotent stem cells to maintain undifferentiated state under feeder-free conditions (feeder-free medium). Preferably, to enable culturing to maintain undifferentiated state, it contains a factor for maintaining undifferentiated state.

The factor for maintaining undifferentiated state is not particularly limited as long as it is a substance having an action to suppress differentiation of pluripotent stem cells. Examples of the factor for maintaining undifferentiated state widely used by those of ordinary skill in the art include a FGF signal transduction pathway agonist, a TGFβ family signal transduction pathway agonist, insulin and the like. As the FGF signal transduction pathway agonist, fibroblast growth factors (e.g., bFGF, FGF4, FGF8) can be specifically mentioned. As the TGFβ family signal transduction pathway agonist, a TGFβ signal transduction pathway agonist, a Nodal/Activin signal transduction pathway agonist can be mentioned. As the TGFβ signal transduction pathway agonist, TGFβ1, TGFβ2 can be mentioned. As the Nodal/Activin signal transduction pathway agonist, Nodal, Activin A, Activin B can be mentioned. When human pluripotent stem cells (human ES cells, human iPS cells) are cultured, the medium in the first step preferably contains bFGF as a factor for maintaining undifferentiated state.

The factor for maintaining undifferentiated state to be used in the present invention is not particularly limited as long as it is a mammal-derived factor for maintaining undifferentiated state. Preferably, a factor for maintaining undifferentiated state of a mammal of the same species as the cells to be cultured is used. For example, for culturing human pluripotent stem cells, human factor for maintaining undifferentiated states (e.g., bFGF, FGF4, FGF8, EGF, Nodal, Activin A, Activin B, TGFβ 1, TGFβ 2 etc.) are used, and an isolated factor for maintaining undifferentiated state can be exogenously added. Alternatively, a factor for maintaining undifferentiated state may be added in advance to the medium to be used in the first step.

The concentration of the factor for maintaining undifferentiated state in the medium to be used in the first step is a concentration capable of maintaining the undifferentiated state of the pluripotent stem cells to be cultured, and can be appropriately determined by those of ordinary skill in the art. For example, specifically, when bFGF is used as a factor for maintaining undifferentiated state in the absence of feeder cells, the concentration thereof is generally about 4 ng - 500 ng/mL, preferably about 10 ng - 200 ng/mL, more preferably about 30 ng - 150 ng/mL.

As a feeder-free medium containing a factor for maintaining undifferentiated state and usable for culturing pluripotent stem cells, many synthetic media have been developed and are commercially available and, for example, Essential 8 medium (manufactured by Life Technologies) can be mentioned. Essential 8 medium is DMEM/F12 medium containing L-ascorbic acid-2-phosphate magnesium (64 mg/l), sodium selenium (14 µg/L), insulin (19.4 mg/l), NaHCO₃ (543 mg/l), transferrin (10.7 mg/l), bFGF (100 ng/mL), and a TGFβ family signal transduction pathway agonist (TGFβ 1 (2 ng/mL) or Nodal (100 ng/mL)) as additives (Nature Methods, 8, 424-429 (2011)). Examples of other commercially available feeder-free medium include S-medium (manufactured by DS Pharma Biomedical), StemPro (manufactured by Life Technologies), hESF9 (Proc. Natl. Acad. Sci. USA. 2008 Sep 9; 105(36):13409-14), mTeSR1 (manufactured by STEMCELL Technologies), mTeSR2 (manufactured by STEMCELL Technologies), TeSR-E8 (manufactured by STEMCELL Technologies), and StemFit (manufactured by Ajinomoto Co., Inc.). The present invention can be performed conveniently by using these in the above-mentioned first step.

In the first step, the pluripotent stem cells may be cultured under any conditions of suspension culture and adhesion culture, preferably adhesion culture.

While a culture vessel used for adhesion culture is not particularly limited as long as "adhesion culture" can be performed, a cell adhesive culture vessel is preferable. Cell-adhesive culture vessels include culture vessels whose surfaces have been artificially treated to improve cell adhesiveness, and specifically, the aforementioned culture vessel whose inside is coated with a coating agent can be mentioned. Examples of the coating agent include extracellular matrix such as laminin [including laminin α5β1γ1 (hereinafter laminin 511), laminin α1β1γ1 (hereinafter laminin 111) and the like and laminin fragment (laminin 511E8 etc.)], entactin, collagen, gelatin, vitronectin, Synthemax (Corning Incorporated), Matrigel and the like, or polymer such as polylysine, polyornithine and the like, and the like. It is also possible to use a culture container whose surface is processed by a positive electric charge treatment and the like. Preferred is laminin and more preferred is laminin 511E-8. Laminin 511E-8 can be a commercially available product (e.g., iMatrix-511, Nippi) .

The medium to be used in the first step contains a TGFβ family signal transduction pathway inhibitor and/or an SHH signal transduction pathway agonist.

The TGFβ family signal transduction pathway inhibitor refers to a substance that inhibits the TGFβ family signal transduction pathway, i.e., signal transduction pathway transmitted by the Smad family, and specific examples include a TGFβ signal transduction pathway inhibitor, a Nodal/Activin signal transduction pathway inhibitor and a BMP signal transduction pathway inhibitor.

A TGFβ signal transduction pathway inhibitor is not particularly limited as long as it inhibits the signal transduction pathway caused by TGFβ, and may be any of nucleic acid, protein, and low-molecular-weight organic compound. Examples of the inhibitor can include a substance that directly acts on TGFβ (e.g., protein, antibody, aptamer etc.), a substance that suppresses expression of a gene encoding TGFβ (e.g., antisense oligonucleotide, siRNA etc.), a substance that inhibits the binding of a TGFβ receptor and TGFβ, and a substance that inhibits a physiological activity caused by signal transduction by TGFβ receptor (e.g., TGFβ receptor inhibitor, Smad inhibitor etc.). A protein known as a TGFβ signal transduction pathway inhibitor, Lefty and the like can be mentioned. As a TGFβ signal transduction pathway inhibitor, a compound well known to those of ordinary skill in the art can be used and, specifically, SB431542 (4[4-(1,3-benzodioxol-5-yl)-5-(2-pyridinyl)-1H-imidazol-2-yl]benzamide), LY-364947 (4-[3-(2-pyridinyl)-1H-pyrazol-4-yl]-quinoline), SB-505124 (2-(5-benzo[1,3]dioxol-5-yl-2-tert-butyl-3H-imidazol-4-yl)-6-methylpyridine), A-83-01 (3-(6-methyl-2-pyridinyl)-N-phenyl-4-(4-quinolinyl)-1H-pyrazole-1-carbothioamide) and the like can be mentioned.

The Nodal/Activin signal transduction pathway inhibitor is not particularly limited as long as it inhibits a signal transduction pathway caused by Nodal or Activin, and may be any of nucleic acid, protein, and low-molecular-weight organic compound. Examples of the inhibitor can include a substance that directly acts on Nodal or Activin (e.g., antibody, aptamer etc.), a substance that suppresses expression of a gene encoding Nodal or Activin (e.g., antisense oligonucleotide, siRNA etc.), a substance that inhibits the binding of a Nodal/Activin receptor and Nodal/Activin, and a substance that inhibits a physiological activity caused by signal transduction by Nodal/Activin receptor. As a Nodal/Activin signal transduction pathway inhibitor, a compound well known to those of ordinary skill in the art can be used and, specifically, SB431542, A-83-01 and the like can be mentioned. Also, a protein (Lefty, Cerberus etc.) known as a Nodal/Activin signal transduction pathway inhibitor may be used. A Nodal/Activin signal transduction pathway inhibitor is preferably SB431542, A-83-01 or Lefty.

The BMP signal transduction pathway inhibitor is not particularly limited as long as it inhibits a signal transduction pathway caused by BMP, and those mentioned above can be used. As a BMP signal transduction pathway inhibitor, a compound well known to those of ordinary skill in the art can be used and, specifically, LDN193189, Dorsomorphin and the like can be mentioned. Also, a protein (Chordin, Noggin etc.) known as a BMP signal transduction pathway inhibitor may be used. A BMP signal transduction pathway inhibitor is preferably LDN193189.

A TGFβ family signal transduction pathway inhibitor is preferably Lefty, SB431542, A-83-01 or LDN193189.

Plural kinds of TGFβ family signal transduction pathway inhibitors having different points of action may be used in combination. By combining them, the aggregate quality improving effect is expected to be enhanced. For example, a combination of a TGFβ signal transduction pathway inhibitor and a BMP signal transduction pathway inhibitor, a combination of a TGFβ signal transduction pathway inhibitor and a Nodal/Activin signal transduction pathway inhibitor, a combination of a BMP signal transduction pathway inhibitor and a Nodal/Activin signal transduction pathway inhibitor can be mentioned. Preferably, a TGFβ signal transduction pathway inhibitor is used in combination with a BMP signal transduction pathway inhibitor. A specific preferable combination is, for example, a combination of SB431542 and LDN193189.

As the SHH signal transduction pathway agonist, those mentioned above can be used. The SHH signal transduction pathway agonist is preferably SHH protein (Genbank accession numbers: NM_000193, NP_000184), SAG or PMA.

A TGFβ family signal transduction pathway inhibitor and a SHH signal transduction pathway agonist may be used in combination. As a specific combination, a combination of any TGFβ family signal transduction pathway inhibitor selected from the group consisting of Lefty, SB431542, A-83-01 and LDN193189, and any SHH signal transduction pathway agonist selected from the group consisting of SHH protein, SAG and PMA can be mentioned. When a TGFβ family signal transduction pathway inhibitor and a SHH signal transduction pathway agonist are used in combination, cells may be cultured in a medium containing both a TGFβ family signal transduction pathway inhibitor and a SHH signal transduction pathway agonist, or cells may be treated with either of a TGFβ family signal transduction pathway inhibitor and a SHH signal transduction pathway agonist, and continuously treated with either or both of them.

The concentrations of the TGFβ family signal transduction pathway inhibitor and the SHH signal transduction pathway agonist can be appropriately determined to fall within a range capable of affording the aforementioned effects. For example, SB431542 is generally used at a concentration of 0.1 µM - 200 µM, preferably 2 µM - 50 µM. A-83-01 is generally used at a concentration of 0.05 µM - 50 µM, preferably 0.5 µM - 5 µM. LDN193189 is generally used at a concentration of 1 nM - 2000 nM, preferably 10 nM - 300 nM. Lefty is generally used at a concentration of 5 ng/mL - 200 ng/mL, preferably 10 ng/mL - 50 ng/mL. SHH protein is generally used at a concentration of 20 ng/mL - 1000 ng/mL, preferably 50 ng/mL - 300 ng/mL. SAG is generally used at a concentration of 1 nM - 2000 nM, preferably 10 nM - 700 nM, more preferably 30 - 600 nM. PMA is generally used at a concentration of 0.002 - 20 µM, preferably 0.02 µM - 2 µM. In one embodiment, a TGFβ family signal transduction pathway inhibitor can be appropriately used in an amount conferring TGFβ family signal transduction pathway inhibiting activity equivalent to that of SB431542 at the aforementioned concentration. In one embodiment, an SHH signal transduction pathway agonist can be appropriately used at a concentration providing SHH signal transduction pathway activating action equivalent to that of SAG at the aforementioned concentration.

A medium to be used in the first step may be a serum-containing medium or a serum-free medium. To avoid contamination with a chemically-undefined component, it is preferably a serum-free medium.

To avoid contamination with a chemically-undefined component, a medium to be used for the first step may be a medium whose components are chemically-defined.

In the first step, the pluripotent stem cells may be cultured under any conditions of suspension culture and adhesion culture, preferably adhesion culture.

For culturing pluripotent stem cells under feeder-free conditions in the first step, an appropriate matrix may be used as a scaffold to provide a scaffold in stead of the feeder cells to the pluripotent stem cell. The pluripotent stem cells are subjected to adhesion culture in a cell container whose surface is coated with a matrix as a scaffold.

As a matrix available as a scaffold, laminin (Nat Biotechnol 28, 611-615 (2010)), laminin fragment (Nat Commun 3, 1236 (2012)), basement membrane preparation (Nat Biotechnol 19, 971-974 (2001)), gelatin, collagen, heparan sulfate proteoglycan, entactin, vitronectin and the like can be mentioned.

Preferably, in the culturing of pluripotent stem cells under feeder-free conditions in the first step, the pluripotent stem cells are cultured in an adhered state in a cell container with surface coated with isolated laminin 511 or E8 fragment of laminin 511 (more preferably, E8 fragment of laminin 511).

While the period for the culturing of pluripotent stem cells in the first step is not particularly limited as long as the effect of improving the quality of the aggregate formed in the second step can be achieved, it is generally 0.5 - 144 hr. The period for the culturing of the pluripotent stem cells in the first step is preferably not less than 1 hr, not less than 2 hr, not less than 6 hr, not less than 12 hr, not less than 18 hr, or not less than 24 hr. The period for the culturing of the pluripotent stem cells in the first step is preferably within 96 hr or within 72 hr. In one embodiment, the period for the culturing of pluripotent stem cells in the first step is preferably 2 - 96 hr, more preferably 6 - 48 hr, further preferably 12 - 48 hr, further more preferably 18 - 28 hr (e.g., 24 hr). That is, the first step is started 0.5 - 144 hr (preferably, 18 - 28 hr) before the start of the second step, and the second step is continuously performed on completion of the first step. In a further embodiment, the period for the culturing of pluripotent stem cells in the first step is preferably 18 - 144 hr, 24 - 144 hr, 24 - 96 hr, or 24 - 72 hr. When the cells are treated with either of a TGFβ family signal transduction pathway inhibitor and a SHH signal transduction pathway agonist, and continuously treated with the other, the treatment time of each can be set to fall within the range of the aforementioned period for the culturing.

The culture conditions such as culture temperature, and CO₂ concentration in the first step can be appropriately determined. The culture temperature is, for example, about 30°C to about 40°C, preferably about 37°C. The CO₂ concentration is, for example, about 1% to about 10%, preferably about 5%.

In a preferable embodiment, the cells obtained in the first step maintain a pluripotent-like state, and the pluripotent-like state is maintained throughout the first step. The pluripotent-like state means a state maintaining at least a part of the characteristics unique to pluripotent stem cells and common to pluripotent stem cells, including pluripotency. The pluripotent-like state does not require strict pluripotency. Specifically, the state expressing all or a part of the markers to be an index of pluripotent state is included in the "pluripotent-like state". As the marker of the pluripotent-like state, Oct3/4-positive, alkaline phosphatase-positive and the like can be mentioned. In one embodiment, a cell maintaining the pluripotent-like state is Oct3/4-positive. It is included in a "cell showing a pluripotent-like state" even when the expression level of Nanog is low as compared to ES cell or iPS cell.

In one embodiment, the cells obtained in the first step are stem cells having a potency to differentiate into at least retinal tissue, retinal cell, retinal progenitor cell, or retinal layer-specific neuron.

In a preferable embodiment, human pluripotent stem cells (e.g., iPS cells) are cultured in an adhered state in the absence of feeder cells and in a serum-free medium containing a TGFβ family signal transduction pathway inhibitor and/or an SHH signal transduction pathway agonist, and bFGF.

The above-mentioned adhesion culture is preferably performed in a cell container whose surface is coated with laminin 511 or E8 fragment of laminin 511. The TGFβ family signal transduction pathway inhibitor is preferably a TGFβ signal transduction pathway inhibitor (e.g., SB431542, A-83-01, Lefty), a Nodal/Activin signal transduction pathway inhibitor (e.g., Lefty, SB431542, A-83-01), a BMP signal transduction pathway inhibitor (e.g., LDN193189, Chordin, Noggin), or a combination thereof (e.g., SB431542 and LDN193189). The TGFβ family signal transduction pathway inhibitor is more preferably Lefty, SB431542, A-83-01, or LDN193189, or a combination thereof (e.g., SB431542 and LDN193189). The SHH signal transduction pathway agonist is preferably SHH protein, SAG or Purmorphamine (PMA), more preferably SAG. A TGFβ family signal transduction pathway inhibitor (e.g., Lefty, SB431542, A-83-01, LDN193189) and an SHH signal transduction pathway agonist (e.g., SHH protein, SAG, PMA) may be used in combination. The period for the culturing is 0.5 - 144 hr (preferably, 18 - 144 hr, 24 - 144 hr, 24 - 96 hr, or 24 - 72 hr (e.g., 18 - 28 hr)).

For example, human pluripotent stem cells (e.g., human iPS cells) are subjected to maintenance culture in the absence of feeder cells and in a serum-free medium containing bFGF. The maintenance culture is preferably performed by adhesion culture. The adhesion culture is preferably performed in a cell container whose surface is coated with vitronectin, laminin 511 or E8 fragment of laminin 511. Then, a TGFβ family signal transduction pathway inhibitor and/or an SHH signal transduction pathway agonist are/is added to the culture, and the culturing is continued. The TGFβ family signal transduction pathway inhibitor is preferably a TGFβ signal transduction pathway inhibitor (e.g., SB431542, A-83-01, Lefty), a Nodal/Activin signal transduction pathway inhibitor (e.g., SB431542, A-83-01, Lefty), a BMP signal transduction pathway inhibitor (e.g., LDN193189), or a combination thereof (e.g., SB431542 and LDN193189). The TGFβ family signal transduction pathway inhibitor is more preferably Lefty, SB431542, A-83-01, or LDN193189, or a combination thereof (e.g., SB431542 and LDN193189). The SHH signal transduction pathway agonist is preferably SHH protein, SAG or PMA. A TGFβ family signal transduction pathway inhibitor (e.g., Lefty, SB431542, A-83-01, LDN193189) and an SHH signal transduction pathway agonist (e.g., SHH protein, SAG, PMA) may be used in combination. After the addition, the culturing is continued for 0.5 - 144 hr (preferably, 18 - 144 hr, 24 - 144 hr, 24 - 96 hr, or 24 - 72 hr (e.g., 18 - 28 hr)).

### [Second step]

The second step wherein the cells obtained in the first step are cultured in suspension in a medium to form a cell aggregate is explained.

The medium to be used in the second step may be a serum-containing medium or serum-free medium. To avoid contamination of chemically-undefined components, a serum-free medium is preferably used in the present invention. For example, a serum-free medium free of both a BMP signal transduction pathway agonist and a Wnt signal transduction pathway inhibitor can be used. To avoid complicated preparation, for example, a serum-free medium supplemented with an appropriate amount of a commercially available serum replacement such as KSR and so on (e.g., medium of 1:1 mixture of IMDM and F-12, which is supplemented with 10% KSR, 450 µM 1-monothioglycerol and 1x Chemically Defined Lipid Concentrate, or medium of GMEM supplemented with 5% - 20% KSR, NEAA, pyruvic acid, 2-mercaptoethanol) is preferably used. The amount of KSR to be added to a serum-free medium in the case of human pluripotent stem cells is generally about 1% to about 30%, preferably about 2% to about 20%.

For formation of an aggregate, dispersed cells are first prepared by a dispersing operation of the cells obtained in the first step. The "dispersed cells" obtained by the dispersing operation refers to a state where, for example, not less than 70% of cells are single cells and not more than 30% of cells are clusters of 2 - 50 cells. Preferably, as the dispersed cells, a state where not less than 80% of cells are single cells, and not more than 20% of cells are clusters of 2 - 50 cells can be mentioned. The dispersed cells refer to a state almost free of mutual adhesion (e.g., plane attachment) of cells.

A dispersion operation of the cells obtained in the first step may contain the above-mentioned mechanical dispersion treatment, cell dispersion solution treatment, and cell protecting agent treatment. These treatments may be performed in combination. Preferably, a cell dispersion solution treatment is performed simultaneously with a cell protecting agent treatment and then a mechanical dispersion treatment is performed.

As a cell protecting agent to be used for the cell protecting agent treatment, a FGF signal transduction pathway agonist (e.g., fibroblast growth factor such as bFGF, FGF4, FGF8 and the like), heparin, an IGF signal transduction pathway agonist (e.g., insulin), serum, and serum replacement can be mentioned. As a cell protecting agent for suppressing cell death of pluripotent stem cells (particularly, human pluripotent stem cells) induced by dispersion, a Rho-associated coiled-coil kinase (ROCK) inhibitor or a Myosin inhibitor may be added. To suppress cell death of pluripotent stem cells (particularly, human pluripotent stem cells) induced by dispersion, and protect the cells, a ROCK inhibitor or a Myosin inhibitor may be added from the start of the second step culture. As a ROCK inhibitor, Y-27632, Fasudil (HA1077), H-1152 and the like can be mentioned. As a Myosin inhibitor, Blebbistatin can be mentioned.

As a cell dispersion solution to be used for the cell dispersion treatment, a solution containing any of enzymes such as trypsin, collagenase, hyaluronidase, elastase, pronase, DNase, papain and so on, and a chelating agent such as ethylenediaminetetraacetic acid and so on can be mentioned. A commercially available cell dispersion solution such as TrypLE Select (manufactured by Life Technologies) and TrypLE Express (manufactured by Life Technologies) can also be used.

As a method of mechanical dispersion treatment, a pipetting treatment or scraping by a scraper can be mentioned.

The dispersed cells are suspended in the above-mentioned medium.

Then, a suspension of the dispersed cells is seeded in the above-mentioned culture vessel, and the dispersed cells are cultured under a condition non-adhesive to the culture vessel, whereby plural cells are assembled to form an aggregate. In this case, plural cell aggregates may be simultaneously formed in one culture vessel by seeding the dispersed cells in a comparatively large culture vessel such as a 10 cm dish. However, the size of the aggregates varies in this case. Thus, for example, a given amount of dispersed stem cells are placed in each well of a multiwell plate (U-bottom, V-bottom) such as a 96-well plate, and static culture is performed, whereby the cells are rapidly aggregated to form one aggregate in each well. The aggregates are recovered from plural wells, whereby a population of uniformed aggregates can be obtained (e.g., SFEBq method).

The concentration of the cells in the second step can be appropriately set so that cell aggregates can be more uniformly and efficiently formed. For example, when human cells (e.g., cells obtained from human iPS cell in the first step) are cultured in suspension using a 96-well plate, a liquid prepared to achieve about 1 x 10³ to about 1 x 10⁵ cells, preferably about 3 x 10³ to about 5 x 10⁴ cells, more preferably about 4 x 10³ to about 2 x 10⁴ cells, further preferably about 4 x 10³ to about 1.6 x 10⁴ cells, further more preferably about 8 x 10³ to about 1.2 x 10⁴ cells, per well is added to the wells, and the plate is stood to form aggregates.

The culture conditions such as culture temperature, CO₂ concentration and so on in the second step can be appropriately determined. The culture temperature is, for example, about 30°C to about 40°C, preferably about 37°C. The CO₂ concentration is, for example, about 1% to about 10%, preferably about 5%.

In the second step, when a medium exchange operation is performed, for example, an operation to add a fresh medium without discarding the existing medium (medium addition operation), an operation to discard about a half amount of the existing medium (about 30 - 90%, for example, 40 - 60% of the volume of the existing medium) and add about a half amount of a fresh medium (30 - 90%, for example, about 40 - 60% of the volume of the existing medium) (half-medium exchange operation), and an operation to discard about the whole amount of the existing medium (not less than 90% of the amount of the existing medium) and add about the whole amount of a fresh medium (not less than 90% of the amount of the existing medium) (full-medium exchange operation) can be mentioned.

While the tool used for the medium exchange operation is not particularly limited, for example, pipetter, micropipette, multichannel micropipette, continuous dispenser, and the like can be mentioned. For example, when a 96 well plate is used as a culture vessel, a multi-channel micropipette may be used.

The period for suspension culture necessary for forming a cell aggregate can be determined as appropriate according to the cell to be used, so that the cells can be aggregated uniformly. To form uniformed cell aggregates, it is desirably as short as possible. The steps for the dispersed cells to form cell aggregates can be divided into a step for assembling cells, and a step for forming cell aggregates from the assembled cells. The period from the time point of seeding the dispersed cells (i.e., at the time of the start of suspension culture) to assembling of the cells, for example, in case of human cells (e.g., stem cells obtained from human iPS cells in the first step), the assembled cells are formed preferably within about 24 hr, more preferably within about 12 hr. The period from the time point of seeding the dispersed cells (i.e., at the time of the start of suspension culture) to form an aggregate in case of human pluripotent stem cells (e.g., human iPS cells) is, for example, preferably within about 72 hr, more preferably within about 48 hr. The period for cell aggregate formation can be appropriately adjusted by controlling the tools for aggregating the cells, centrifugation conditions and so on.

Formation of cell aggregates and uniformity thereof can be determined based on the size and cell number of the aggregates, macroscopic morphology, microscopic morphology by tissue staining analysis and uniformity thereof, expression of differentiation- and undifferentiation-markers and uniformity thereof, control of expression of differentiation marker and synchrony thereof, reproducibility of differentiation efficiency between the aggregates, and so on.

After aggregate formation, the aggregate may be continuously cultured as it is. The period of the suspension culture in the second step may generally be continued until a BMP signal transduction pathway agonist is added. Specifically, it is generally continued for 12 hr - 6 days, preferably about 12 hr - 3 days.

As one embodiment of the medium to be used in the second step, a medium containing an SHH signal transduction pathway agonist (see WO 2016/063985), a medium containing a Wnt signal transduction pathway inhibitor, or a medium containing a Wnt signal transduction pathway inhibitor and an SHH signal transduction pathway agonist (see WO 2017/183732) can be mentioned. In the first step, pluripotent stem cells are treated with a TGFβ family signal transduction pathway inhibitor and/or a SHH signal transduction pathway agonist; and in the second step, the cells obtained in the first step are cultured in suspension in a medium (preferably serum-free medium) containing a SHH signal transduction pathway agonist and/or Wnt signal transduction pathway inhibitor to form aggregates, which results in further quality improvement of the aggregate and enhancement of its differentiation potency into retinal tissue. Using this high quality aggregate, an aggregate containing retinal progenitor cell or neural retinal progenitor cell can be induced with high efficiency.

As the SHH signal transduction pathway agonist, those mentioned above can be used. Preferably, the SHH signal transduction pathway agonist is SHH protein, SAG or PMA. The concentration of the SHH signal transduction pathway agonist in the medium can be appropriately determined to fall within a range capable of achieving the aforementioned effects. SAG is generally used at a concentration of 1 nM - 2000 nM, preferably 10 nM - 700 nM, more preferably 30 nM - 600 nM. PMA is generally used at a concentration of 0.002 µM - 20 µM, preferably 0.02 µM - 2 µM. SHH protein is generally used at a concentration of 20 ng/ml - 1000 ng/ml, preferably 50 ng/ml - 300 ng/ml. When a SHH signal transduction pathway agonist other than SAG, PMA, and SHH protein is used, it is desirably used at a concentration affording an SHH signal transduction pathway activating action which is equivalent to the above-mentioned concentration of SAG.

The concentration of the SHH signal transduction pathway agonist in the medium may be varied during the period of the second step. For example, the SHH signal transduction pathway agonist is provided to fall within the above-mentioned range of a concentration at the time of the start of the second step, and the concentration may be gradually or stepwisely decreased at a ratio of 40 - 60% per 2 - 4 days.

The timing of addition of a SHH signal transduction pathway agonist to the medium is not particularly limited as long as the above-mentioned effects can be afforded, but a higher effect can be obtained when it is added earlier. A SHH signal transduction pathway agonist is added to the medium generally within 6 days, preferably within 3 days, more preferably within 1 day, from the start of the second step, and further preferably at the time of the start of the second step.

The Wnt signal transduction pathway inhibitor is not particularly limited as long as it can suppress signal transduction mediated by Wnt. For example, a substance that directly acts on Wnt or Wnt receptor (anti-Wnt neutralizing antibody, anti-Wnt receptor neutralizing antibody etc.), a substance that suppresses expression of gene encoding Wnt or Wnt receptor (e.g., antisense oligonucleotide, siRNA etc.), a substance that inhibits binding of Wnt receptor and Wnt (soluble Wnt receptor, dominant-negative Wnt receptor etc., Wnt antagonist, Dkk1, Cerberus protein etc.), a substance that inhibits physiological activity caused by signal transduction by Wnt receptor [low-molecular-weight compounds such as CKI-7 (N-(2-aminoethyl)-5-chloroisoquinoline-8-sulfonamide), D4476 (4-[4-(2,3-dihydro-1,4-benzodioxin-6-yl)-5-(2-pyridinyl)-1H-imidazol-2-yl]benzamide), IWR-1-endo (IWR1e) (4-[(3aR,4S,7R,7aS)-1,3,3a,4,7,7a-hexahydro-1,3-dioxo-4,7-methano-2H-isoindol-2-yl]-N-8-quinolinyl-benzamide), and IWP-2 (N-(6-methyl-2-benzothiazolyl)-2-[(3,4,6,7-tetrahydro-4-oxo-3-phenylthieno[3,2-d]pyrimidin-2-yl)thio]acetamide) and the like, and the like] and the like can be mentioned. As a preferred Wnt signal transduction pathway inhibitor, IWR1e is used.

The concentration of the Wnt signal transduction pathway inhibitor in the medium can be appropriately determined to fall within a range capable of achieving the aforementioned effects. IWR1e is added to a medium such that the concentration is about 0.1 µM to about 100 µM, preferably about 0.3 µM to about 30 µM, more preferably about 1 µM to about 10 µM, further preferably about 3 µM. When a Wnt signal transduction pathway inhibitor other than IWR-1-endo is used, it is desirably used at a concentration exhibiting a Wnt signal transduction pathway inhibiting activity equivalent to that of IWR-1-endo at the above-mentioned concentration.

The concentration of the Wnt signal transduction pathway inhibitor in the medium may be varied during the period of the second step. For example, the Wnt signal transduction pathway inhibitor is provided to fall within the above-mentioned range of a concentration at the time of the start of the second step, and the concentration may be gradually or stepwisely decreased at a ratio of 40 - 60% per 2 - 4 days.

The timing of addition of a Wnt signal transduction pathway inhibitor to the medium is not particularly limited as long as the above-mentioned effect can be achieved; however, a high effect is achieved when it is added earlier. Wnt signal transduction pathway inhibitor is added to the medium generally within 6 days, preferably within 3 days, more preferably within 1 day, more preferably within 12 hours, from the start of the suspension culture in the second step, and further preferably at the time of the start of the suspension culture in the second step. Specifically, for example, it is possible to add a basal medium to which a Wnt signal transduction pathway inhibitor is added, or exchange a part or the whole of the medium with the basic medium. While the period during which the cells obtained in the first step are treated with the Wnt signal transduction pathway inhibitor in the second step is not particularly limited as long as the above-mentioned effect can be achieved, preferably, the inhibitor is added to the medium when the suspension culture is started in the second step and acted until the end of the second step. Furthermore, the cells can be continuously exposed to the Wnt signal transduction pathway inhibitor even after completion of the second step (that is, during the period of the third step). In one embodiment, the Wnt signal transduction pathway inhibitor may be allowed to continuously act even after completion of the second step (that is, during the period of the third step) until a neuroepithelial tissue and/or a neural tissue is/are formed.

In a preferable embodiment, the human cells obtained in the first step (e.g., cells obtained from human iPS cells in the first step) are subjected to suspension culture in a serum-free medium containing a SHH signal transduction pathway agonist (e.g., SAG, PMA, SHH protein) and/or Wnt signal transduction pathway inhibitor (e.g., IWR1e) to form aggregates. A SHH signal transduction pathway agonist is preferably contained in the medium from the time of the start of suspension culture. A ROCK inhibitor (e.g., Y-27632) may also be added to the medium. The period for the culturing is 12 hr - 6 days, preferably 12 hr - 3 days. The aggregates formed are preferably uniformed aggregates.

For example, the human cells obtained in the first step (e.g., cells obtained from human iPS cells in the first step) are recovered, dispersed into single cells or a state close thereto in a serum-free medium containing a SHH signal transduction pathway agonist (e.g., SAG, PMA) and/or Wnt signal transduction pathway inhibitor (e.g., IWR1e), and subjected to suspension culture. The serum-free medium may contain a ROCK inhibitor (e.g., Y-27632). A suspension of human stem cells (e.g., stem cells derived from human iPS cells) is seeded in the above-mentioned culture vessel and the dispersed cells are cultured under conditions where they are non-adhesive to the culture vessel, whereby plural cells are assembled to form an aggregate. The period for the culturing is 12 hr - 6 days (preferably 12 hr - 3 days). The aggregates formed are preferably uniformed aggregates.

By performing the second step in this manner, aggregates of the cells obtained in the first step, or the cells derived therefrom can be formed. The aggregate obtained in the second step have higher quality than the one formed by a treatment without a TGFβ family signal transduction pathway inhibitor and/or a SHH signal transduction pathway agonist is not performed in the first step. To be specific, a population of spherical cell aggregates having a smooth surface and a dense inside, and having a high ratio of uncollapsed aggregates can be obtained. In one embodiment, when aggregates (e.g., not less than 100 aggregates) are randomly selected on day 6 from the start of the second step, the ratio of non-cystic aggregates is, for example, not less than 70%, preferably not less than 80%.

The aggregate obtained in the second step has a potency to differentiate into a retinal tissue.

In one preferable embodiment, in the first step, pluripotent stem cells are treated with a TGFβ signal transduction pathway inhibitor, and in the second step, the cells obtained in the first step are subjected to suspension culture in a medium containing a SHH signal transduction pathway agonist (e.g., SAG, PMA, SHH protein) and/or Wnt signal transduction pathway inhibitor (e.g., IWR1e). Preferably, SB431542 or A-83-01 may be used here as a TGFβ signal transduction pathway inhibitor.

In one preferable embodiment, in the first step, pluripotent stem cells are treated with a BMP signal transduction pathway inhibitor, and in the second step, the cells obtained in the first step are subjected to suspension culture in a medium free of a SHH signal transduction pathway agonist (e.g., SAG, PMA, SHH protein). Preferably, LDN193189 may be used here as a BMP signal transduction pathway inhibitor.

In one preferable embodiment, in the first step, pluripotent stem cells (e.g., human pluripotent stem cell) are treated with a TGFβ family signal transduction pathway inhibitor (e.g., a TGFβ signal transduction pathway inhibitor (e.g., Lefty, SB431542, A-83-01), a Nodal/Activin signal transduction pathway inhibitor (e.g., Lefty, SB431542, A-83-01), a BMP signal transduction pathway inhibitor (e.g., LDN193189), or a combination thereof (e.g., SB431542 and LDN193189) etc.); a SHH signal transduction pathway agonist (e.g., SHH protein, SAG, PMA); or a combination of a TGFβ family signal transduction pathway inhibitor (e.g., Lefty, SB431542, A-83-01, LDN193189) and an SHH signal transduction pathway agonist (e.g., SHH protein, SAG, PMA) and, in the second step, suspension culture of the cells obtained in the first step is performed in a medium containing an SHH signal transduction pathway agonist (e.g., SAG, PMA, SHH protein).

In another embodiment, in the first step, pluripotent stem cells (e.g., human pluripotent stem cells) are treated with a TGFβ family signal transduction pathway inhibitor (e.g., a TGFβ signal transduction pathway inhibitor (e.g., a Lefty, SB431542, A-83-01), a Nodal/Activin signal transduction pathway inhibitor (e.g., Lefty, SB431542, A-83-01), a BMP signal transduction pathway inhibitor (e.g., LDN193189), or a combination thereof (e.g., SB431542 and LDN193189) etc.); a SHH signal transduction pathway agonist (e.g., SHH protein, SAG, PMA); or a combination of a TGFβ family signal transduction pathway inhibitor (e.g., Lefty, SB431542, A-83-01, LDN193189) and an SHH signal transduction pathway agonist (e.g., SHH protein, SAG, PMA) and, in the second step, suspension culture of the cells obtained in the first step is performed in a medium free of a SHH signal transduction pathway agonist (e.g., SAG, PMA, SHH protein).

In any embodiment, the medium in the second step preferably contains a ROCK inhibitor (e.g., Y-27632).

### [Third step]

The aggregate obtained in the second step is cultured in suspension in the presence of a BMP signal transduction pathway agonist, whereby an aggregate containing a retinal progenitor cell or a neural retinal progenitor cell can be obtained. In this step, production can be performed according to the second step of the aforementioned starting material production method 1.

In one embodiment, when the concentration of the SHH signal transduction pathway agonist added to the medium in the second step is comparatively low (e.g., not more than 700 nM for SAG, and a concentration conferring SHH signal transduction pathway activating action equivalent to or lower than that of SAG at the above-mentioned concentration, for other SHH signal transduction pathway agonists), medium exchange is not necessary, and a BMP signal transduction pathway agonist (e.g., BMP4) may be added to the medium used in the second step. On the other hand, when the concentration of the SHH signal transduction pathway agonist is comparatively high (e.g., exceeding 700 nM or not less than 1000 nM for SAG, and a concentration conferring a SHH signal transduction pathway activating action equivalent to that of SAG at the above-mentioned concentration, for other SHH signal transduction pathway agonists), it is desirable to exchange the medium with a fresh medium containing a BMP signal transduction pathway agonist (e.g., BMP4) to suppress an influence of the SHH signal transduction pathway agonist remaining when a BMP signal transduction pathway agonist is added.

In a preferable embodiment, the concentration of a SHH signal transduction pathway agonist in the medium to be used in the third step is, when calculated in terms of SHH signal transduction promoting activity of SAG, not more than 700 nM, preferably not more than 300 nM, more preferably not more than 10 nM, further preferably not more than 0.1 nM. Further preferably, it is free of a SHH signal transduction pathway agonist. The medium "free of a SHH signal transduction pathway agonist" also includes a medium substantially free of a SHH signal transduction pathway agonist, for example, a medium free of a SHH signal transduction pathway agonist at a concentration imparting an adverse influence on selective differentiation into a retinal progenitor cell or a retinal tissue. The medium "free of a SHH signal transduction pathway agonist" also includes a medium substantially not supplemented with a SHH signal transduction pathway agonist, for example, a medium not supplemented with a SHH signal transduction pathway agonist at a concentration imparting an adverse influence on selective differentiation into a retinal progenitor cell or a retinal tissue.

In a preferable embodiment of the production of a retinal tissue in the initial developmental stage, in the first step, human pluripotent stem cells (e.g., human iPS cells) are cultured in an adhered state in the absence of feeder cells in a serum-free medium containing a TGFβ signal transduction pathway inhibitor (e.g., SB431542, A-83-01) and bFGF; in the second step, the cells are cultured in suspension in a serum-free medium containing a SHH signal transduction pathway agonist (e.g., SAG, PMA, SHH protein); and in the third step, the aggregate is cultured in suspension in a serum-free medium containing a BMP signal transduction pathway agonist (e.g., BMP4).

In addition, in a preferable embodiment of the production of a retinal tissue in the initial developmental stage, in the first step, human pluripotent stem cells (e.g., human iPS cells) are cultured in an adhered state in the absence of feeder cells in a serum-free medium containing a BMP signal transduction pathway inhibitor (e.g., LDN193189) and bFGF; in the second step, the cells are cultured in suspension in a serum-free medium free of or containing a SHH signal transduction pathway agonist (e.g., SAG, PMA); and in the third step, the aggregate is cultured in suspension in a serum-free medium containing a BMP signal transduction pathway agonist (e.g., BMP4).

In a preferable embodiment of the production of a retinal tissue in the initial developmental stage, in the first step, human pluripotent stem cells (e.g., human iPS cells) are cultured in an adhered state in the absence of feeder cells in a serum-free medium containing a SHH signal transduction pathway agonist (e.g., SAG, PMA) and bFGF for preferably not less than 1 day and not more than 6 days, further preferably 2 - 4 days, in the second step, the cells are cultured in suspension in a serum-free medium containing a SHH signal transduction pathway agonist (e.g., SAG, PMA), and in the third step, the aggregates are cultured in suspension in a serum-free medium containing a BMP signal transduction pathway agonist (e.g., BMP4).

In a preferable embodiment of the production of a retinal tissue in the initial developmental stage, in the first step, human pluripotent stem cells (e.g., human iPS cells) are cultured in an adhered state in the absence of feeder cells in a serum-free medium containing
a TGFβ family signal transduction pathway inhibitor (e.g., a TGFβ signal transduction pathway inhibitor (e.g., Lefty, SB431542, A-83-01), a Nodal/Activin signal transduction pathway inhibitor (e.g., Lefty, SB431542, A-83-01), a BMP signal transduction pathway inhibitor (e.g., LDN193189), or a combination thereof (e.g., SB431542 and LDN193189) etc.); a SHH signal transduction pathway agonist (e.g., SHH protein, SAG, PMA); or
a combination of a TGFβ family signal transduction pathway inhibitor (e.g., Lefty, SB431542, A-83-01, LDN193189) and a SHH signal transduction pathway agonist (e.g., SHH protein, SAG, PMA); and
bFGF,
in the second step, the cells obtained the first step are cultured in suspension in a serum-free medium containing a SHH signal transduction pathway agonist (e.g., SAG, PMA, SHH protein) to form a cell aggregate, and
in the third step, the aggregate is cultured in suspension in a serum-free medium containing a BMP signal transduction pathway agonist (e.g., BMP4) to give an aggregate containing a retinal progenitor cell or a neural retinal progenitor cell.

### 4-3. Starting material production method 3

As one preferable embodiment of production of retinal tissue in an initial developmental stage, the method described in WO 2016/063986 and containing the following steps can also be mentioned:
(1) a first step of culturing pluripotent stem cells in the absence of feeder cells and in a medium containing a factor for maintaining undifferentiated state,
(2) a second step of culturing the pluripotent stem cells obtained in the first step in suspension in the presence of a SHH signal transduction pathway agonist to form a cell aggregate, and
(3) a third step of culturing the aggregate obtained in the second step in suspension in the presence of a BMP signal transduction pathway agonist to obtain an aggregate containing a retinal progenitor cell or neural retinal progenitor cell.

### [First step]

The first step can be performed according to the method described in WO 2016/063986. That is, in the first step, human pluripotent stem cells, preferably human induced pluripotent stem cells (iPS cells) or human embryonic stem cells (ES cell) are cultured in the absence of feeder cells and in a medium containing a factor for maintaining undifferentiated state. The absence of feeder cells (feeder-free) in the first step means a condition substantially free of feeder cells (e.g., the ratio of the number of feeder cells relative to the total number of cells is not more than 3%). Preferably, the first step is performed under a condition free of feeder cells.

The medium to be used in the first step is not particularly limited as long as it is a medium enabling culturing of pluripotent stem cells to maintain undifferentiated state under feeder-free conditions (feeder-free medium). Preferably, to enable culturing to maintain undifferentiated state, it contains a factor for maintaining undifferentiated state. For example, it is a medium containing a factor for maintaining undifferentiated state, and free of a TGFβ family signal transduction pathway inhibitor and an SHH signal transduction pathway agonist.

As the undifferentiated maintenance factor and feeder-free medium, those described in the aforementioned starting material production method 2 can be mentioned.

While the period for the culturing of pluripotent stem cells in the first step is not particularly limited as long as the effect of improving the quality of the aggregate formed in the second step can be achieved, it is generally 0.5 - 144 hr, preferably 2 - 96 hr, more preferably 6 - 48 hr, further preferably 12 - 48 hr, further more preferably 18 - 28 hr (e.g., 24 hr). That is, the first step is started 0.5 - 144 hr (preferably, 18 - 28 hr) before the start of the second step, and the second step is continuously performed on completion of the first step.

In the first step, the medium may be exchanged as appropriate, and one embodiment specifically includes a method including medium exchange every 1 - 2 days. Here, for example, the medium may be exchanged with a medium free of the cell protecting agent or an agent suppressing cell death such as ROCK inhibitor and the like.

The culture conditions such as culture temperature, CO₂ concentration and so on in the first step can be appropriately determined. The culture temperature is, for example, about 30°C to about 40°C, preferably about 37°C. The CO₂ concentration is, for example, about 1% to about 10%, preferably about 5%.

In one preferable embodiment, human pluripotent stem cells (e.g., human iPS cells) are cultured in an adhered state in the absence of feeder cells and in a serum-free medium containing bFGF. The adhesion culture is preferably performed in a cell container with surface coated with laminin 511, E8 fragment of laminin 511 or vitronectin. The adhesion culture is preferably performed using Essential 8, TeSR medium, mTeSR medium, mTeSR-E8 medium, or StemFit medium, more preferably Essential 8 or StemFit medium, as a feeder-free medium.

### [Second step]

The second step in which the pluripotent stem cells obtained in the first step are cultured in suspension in the presence of a SHH signal transduction pathway agonist to form a cell aggregate of pluripotent stem cells may be performed according to the method described in the second step of the above-mentioned starting material production method 2.

### [Third step]

The third step can be performed according to the second step of the aforementioned starting material production method 1, or the third step of the aforementioned starting material production method 2.

### 4-4. Starting material production method 4

As one preferable embodiment of production of a retinal tissue in an initial developmental stage, the method described in WO 2013/077425 and including the following steps can also be mentioned:
(1) a first step of forming an aggregate of pluripotent stem cells by culturing pluripotent stem cells in suspension in a serum-free medium containing a Wnt signal transduction pathway inhibitor, and
(2) a second step of obtaining an aggregate containing a retinal progenitor cell or a neural retinal progenitor cell by culturing in suspension the aggregate formed in the first step in a serum-free medium containing a basement membrane preparation.

The starting material production method 4 can be performed according to the description of WO 2013/077425 (& US2014/341864).

### [First step]

As the Wnt signal transduction pathway inhibitor, those mentioned above can be mentioned.

The concentration of the Wnt signal transduction pathway inhibitor used here may be any as long as it is a concentration at which an aggregate of pluripotent stem cells can be formed. For example, in the case of general Wnt signal transduction pathway inhibitors such as IWR1e and the like, the concentration is 0.1 µM - 100 µM, preferably 1 µM - 10 µM, more preferably about 3 µM.

A Wnt signal transduction pathway inhibitor may be added to a serum-free medium before the start of the suspension culture, or may be added to a serum-free medium within several days (e.g., within 5 days) after the start of the suspension culture. Preferably, a Wnt signal transduction pathway inhibitor is added to a serum-free medium within 5 days, more preferably 3 days, after the start of the suspension culture, most preferably simultaneously with the start of the suspension culture. The suspension culture is performed until 18 days, more preferably 12 days, after the start of the suspension culture with addition of a Wnt signal transduction pathway inhibitor.

Culture conditions such as culture temperature, CO₂ concentration and the like can be appropriately set. The culture temperature is not particularly limited and it is, for example, about 30°C - about 40°C, preferably about 37°C. The CO₂ concentration is, for example, about 1% - about 10%, preferably about 5%.

The concentration of the pluripotent stem cells can be appropriately determined by those of ordinary skill in the art so that aggregates of pluripotent stem cells can be more uniformly and efficiently formed. The concentration of the pluripotent stem cells during formation of the aggregate is not particularly limited as long as it is a concentration at which a uniform aggregate of stem cells can be formed. For example, when human ES cells are cultured in suspension using a 96-well plate, a liquid prepared to achieve about 1 x 10³ to about 5 x 10⁴ cells, preferably about 3 x 10³ to about 3 x 10⁴ cells, more preferably about 5 x 10³ to about 2 x 10⁴ cells, most preferably about 9 x 10³, per well is added, and the plate is stood to form aggregates.

The period for suspension culture necessary for forming an aggregate can be determined as appropriate according to the pluripotent stem cell to be used as long as the cells can be rapidly aggregated. To form uniformed cell aggregates, it is desirably as short as possible (e.g., SFEBq method). For example, in the case of a human ES cell or human iPS cell, it is desirable to form an aggregate preferably within 24 hr, more preferably within 12 hr. The period for cell aggregate formation can be appropriately adjusted by those of ordinary skill in the art by controlling the tools for aggregating the cells, centrifugation conditions and so on.

Formation of aggregates of pluripotent stem cells can be determined by those of ordinary skill in the art based on the size of the aggregate and the number of cells therein, macroscopic morphology, microscopic morphology by tissue staining analysis and uniformity thereof, expression of differentiation and undifferentiation markers and uniformity thereof, control of expression of differentiation marker and synchrony thereof, reproducibility of differentiation efficiency between aggregates, and so on.

### [Second step]

The second step to obtain an aggregate containing a retinal progenitor cell or a neural retinal progenitor cell by culturing the aggregate formed in the first step in suspension in a serum-free medium containing a basement membrane preparation is explained.

The "basement membrane preparation" refers to one containing basement membrane-constituting components having a function to control cell morphology, differentiation, growth, motility, expression of function and so on which are similar to those of epithelial cell, when intended cells capable of forming a basement membrane are plated thereon and cultured. Here, the "basement membrane constituting components" refers to extracellular matrix molecules in the form of a thin membrane present between epithelial cell layer and interstitial cell layer and so on in animal tissues. A basement membrane preparation can be produced by, for example, removing cells capable of forming a basement membrane, which adhere onto a support via a basement membrane, with a solution capable of dissolving the lipid of the cells, an alkali solution and so on. Examples of a preferable basement membrane preparation include products commercially available as basement membrane component (e.g., Matrigel (hereinafter sometimes referred to as Matrigel)), and extracellular matrix molecules known as basement membrane components (e.g., laminin, type IV collagen, heparan sulfate proteoglycan, entactin and so on).

Matrigel is a basement membrane preparation derived from Engelbreth Holm Swarn (EHS) mouse sarcoma. The main component of Matrigel is laminin, type IV collagen, heparan sulfate proteoglycan, and entactin. In addition to these, TGF-β, fibroblast growth factor (FGF), tissue plasminogen activator, and a growth factor naturally produced by EHS tumor are contained. The "growth factor reduced product" of Matrigel has a lower growth factor concentration than common Matrigel, and the standard concentration thereof is <0.5 ng/ml for EGF, <0.2 ng/ml for NGF, <5 pg/ml for PDGF, 5 ng/ml for IGF1, and 1.7 ng/ml for TGF-β. In starting material production method 4, "growth factor reduced product" is preferably used.

The concentration of the basement membrane preparation added to the serum-free medium in the suspension culture in the second step is not particularly limited as long as the epithelial structure of neural tissue (e.g., retinal tissue) is stably maintained. For example, when Martigel is used, it is preferably a volume of 1/20 to 1/200, more preferably about 1/100, of the culture medium. The basement membrane preparation may already be added to the medium at the start of culturing an aggregate of pluripotent stem cells. Preferably, it is added to the serum-free medium within 5 days, more preferably within 2 days, after the start of suspension culture.

The serum-free medium used in the second step may be the serum-free medium used in the first step as it is or may be that exchanged with a new serum-free medium.

When the serum-free medium used in the first step is directly used in this step, the "basement membrane preparation" may be added to the medium.

The serum-free medium used for the suspension culture in the first step and the second step is not particularly limited as long as it is as mentioned above. However, to avoid complicated preparation, for example, a serum-free medium (GMEM or DMEM, 0.1 mM 2-mercaptoethanol, 0.1 mM non-essential amino acid Mix, 1 mM sodium pyruvate) supplemented with an appropriate amount of commercially available KSR is preferably used as such serum-free medium. The dose of KSR in the serum-free medium is not particularly limited and, for example, it is generally 1 - 20%, preferably 2 - 20%, in the case of a human ES cell.

The culture conditions such as culture temperature, CO₂ concentration and so on in the second step can be appropriately determined. While the culture temperature is not particularly limited, it is, for example, about 30°C to about 40°C, preferably about 37°C. The CO₂ concentration is, for example, about 1% to about 10%, preferably about 5%.

The aggregate obtained in the second step can be used as a retinal tissue in an initial developmental stage. To increase the content of a retinal progenitor cell or neural retinal progenitor cell contained therein, after suspension culture in a serum-free medium containing a basement membrane preparation, the following third step can be performed, and the obtained aggregate can also be used as a retinal tissue in an initial developmental stage:
(3) the third step of culturing the aggregate cultured in the second step in suspension in a serum-containing medium.

The serum-containing medium used in the third step may be the serum-free medium used in the second step for culture to which a serum is directly added or may be exchanged with a new serum-containing medium.

The serum added to the medium in the third step may be, for example, mammalian serum such as bovine serum, calf serum, fetal bovine serum, equine serum, foal serum, fetal equine serum, rabbit serum, baby rabbit serum, fetal rabbit serum, human serum and the like, and the like.

The serum is added on day 7 or later, more preferably on day 9 or later, most preferably on day 12, from the start of suspension culture (i.e., the first step). The serum concentration is 1 - 30%, preferably 3 - 20%, more preferably about 10%.

The serum-containing medium used in the third step is not particularly limited as long as it is as described above. The aforementioned serum-free medium (GMEM or DMEM, 0.1 mM 2-mercaptoethanol, 0.1 mM non-essential amino acid Mix, 1 mM sodium pyruvate) supplemented with serum is preferably used.

In addition, an appropriate amount of a commercially available serum replacement such as KSR and the like may be added to the serum-containing medium and used.

In the third step, the production efficiency of the retinal tissue in an initial developmental stage can be increased by adding an SHH signal transduction pathway agonist in addition to the serum.

The SHH signal transduction pathway agonist is not particularly limited as long as it can enhance signal transduction mediated by SHH, and includes those described above.

The concentration of the SHH signal transduction pathway agonist used in this step is, for example, 0.1 nM - 10 µM, preferably 10 nM - 1 µM, more preferably about 100 nM, in the case of general SHH signal transduction pathway agonists such as SAG and the like.

The thus-obtained aggregate can also be used as a retinal tissue in an initial developmental stage.

In one preferable embodiment of production of a retinal tissue in an initial developmental stage, after performing the aforementioned third step, the following fourth step can be performed, and the obtained optic cup-like structure can also be used as a retinal tissue in an initial developmental stage:
(4) the fourth step of culturing the aggregate cultured in the third step in suspension in a serum-free medium or serum-containing medium containing an SHH signal transduction pathway agonist and a Wnt signal transduction pathway agonist.

The SHH signal transduction pathway agonist is not particularly limited as long as it can enhance signal transduction mediated by SHH, and includes those described above.

The concentration of the SHH signal transduction pathway agonist used here is, for example, 0.1 nM - 10 µM, preferably 10 nM - 1 µM, more preferably about 100 nM, in the case of general SHH signal transduction pathway agonists such as SAG and the like.

The Wnt signal transduction pathway agonist is not particularly limited as long as it can enhance signal transduction mediated by Wnt and includes those described above.

The concentration of the Wnt signal transduction pathway agonist used here is, for example, 0.1 µM - 100 µM, preferably 1 µM - 30 µM, more preferably about 3 µM, in the case of general Wnt signal transduction pathway agonists such as CHIR99021 and the like.

The SHH signal transduction pathway agonist and the Wnt signal transduction pathway agonist are added on day 12 or later and until day 25, preferably on day 15 and up to day 18, after the start of suspension culture (start of the first step). At this time, it is preferable to use a medium that does not contain a Wnt signal transduction pathway inhibitor added in the aggregate formation step.

On day 18 or later from the start of suspension culture, an optic cup-like structure is formed in a protuberance state from the inside of the aggregate. The optic cup-like structure produced by the above-mentioned fourth step can also be used as a retinal tissue in an initial developmental stage which is used as a starting material in the above-mentioned method 2 and the above-mentioned method 3.

The aggregate obtained in the above-mentioned fourth step is subjected to suspension culture in a serum-free medium or serum-containing medium not containing an SHH signal transduction pathway agonist or a Wnt signal transduction pathway agonist for 1 to 20 days, and can also be used as a retinal tissue in an initial developmental stage which is used as a starting material in the methods of the above-mentioned 2. and the above-mentioned 3. Neural tissues other than the retinal tissue may be simultaneously formed by the present starting material production method. These may express a Wnt signal transduction pathway agonist and the like which are dorsalization signal transmitters. For this reason, preferably, to eliminate the influence of the Wnt signal transduction pathway agonist that is an excessive dorsalization signal transmitter and the like, the optic cup-like structure present on the surface of the aggregate can also be physically cut out from the aggregate by using tweezers, scissors, injection needle, razor, one analogous thereto and the like.

### 4-5. Starting material production method 5

The retinal tissue in an initial developmental stage may contain a ciliary marginal zone structure, and a retinal tissue in an initial developmental stage containing a ciliary marginal zone structure can be produced by the method described in WO 2015/087614 (& US2016/376554).

To be specific, a cell aggregate containing a retinal tissue in which Chx10-positive cells are present in a proportion of 20% or more and 100% or less of the retinal tissue [for example, in the production methods of the starting material production methods 1 - 4, a cell aggregate corresponding to about day 9 - 60, preferably day 9 - 40 from the start of suspension culture, further preferably about day 15 - 20, for example, day 18, from the start of suspension culture], and obtained by a step of culturing a cell aggregate in a serum-free medium or serum-containing medium each containing a Wnt signal transduction pathway agonist and a FGF signal transduction pathway inhibitor for only a period before the emergence of a RPE65 gene-expressing cell, or an aggregate containing a ciliary marginal zone-like structure which is obtained by a step of further culturing the obtained "cell aggregate in which a RPE65 gene-expressing cell has not emerged" in a serum-free medium or a serum-containing medium not containing a Wnt signal transduction pathway agonist can also be used as a retinal tissue in an initial developmental stage which is used as a starting material in the methods of the above-mentioned 2 and the above-mentioned 3.

Specifically, for example, an aggregate containing a ciliary marginal zone structure, which is prepared by the following method, is also included in the retinal tissue in an initial developmental stage:
(1) a method for producing an aggregate containing a ciliary marginal zone-like structure, which includes a step of culturing a cell aggregate containing a retinal tissue in which Chx10-positive cells are present in a proportion of 20% or more and 100% or less of the retinal tissue in a serum-free medium or serum-containing medium each containing a Wnt signal transduction pathway agonist and an FGF signal transduction pathway inhibitor for only a period before the emergence of a RPE65 gene-expressing cell, after which culturing the obtained "cell aggregate in which a RPE65 gene-expressing cell has not emerged " in a serum-free medium or serum-containing medium each not containing a Wnt signal transduction pathway agonist.

A "cell aggregate containing a retinal tissue in which Chx10-positive cells are present in a proportion of 20% or more and 100% or less of the retinal tissue" can be obtained by the method described in the aforementioned starting material production methods 1 - 4. That is, the cell aggregate is an aggregate containing a retinal tissue in an initial developmental stage. For example, a retinal tissue in an initial developmental stage, namely, "a cell aggregate containing a retinal tissue in which Chx10-positive cells are present in a proportion of 20% or more and 100% or less of the retinal tissue" can be obtained by culturing for 6 - 15 days in the presence of a BMP signal transduction pathway agonist such as BMP4 and the like in the second step of the starting material production method 1 or the third step of the starting material production method 2 or 3. The above-mentioned "step of culturing a cell aggregate containing a retinal tissue in which Chx10-positive cells are present in a proportion of 20% or more and 100% or less of the retinal tissue in a serum-free medium or serum-containing medium each containing a Wnt signal transduction pathway agonist and an FGF signal transduction pathway inhibitor for only a period before the emergence of a RPE65 gene-expressing cell" is preferably started before not less than 50%, preferably not less than 80%, more preferably not less than 90%, further more preferably not less than 99%, of the cells contained in the retinal tissue can express RPE65 gene by continuously culturing in a serum-free medium or serum-containing medium each containing a Wnt signal transduction pathway agonist and a FGF signal transduction pathway inhibitor (e.g., for not less than 30 days), that is, before the above-mentioned proportion of the cells contained in the retinal tissue can differentiate into retinal pigment epithelium. Specifically, it is started by 40 days, preferably 30 days, more preferably 20 days, after the start of the suspension culture.

The thus-obtained cell aggregate can be used as the "cell aggregate containing a retinal tissue in which Chx10-positive cells are present in a proportion of 20% or more and 100% or less of the aforementioned retinal tissue" in this step.

First, "a cell aggregate containing a retinal tissue in which Chx10-positive cells are present in a proportion of 20% or more and 100% or less of the retinal tissue" is cultured according to the method described in WO 2015/087614 and in a serum-free medium or serum-containing medium each containing a Wnt signal transduction pathway agonist and a FGF signal transduction pathway inhibitor for only a period before the emergence of a RPE65 gene-expressing cell. As a preferable culture here, suspension culture can be mentioned.

As a serum-free medium, a serum-free medium which is a basal medium supplemented with N2 or KSR can be mentioned. More specifically, a serum-free medium which is a DMEM/F-12 medium supplemented with N2 supplement (N2, Invitrogen) can be mentioned. As the serum-containing medium, a serum-containing medium which is a basal medium supplemented with fetal bovine serum can be mentioned.

The culture conditions such as culture temperature, CO₂ concentration can be appropriately set. The culture temperature is, for example, in the range of about 30°C to about 40°C, preferably, for example, about 37°C. The CO₂ concentration is, for example, in the range of about 1% to about 10%, preferably, for example, about 5%.

When the above-mentioned cell aggregate is cultured in a serum-free medium or serum-containing medium, the Wnt signal transduction pathway agonist to be contained in the medium is not particularly limited as long as it can enhance signal transduction mediated by Wnt, and those mentioned above can be recited.

The concentration of the Wnt signal transduction pathway agonist to be contained in a serum-free medium or serum-containing medium in the case of a common Wnt signal transduction pathway agonist such as CHIR99021 is, for example, in the range of about 0.1 µM to about 100 µM, preferably, for example, in the range of about 1 µM to about 30 µM, more preferably, for example, about 3 µM.

When the above-mentioned "cell aggregate containing a retinal tissue" is cultured in a serum-free medium or serum-containing medium, the FGF signal transduction pathway inhibitor to be contained in the medium is not particularly limited as long as it can inhibit signal transduction mediated by FGF. Examples of the FGF signal transduction pathway inhibitor include FGF receptor, FGF receptor inhibitor (e.g., SU-5402, AZD4547, BGJ398), MAP kinase cascade inhibiting substance (e.g., MEK inhibitor, MAPK inhibitor, ERK inhibitor), PI3 kinase inhibitor, Akt inhibitor and so on.

The concentration of the FGF signal transduction pathway inhibitor contained in a serum-free medium or serum-containing medium only needs to be a concentration at which differentiation of the cells forming an aggregate of pluripotent stem cells into retinal cells can be induced. For example, in the case of SU-5402, it is added to the medium at a concentration of about 0.1 µM to about 100 µM, preferably about 1 µM to about 30 µM, more preferably about 5 µM.

In the present specification, "culturing for only a period before the emergence of a RPE65 gene-expressing cell" means culturing only in the whole or a part of the period before the emergence of a RPE65 gene-expressing cell. That is, culturing only in the whole or a part of the period (any period) during which the aforementioned "cell aggregate containing a retinal tissue" in the culture system is constituted by cells that do not substantially express RPE65 gene suffices. By employing such culturing, a cell aggregate in which a RPE65 gene-expressing cell has not emerged can be obtained. The "cell aggregate in which a RPE65 gene-expressing cell has not emerged" includes a "cell aggregate in which a RPE65 gene-expressing cell has not emerged at all" and a "cell aggregate in which a RPE65 gene-expressing cell does not substantially emerge". As the "cell aggregate in which a RPE65 gene-expressing cell does not substantially emerge", a cell aggregate in which the proportion of RPE65-positive cells in the retinal tissue contained in the cell aggregate is not more than about 1% can be mentioned.

To determine such particular period, the aforementioned "cell aggregate containing a retinal tissue" is used as a sample, and the presence or absence of expression of RPE65 gene contained in the sample or the level thereof may be measured by a general genetic engineering method or a biochemical method. Specifically, for example, the presence or absence of expression of RPE65 gene or the level thereof can be examined by subjecting a cryosection of the aforementioned "cell aggregate containing a retinal tissue" to an immunostaining method using an antibody against RPE65 protein.

As a "period before the emergence of a RPE65 gene-expressing cell", for example, a period during which the ratio of Chx10-positive cells present in the above-mentioned retinal tissue decreases as compared to that at the time of start of the culturing of the aforementioned cell aggregate in a serum-free medium or serum-containing medium each containing a Wnt signal transduction pathway agonist and a FGF signal transduction pathway inhibitor, and falls within the range of 30% to 0% can be mentioned. As the "cell aggregate in which a RPE65 gene-expressing cell has not emerged", a cell aggregate in which Chx10-positive cells are present in the above-mentioned retinal tissue in a proportion of within 30% to 0% of the tissue can be mentioned.

While the number of days of the "period before the emergence of a RPE65 gene-expressing cell" varies depending on the kind of the Wnt signal transduction pathway agonist and the FGF signal transduction pathway inhibitor, the kind of the serum-free medium or serum-containing medium, other culture conditions and so on, it is, for example, within 14 days. More specifically, when a serum-free medium (e.g., serum-free medium which is a basal medium supplemented with N2) is used, the above-mentioned period is preferably, for example, within 10 days, more preferably, for example, 2 days to 6 days, further specifically 3 days to 5 days. When a serum-containing medium (e.g., serum-containing medium which is a basal medium supplemented with fetal bovine serum) is used, the aforementioned period is preferably, for example, within 12 days, more preferably, for example, 6 days to 9 days.

The thus-obtained aggregate can be used as a retinal tissue in an initial developmental stage which is used as a starting material in the methods used in the above-mentioned 2 and the above-mentioned 3.

Then, the "cell aggregate in which a RPE65 gene-expressing cell has not emerged" obtained by culturing as mentioned above may be further cultured in a serum-free medium or serum-containing medium without containing a Wnt signal transduction pathway agonist for 1 day - 50 days (corresponding to "from initial developmental stage to a stage where emergence rate of cone photoreceptor precursor reaches maximum"), preferably 1 day - 15 days (corresponding to within about 5 days from emergence of ganglion cell), further preferably 1 day - 7 days (corresponding to stage where ganglion cell begins to emerge), and then used as a retinal tissue in an initial developmental stage which is used as a starting material in the methods of the above-mentioned 2 and the above-mentioned 3. As for the culture method, WO 2015/087614 (e.g., paragraph [0076] - [0079]) can be referred to.

### 4-6. Starting material production method 6

A retinal tissue in an initial developmental stage and containing a ciliary marginal zone structure which can be used as a starting material in the production method of the present invention can also be produced by the method described in WO 2013/183774 (&US2015/132787).

Specifically, an aggregate obtained by a step of culturing a cell aggregate containing a retinal tissue, in which Chx10-positive cells are present in a proportion of 20% or more and 100% or less of the retinal tissue, in a serum-free medium or serum-containing medium each containing a Wnt signal transduction pathway agonist for only a period before the emergence of a RPE65 gene-expressing cell, or further, an aggregate containing a ciliary marginal zone-like structure which is obtained by a step of culturing the obtained "cell aggregate in which a RPE65 gene-expressing cell has not emerged" in a serum-free medium or serum-containing medium each free of a Wnt signal transduction pathway agonist is also a retinal tissue in the initial developmental stage.

As the "cell aggregate containing a retinal tissue in which Chx10-positive cells are present in a proportion of 20% or more and 100% or less of the retinal tissue" which can be used as the starting material here, and a "Wnt signal transduction pathway agonist", those used in the above-mentioned starting material production method 5 can be mentioned.

As preferable culture, suspension culture can be mentioned. As a preferable medium, a serum-free medium can be mentioned.

The culture conditions such as culture temperature, CO₂ concentration and the like can be appropriately set. The culture temperature is, for example, in the range of about 30°C to about 40°C, preferably, for example, about 37°C. The CO₂ concentration is, for example, in the range of about 1% to about 10%, preferably, for example, about 5%.

The Wnt signal transduction pathway agonist to be contained in the medium is not particularly limited as long as it can enhance signal transduction mediated by Wnt, and those mentioned above can be recited.

The concentration of the Wnt signal transduction pathway agonist to be contained in a serum-free medium or serum-containing medium in the case of a common Wnt signal transduction pathway agonist such as CHIR99021 is, for example, in the range of about 0.1 µM to 100 µM, preferably, for example, in the range of about 1 µM to 30 µM, more preferably, for example, about 3 µM.

In the same manner as in the production method 5 except that an FGF signal transduction pathway inhibitor may not be contained, the cell aggregate is "cultured for only a period before the emergence of a RPE65 gene-expressing cell".

As a preferable "period before the emergence of a RPE65 gene-expressing cell", for example, a period during which the ratio of Chx10-positive cells present in the above-mentioned retinal tissue is within the range of 50% to 1% can be mentioned. In this case, the "cell aggregate in which a RPE65 gene-expressing cell has not emerged" is a cell aggregate in which Chx10-positive cells are present in the above-mentioned retinal tissue in a proportion of within 50% to 1% of the tissue.

While the number of days of the "period before the emergence of a RPE65 gene-expressing cell" varies depending on the kind of the Wnt signal transduction pathway agonist, the kind of the serum-free medium or serum-containing medium, other culture conditions and so on, it is, for example, within 14 days. More specifically, when a serum-free medium (e.g., serum-free medium which is a basal medium supplemented with N2) is used, the above-mentioned period is preferably, for example, within 10 days, more preferably, for example, 2 days to 6 days, further specifically 3 days to 5 days. When a serum-containing medium (e.g., serum-containing medium which is a basal medium supplemented with fetal bovine serum) is used, the aforementioned period is preferably, for example, within 12 days, more preferably, for example, 6 days to 9 days.

The thus-obtained aggregate can be used as a retinal tissue in an initial developmental stage which is used as a starting material in the methods used in the above-mentioned 2 and the above-mentioned 3. The "cell aggregate in which a RPE65 gene-expressing cell has not emerged" obtained by culturing as mentioned above may be directly used as a retinal tissue in an initial developmental stage. It may be further cultured in a serum-free medium or serum-containing medium without containing a Wnt signal transduction pathway agonist for 1 day - 50 days, preferably 1 day - 15 days, further preferably 1 day - 7 days, and then used as an aggregate containing a retinal tissue in an initial developmental stage. As for the culture method, WO 2015/087614 (e.g., paragraph [0076] - [0079]) can be referred to.

### 4-7. Starting material production method 7

The retinal tissue in an initial developmental stage may contain a ciliary marginal zone structure, and a retinal tissue in an initial developmental stage containing a ciliary marginal zone structure can be produced by the method described in WO 2015/107738 (and US patent application No. 15/112,187). Specifically, for example, a retinosphere prepared by a method containing the following step can also be used as a retinal tissue in an initial developmental stage which is used as a starting material in the method used in the above-mentioned 2 and the above-mentioned 3:
(1) a step of obtaining a retinosphere by proliferation culturing in suspension the cells obtained from a cell aggregate containing a ciliary marginal zone-like structure induced to differentiate from a pluripotent stem cell.

The "cell aggregate containing a ciliary marginal zone-like structure induced to differentiate from a pluripotent stem cell" can be produced according to the above-mentioned starting material production method 5 or 6. The cells obtained therefrom are dispersed and cultured in suspension to give a retinosphere.

Examples of the cells include cells obtained by dispersing the above-mentioned "cell aggregate containing a ciliary marginal zone-like structure induced to differentiate from a pluripotent stem cell", cells obtained by dispersing the ciliary marginal zone-like structure separated from the aforementioned cell aggregate, and cells obtained by dispersing the cells sorted from the aforementioned cell aggregate. When such cells are cultured in suspension at a low density in the presence of a growth factor or the like, a single cell or a spherical cell aggregate derived from a small number of cells of about 2 to 10 cells, namely, retinosphere, is formed. For a method for producing the retinosphere, WO 2015/107738 (and US Patent Application No. 15/112,187) can be referred to.

Specifically, the dispersed cells can be cultured in suspension in a serum-free medium or serum-containing medium supplemented with additive for nerve cell culture and a growth factor. As a medium, preferably, a serum-free medium or serum-containing medium containing one or more substances selected from the group consisting of an FGF signal transduction pathway agonist and an EGF signal transduction pathway agonist can be mentioned. Examples of the FGF signal transduction pathway agonist used here include FGF proteins such as FGF1, bFGF, FGF4, FGF7, FGF8, FGF9 and the like and heparine as an auxiliary agent of FGF signal and the like. Examples of the EGF signal transduction pathway agonist include EGF, TGF-alpha and the like.

The retinosphere produced as mentioned above can be used as a retinal tissue in an initial developmental stage that is the starting material in the production method of the present invention since it contains a retinal progenitor cell or a neural retinal progenitor cell, like retinal tissues. In addition, a retinosphere suspension cultured in a serum-free medium or serum-containing medium containing a BMP signal transduction pathway agonist (e.g., BMP4) after the above-mentioned step (1) can also be used as a retinal tissue in an initial developmental stage to be the starting material of the production method of the present invention. Then, the retinosphere obtained as mentioned above may be further cultured in a serum-free medium or serum-containing medium without containing a Wnt signal transduction pathway agonist for 1 day - 50 days, preferably 1 day - 15 days, further preferably 1 day - 7 days, and then the obtained cell aggregate may be used as a retinal tissue in an initial developmental stage.

### 5. Retinal tissue with high proportion of cone photoreceptor

The present invention provides a retinal tissue containing a photoreceptor precursor rich in cone photoreceptor precursor and/or a photoreceptor rich in cone photoreceptor (hereinafter to be referred to as the retinal tissue with high proportion of cone photoreceptor). That is, the present invention provides a retinal tissue containing a photoreceptor precursor rich in cone photoreceptor precursor and/or a photoreceptor rich in cone photoreceptor wherein the number of cells of the cone photoreceptor precursor and the cone photoreceptor in the photoreceptor precursor and the photoreceptor is not less than 2 times, preferably not less than 4 times, more preferably not less than 5 times, the number of cells of a rod photoreceptor precursor and a rod photoreceptor. In the retinal tissue, specifically, the number of CRX-positive and TRβ2-positive and NRL-negative cells, or CRX-positive and RXR-γ-positive and NRL-negative cells is not less than 2 times, preferably not less than 4 times, more preferably not less than 5 times, the number of cells of CRX-positive and NRL-positive cells, and a ganglion cell is contained.

The retinal tissue may show differentiation induction that has progressed to a level containing a retinal tissue in a differentiation stage where Muller cell emerges.

That is, the retinal tissue obtained by the production method of the above-mentioned 2. is 1) a retinal tissue differentiated to the level showing Muller cell in the retinal tissue, wherein not less than 25%, preferably not less than 30% are cone photoreceptor precursors and cone photoreceptors and not more than 12%, preferably not more than 10%, more preferably not more than 7%, are rod photoreceptor precursors and rod photoreceptors, of the total number of cells contained in the neural retinal tissue, or 2) a retinal tissue that can be the retinal tissue of 1) by further maturation in vitro or in a transplanted body.

In the aforementioned 1), the proportion of the cone photoreceptor precursor and cone photoreceptor contained in all photoreceptor precursor and all photoreceptor in the retinal tissue is preferably not less than 70%, more preferably not less than 80%. Here, the Muller cell can be identified by detecting a well-known marker, for example, CRABP-positive cell, and/or CRALBP-positive cell. In addition, a neural retinal tissue containing a photoreceptor precursor differentiated to the level showing Muller cell, which is obtained by the production method described in the above-mentioned 2., is characterized in that the proportion of PAX6-positive and CHX10-negative cells (ganglion cell, horizontal cell and/or amacrine cell) is lower by not less than 10%, preferably not less than 20%, than when a dorsalization signal transmitter is not acted.

In one embodiment of the present invention, in the retinal tissue obtained by the production method described in the above-mentioned 2., in a neural retinal tissue in a stage where emergence rate of cone photoreceptor precursor reaches maximum, an average of not less than 10%, preferably not less than 13%, more preferably not less than 16%, further more preferably not less than 17%, of the total number of cells are photoreceptor precursors or photoreceptors.

Furthermore, the retinal tissue obtained by the production method described in the above-mentioned 2. has a higher proportion of a cone photoreceptor precursor that can be differentiated into a L cone photoreceptor and/or a M cone photoreceptor as compared to when cultured in the absence of a dorsalization signal transmitter or in the presence of a ventralization signal transmitter.

In the production method described in the above-mentioned 2., the retinal tissue obtained by culturing in the presence of a dorsalization signal transmitter at a concentration that suppresses expression of ALDH1A3 is a retinal tissue similar to macula and the proportion of bipolar cells in the total number of cells contained in a neural retinal tissue is not less than 15% on average (about 1.5 to 2 times that when not acted). "Culturing in the presence of a dorsalization signal transmitter at a concentration that suppresses expression of ALDH1A3" is, for example, a step of culturing for 50 days - 130 days after the start of suspension culture in a medium containing BMP4 at 0.1 nM - 0.15 nM.

The retinal tissue obtained by the production method of the above-mentioned 2. and in a stage before arriving at "retinal tissue in a differentiation stage where Muller cells are observed" is also a retinal tissue that has a high proportion of cone photoreceptor precursor or cone photoreceptor or will have a high proportion of cone photoreceptor due to maturation, and is encompassed in the "retinal tissue with high proportion of cone photoreceptor" of the present invention.

Specifically, a retinal tissue in a stage where emergence rate of cone photoreceptor precursor reaches maximum and having at least one, preferably not less than 3, further preferably not less than 5, further preferably all, characteristics of the following (i) - (xi) can be mentioned:
(i) continuous epithelial rate is not less than 50%, preferably not less than 80%, more preferably not less than 95%,
(ii) an average of not less than 10%, preferably not less than 13%, more preferably not less than 16%, further more preferably not less than 17%, of the total number of cells in the neural retinal tissue are photoreceptor precursor or photoreceptor;
(iii) the proportion of the cone photoreceptor precursor and cone photoreceptor in the total number of cells in the neural retinal tissue is preferably not less than 8%, further more preferably not less than 10%;
(iv) in the neural retinal tissue, the region showing expression of ventral marker (ALDH1A3, COUP-TF-I etc.) is not more than 50%, preferably not more than 20%, more preferably not more than 1%, further more preferably not more than 0.01%;
(v) ALDH1A3 gene expression level of the whole retinal tissue is not more than 50%, preferably not more than 20%, more preferably not more than 5%, as compared to a retinal tissue ventralized by a BMP signal transduction pathway inhibitor, and showing high expression of a ventral marker such as ALDH1A3 and the like;
(vi) a region in which expression of the most dorsal marker is not induced (i.e., region showing no expression of most dorsal marker) in the above-mentioned neural retinal tissue is not less than 50%, preferably not less than 80%, more preferably not less than 90%, further more preferably not less than 99%;
(vii) the expression level of CYP26A1 is not less than 1.2 times, preferably not less than 1.5 times, more preferably not less than 2 times, that of a retinal tissue ventralized by the aforementioned BMP signal transduction pathway inhibitor, and in which expression of CYP26A1 is suppressed;
(viii) the proportion of the number of cells expressing OC2 relative to the total number of cells contained in the neural retinal tissue is about 20% - 70%, preferably about 30% - 70%, more preferably about 50% - 65%, of that when a dorsalization signal transmitter is not added;
(ix) the proportion of the number of cells expressing OC2 relative to the total number of cells contained in the neural retinal tissue is suppressed to about 30% - 60%, preferably about 40% - 50%, of that when the aforementioned BMP signal transduction pathway inhibitor is added;
(x) the proportion of the number of cells expressing OC2 is not more than 25%, preferably not more than 17%, of the total number of cells contained in the neural retinal tissue; and (xi) the OC2 protein expression level of the OC2-positive cell in the neural retinal tissue is an average of about 20% - 70%, preferably about 30% - 70%, more preferably about 30 - 40%, as compared to when a dorsalization signal transmitter is not added or the aforementioned BMP signal transduction pathway inhibitor is added.

In the above, the "continuous epithelial rate" means a ratio of a part having a continuous epithelial structure relative to the surface area of the retinal tissue.

### 6. Retinal tissue with high proportion of rod photoreceptor

The present invention provides a retinal tissue containing a photoreceptor precursor rich in rod photoreceptor precursor and/or a photoreceptor rich in rod photoreceptor (hereinafter to be referred to as the retinal tissue with high proportion of rod photoreceptor). That is, it provides a retinal tissue having a ganglion cell, in which the number of cells of the rod photoreceptor precursor and rod photoreceptor is not less than 40%, preferably not less than 55%, of the number of cells of the photoreceptor precursor and photoreceptor. In other words, in the retinal tissue, the number of cells of the rod photoreceptor precursor and rod photoreceptor is not less than 0.6 times, preferably not less than 1.3 times, the number of cells of the cone photoreceptor precursor and cone photoreceptor. Specifically, the number of CRX-positive and NRL-positive cells is not less than 0.6 times, preferably not less than 1.3 times, the number of CRX-positive and TRβ2-positive and NRL-negative cells or CRX-positive and RXR-γ-positive and NRL-negative cells.

In one embodiment of the retinal tissue, differentiation induction of the retinal tissue may progress to the level of emergence of Muller cell. That is, the retinal tissue obtained by the production method of the above-mentioned 3. is 1) a retinal tissue differentiated to the level showing Muller cell in the retinal tissue, wherein preferably not less than 13%, preferably not less than 15%, more preferably not less than 17% of the total number of cells contained in the neural retinal tissue are rod photoreceptor precursors and rod photoreceptors and not more than 25%, preferably not more than 21%, more preferably not more than 15%, are cone photoreceptor precursors and cone photoreceptors, or 2) a retinal tissue that can be the retinal tissue of 1) by further maturation in vitro or in a transplanted body.

In the aforementioned 1), the proportion of the rod photoreceptor precursor contained in all photoreceptor precursor in the retinal tissue is preferably not less than 40%, more preferably not less than 55%.

The Muller cell can be identified by detecting a well-known marker, for example, CRABP-positive cell, and/or CRALBP-positive cell.

That is, a retinal tissue with a high proportion of rod photoreceptor of the present invention is
1) a retinal tissue differentiated to the level showing Muller cell in the retinal tissue, wherein not more than 25%, preferably not more than 21%, more preferably not more than 15%, of the total number of cells contained in the neural retinal tissue are cone photoreceptor precursors and not less than 13%, preferably not less than 15%, more preferably not less than 17%, are rod photoreceptor precursors, or
2) a retinal tissue that can be the retinal tissue of the aforementioned 1) by further maturation in vitro or in a transplanted body.

The proportion of the rod photoreceptor precursor contained in all photoreceptor precursor in the aforementioned 1) of retinal tissue is preferably not less than 40%, more preferably not less than 55%.

The retinal tissue obtained by the production method of the above-mentioned 3. and in a stage before arriving at "retinal tissue in a differentiation stage where Muller cells are observed" is also a retinal tissue that has a high proportion of rod photoreceptor or may have a high proportion of rod photoreceptor, and is encompassed in the "retinal tissue with high proportion of rod photoreceptor" of the present invention.

Specifically, a retinal tissue in a stage where emergence rate of cone photoreceptor precursor reaches maximum and having at least one, preferably not less than 3, further preferably all, characteristics of the following (i) - (v) can be mentioned:
(i) a continuous epithelial rate is not less than 50%, preferably not less than 80%, more preferably not less than 95%;
(ii) an average of not more than 10%, preferably not more than 7%, more preferably not more than 6%, of the total number of cells in the retinal tissue are photoreceptor precursors and photoreceptors;
(iii) the proportion of cone photoreceptor precursor and cone photoreceptor in the total number of cells in the retinal tissue is not more than 6%, preferably not more than 4%;
(iv) the number of cells showing expression of ventral marker (ALDH1A3, COUP-TF-I etc.) in the retinal tissue is not less than 50%, preferably not less than 70%, more preferably not less than 90%, further more preferably not less than 99%, of the total number of cells; and
(v) ALDH1A3 gene expression level is not less than 2 times, preferably not less than 5 times, more preferably not less than 20 times, that of retinal tissue dorsalized by a BMP signal transduction pathway agonist which is a dorsalization signal transmitter, and showing suppression of ventral marker such as ALDH1A3 and the like.

### 7. Pharmaceutical composition

The present invention provides a pharmaceutical composition containing an effective amount of a retinal tissue containing a high proportion of cone photoreceptors or rod photoreceptors wherein the tissue is produced by the production method of the present invention.

The pharmaceutical composition contains an effective amount of a retinal tissue produced by the production method of the present invention, and a pharmaceutically acceptable carrier.

As a pharmaceutically acceptable carrier, a physiological aqueous solvent (saline, buffer, serum-free medium etc.) can be used. Where necessary, in a transplantation therapy, a medicament containing a tissue or cells to be transplanted may contain conventionally used preservative, stabilizer, reducing agent, isotonizing agent and the like.

The pharmaceutical composition of the present invention can be produced as a suspension by suspending a retinal tissue produced by the production method of the present invention in an appropriate physiological aqueous solvent. Where necessary, the composition may be cryopreserved by adding a cryopreservative, and when in use, thawed and washed with buffer for use of a transplantation therapy.

The retinal tissue obtained by the production method of the present invention can take various forms suitable for medical use. Various shapes such as a sheet shape, a column shape, a lump shape, a plug shape and the like may be used, and the sheet shape is preferable from the viewpoint of superior therapeutic effect, convenience, and the like.

The retinal tissue obtained by the production method of the present invention may be cut in an appropriate size with a tool such as tweezers and the like to prepare a retinal tissue section of administration. In addition, a retinal tissue section cut into a sheet like form can be used as a sheet formulation. When forming a sheet, a suitable sheet or mesh sheet made of a biocompatible polymer, monomer, or gel for the purpose of extending the retinal tissue of the present invention may also be used.

That is, a pharmaceutical composition containing a retinal tissue section cut out from the retinal tissue of the present invention is also within the scope of the present invention.

A retinal cell suspension for administration can be prepared by dispersing a retinal tissue obtained by the production method of the present invention by using a cell dispersant containing protease such as papain and the like. In addition, it is also possible to separate a cell desirable as an active ingredient from the cells contained in the cell suspension by the use of specific antibody, aptamer, peptide, and the like of an antigen protein expressed by the target cell, for purification, and provide a pharmaceutical composition thereof.

That is, a pharmaceutical composition containing a cell suspension prepared by dispersing and/or purifying the retinal tissue of the present invention is also within the scope of the present invention.

### 8. Therapeutic drug and method of treatment

A retinal tissue produced by the production method of the present invention is useful for a transplantation therapy for a disease due to (caused by) a disorder of the retinal tissue, or a disorder of the retinal cell contained therein. Thus, the present invention provides a therapeutic drug for a disease due to a disorder of a retinal tissue, which contains a retinal tissue produced by the production method of the present invention as an active ingredient, and a method of treatment comprising administering the therapeutic drug to a patient. The retinal tissue produced by the production method of the present invention can be used as a therapeutic drug for a disease based on a disorder of a retinal tissue or to supplement a retinal tissue to the corresponding damaged site in a damaged state of the retinal tissue. A disease due to a disorder of a retinal tissue, and a damaged state of a retinal tissue can be treated by transplanting a retinal cell produced by the production method of the present invention to a patient with a disease due to a disorder of a retinal tissue, or in a damaged state of a retinal tissue, who requires transplantation, to supplement the disordered retinal tissue itself. Examples of the disease due to a disorder of a retinal tissue include retinal denaturation, retinitis pigmentosa, age-related macular degeneration, organic mercury poisoning, chloroquine retinopathy, glaucoma, diabetic retinopathy, retinopathy of newborn babies, retinal light disorders such as ultraviolet or blue light retinal injury and the like, and the like.

Among the retinal tissues of the present invention, a retinal tissue containing photoreceptor precursor rich in cone photoreceptor cell precursor is useful as a pharmaceutical composition for transplantation into a region of patients' eyes where cone photoreceptor is abundant. The region where cone photoreceptor is abundant is specifically a region containing a Rod-free zone. The region containing a Rod-free zone includes a region having a macular-like structure, preferably macula. That is, the retinal tissue of the present invention rich in cone photoreceptor precursor and containing photoreceptor is useful as a pharmaceutical composition for transplantation into the macula, preferably more central region (e.g., Fovea) of the macula, of patients. The diseases that require transplantation to the macula include conditions such as age-related macular degeneration, where visual acuity in light place (i.e., visual acuity in the daytime) is reduced, visual field constriction in light place, total blindness, and the like, and the composition can be utilized to improve or treat these.

On the other hand, among the retinal tissues of the present invention, a retinal tissue containing photoreceptor precursor rich in rod photoreceptor precursor is useful as a pharmaceutical composition for transplantation into a region such as the periphery of the macula, which has many rod photoreceptors in the patients' eye, or the outer edge of the region. As a specific transplantation site, the periphery of the macula and the outside thereof can be mentioned. That is, the retinal tissue of the present invention containing cells rich in rod photoreceptor precursor is useful as a pharmaceutical composition for transplantation into the periphery of the macula and the outside thereof and the like of the patients. The diseases that require transplantation to the periphery of the macula and the outside thereof include age-related macular degeneration and the like with low visual acuity in the dark place, night blindness and the like, and the composition can be utilized to improve or treat these. Even when initial manifestation such as low visual acuity in the dark place, night blindness and the like is not found in age-related macular degeneration and the like, the retinal tissue of the present invention can be utilized for treating or preventing denaturation of the retinal tissue when annular scotoma, map scotoma and the like are observed. In addition, by transplantation of a retinal tissue containing photoreceptor precursor rich in rod photoreceptor precursor to the periphery of the macula and the outside thereof, infiltration of a denatured region into the macula can be suppressed (i.e., prevented).

In transplantation therapy, rejection due to the difference in histocompatibility antigens often poses a problem. The problem can be solved by using pluripotent stem cells (e.g., induced pluripotent stem cells) established from the somatic cells of the transplantation recipient. That is, in a preferable embodiment, by using pluripotent stem cells (e.g., induced pluripotent stem cells) established from the somatic cell of the recipient as pluripotent stem cells in the method of present invention, a neural tissue or neural cell which is immunological self to the recipient is produced, and they are transplanted to the recipient (autologous transplantation).

In addition, an allogenic retinal tissue or retinal cell may be produced from pluripotent stem cells (e.g., induced pluripotent stem cells) established from the somatic cell of others who are immunologically compatible with the recipient (e.g., compatible in part or all of HLA type and MHC type), and transplanted to the recipient (allotransplantation).

### 9. Toxicity, efficacy evaluation method

Since a retinal tissue produced by the production method of the present invention is useful as a material for disease study or drug discovery in a screening or toxicity evaluation for a medicament for treating a disease due to a disorder of a retinal tissue, it can be used as a reagent for evaluating toxicity or efficacy of a test substance. For example, iPS cells are established from a human patient with a disease due to a disorder of a retinal tissue, particularly a hereditary disease, and using the iPS cells, the retinal tissue of the present invention is produced by the method of the present invention. The retinal tissue can reproduce the disorder of retinal tissue causing the disease of the patient in vitro. Therefore, the present invention provides a method for evaluating toxicity or efficacy of a test substance, which includes contacting a test substance with a retinal tissue produced by the production method of the present invention and detecting an influence of the substance on the tissue.

For example, a retinal tissue having a particular disorder (e.g., hereditary disorder) which is produced by the production method of the present invention is cultured in the presence or absence (negative control) of a test substance. Then, the severity of disorder of the retinal tissue treated with a test substance is compared with that of the negative control. As a result, a test substance that reduced the severity of the disorder can be selected as a candidate substance for a medicament for treating the disease resulting from the disorder. For example, a test substance that improves the physiological activity (e.g., survival promotion, functional improvement or maturation) of the retinal tissue produced by the production method of the present invention can be searched for as a candidate substance of a pharmaceutical product. Alternatively, an induced pluripotent stem cell is prepared from a somatic cell having a gene mutation that causes a particular disorder such as a disease having a disorder of a retinal tissue and the like, a test substance is added to a retinal progenitor cell or retinal layer-specific neuron produced by differentiation induction of the cell by the production method of the present invention, and a candidate of a test substance effective as a therapeutic drug or prophylactic drug for the disorder can be searched for based on whether they show the disorder as an index.

For toxicity evaluation, the retinal tissue produced by the production method of the present invention is cultured in the presence or absence (negative control) of a test substance. Then, the severity of toxicity on the retinal tissue treated with the test substance is compared with that of the negative control. As a result, a test substance that exerted toxicity as compared to the negative control can be judged as a substance having toxicity to the retinal tissue.

That is, the present invention encompasses a method for evaluating toxicity including the following steps:
(step 1) a step of culturing a retinal tissue produced by the method of the present invention under viable culture conditions for a given time in the presence of a test substance, and measuring the severity of cell injury,
(step 2) a step of culturing a retinal tissue produced by the production method of the present invention under viable culture conditions for a given time in the absence of test substance or in the presence of a positive control, and measuring the severity of cell injury,
(step 3) a step of evaluating the toxicity of the test substance in step 1, based on the difference in the results measured in (step 1) and (step 2).

As used herein, "in the absence of a test substance" encompasses adding only a culture medium or a solvent used to dissolve the test substance instead of adding a test substance. In addition, "positive control" means a known compound having toxicity. Examples of the method for measuring the severity of cell injury include a method for measuring the number of viable cells, for example, a method for measuring intracellular ATP amount, a method for measuring the number of viable cells by cell staining (e.g., nucleus staining, cytotoxic marker) and morphology observation and the like.

In (step 3), as a method for evaluating the toxicity of a test substance, the measurement value in (step 1) and the measurement value of the negative control in (step 2) are compared, and when the severity of cell injury in (step 1) is higher, the test substance can be judged to have toxicity. In addition, the measurement value in (step 1) and the measurement value of the positive control in (step 2) are compared, and when the severity of cell injury in (step 1) is the same or above, the test substance can be judged to have toxicity.

The obtained retinal tissue may be used as it is as a reagent for evaluating toxicity or efficacy. The retinal tissue is subjected to a dispersion treatment (e.g., trypsin/EDTA treatment or papain treatment), and the obtained cells are subjected to a selection using FACS or MACS, whereby highly pure retinal progenitor cells (cone photoreceptor precursor and rod photoreceptor precursor) can also be obtained. In addition, the photoreceptor precursor (S cone photoreceptor precursor, L cone photoreceptor precursor, M cone photoreceptor precursor, or rod photoreceptor precursor) contained in the neural retinal tissue may be differentiated, after final maturation, into photoreceptors (S cone photoreceptor, L cone photoreceptor, M cone photoreceptor, or rod photoreceptor) expressing a visual pigment and used as a reagent for evaluating toxicity and efficacy.

### [Example]

While the present invention is explained in detail by referring to the following Examples, the present invention is not limited thereto.

### Example 1

### (Production Example of cell aggregate containing retinal tissue using human ES cell and method for cutting out retinal tissue)

CRX::Venus knock-in human ES cells (derived from KhES-1; Nakano, T. et al. Cell Stem Cell 2012, 10(6), 771-785) were cultured according to the methods described in "Ueno, M. et al. PNAS 2006, 103(25), 9554-9559" "Watanabe, K. et al. Nat Biotech 2007, 25, 681-686". As a medium for culturing human ES cells, DMEM/F12 medium (Sigma) supplemented with 20% KSR (KnockOut™ Serum Replacement; Invitrogen), 0.1 mM 2-mercaptoethanol, 2 mM L-glutamine, 1x non-essential amino acid, 7.5 ng/mL bFGF was used.

A cell aggregate containing a retinal tissue was prepared by the method described in "Kuwahara et al. Nat Commun 2015, 19(6), 6286-" after modification in part. That is, the aforementioned cultured ES cells were individually dispersed using TrypLE Express (Invitrogen), and the individually dispersed human ES cells were suspended in 100 µL of a serum-free medium in a cell non-adhesive 96 well plate (SUMILON spheroid plate, SUMITOMO BAKELITE CO., LTD.) at 9x10³ cells per well to allow rapid formation of aggregates, after which they were cultured at 37°C, 5% CO₂. As the serum-free medium therefor, a serum-free medium obtained by adding 10% KSR, 450 µM 1-monothioglycerol, 1x Chemically defined lipid concentrate, 5 mg/mL BSA, 20 µM Y27632 to a 1:1 mixture of F-12 medium and IMDM medium was used. At 6 days after the start of suspension culture, BMP4 at a final concentration of 1.5 nM was added and suspension culture was continued. A half amount of the culture medium in the well was exchanged with the above-mentioned medium not supplemented with a BMP signal transduction pathway agonist every 3 or 4 days. A cell aggregate containing a retinal tissue at day 18 from the start of suspension culture was cultured in suspension in a serum-free medium (DMEM/F12 medium supplemented with 1% N2 supplement) containing 3 µM CHIR99021 and 5 µM SU5402 for 4 days, namely, up to day 22 from the start of suspension culture. Thereafter, suspension culture of the cell aggregate containing the retinal tissue was continued until it was appropriately used for analysis and the like. As the culture medium during this period, serum-containing media shown in the following [1] to [3] were used and the cell aggregate was cultured under 5% CO₂ conditions.
[1] day 22 to day 38 from the start of suspension culture; DMEM/F12 medium supplemented with 10% fetal calf serum, 1% N2 supplement, and 100 µM taurine.
[2] day 38 to day 60 from the start of suspension culture; a 1:3 mixed medium of DMEM/F12 medium supplemented with 10% fetal calf serum, 1% N2 supplement, and 100 µM taurine, and Neurobasal medium supplemented with 10% fetal calf serum, 2% B27 supplement, and 100 µM taurine.
[3] after day 60 from the start of suspension culture; Neurobasal medium supplemented with 10% fetal calf serum, 2% B27 supplement, and 100 µM taurine.

The portion of the cell aggregate that is not the retinal tissue can be visually checked and then appropriately excised using tweezers to cut out the retinal tissue from the cell aggregate that is mostly retinal tissue. Fig. 1a, b show an example in which a retinal tissue was actually cut out from a cell aggregate containing a retinal tissue on day 35 from the start of suspension culture. Furthermore, a cell aggregate containing a retinal tissue cultured according to the above-mentioned culture method was observed under a fluorescence microscope (Biorevo BZ-9000, Keyence). As a result, by day 42 after the start of suspension culture, green fluorescence exhibited by knock-in CRX::Venus could be observed in almost all retinal tissues (Fig. 1c, d).

### Example 2

In the method described in Example 1, a dorsalization signal transmitter (BMP4 or Cyclopamine-KAAD), or a ventralization signal transmitter (LDN193189 or SAG) was added as follows to prepare an aggregate containing a retinal tissue rich in cone photoreceptor precursor or rod photoreceptor precursor.
[1] Control group (Fig. 2a, f, k, p); according to the method described in Example 1, culture was performed up to day 70 or day 75 from the start of suspension culture and without addition.
[2] +BMP group (Fig. 2b, g, l, q); 0.15 nM BMP4 was added from day 22 from the start of suspension culture to completion of suspension culture, and cultured up to day 70 or day 75 from the start of suspension culture.
[3] +Cyclopamine-KAAD group (Fig. 2c, h, m, r); 500 nM Cyclopamine-KAAD was added from day 22 from the start of suspension culture to completion of the suspension culture, and cultured up to day 70 or day 75 from the start of suspension culture.
[4] +LDN193189 group (Fig. 2d, i, n, s); 100 nM LDN193189 was added from day 22 from the start of suspension culture to completion of the suspension culture, and cultured up to day 70 or day 75 from the start of suspension culture.
[5] +SAG group (Fig. 2e, j, o, t); 100 nM SAG was added from day 22 from the start of suspension culture to completion of the suspension culture, and cultured up to day 70 or day 75 from the start of suspension culture.

Cell aggregates containing retinal tissue and cultured under the above conditions were observed with a fluorescence microscope (Biorevo BZ-9000, Keyence). After fixing these cell aggregates containing retinal tissue with 4% para-formaldehyde, cryosections were prepared and subjected to immunostaining for CRX, TRβ2 (TRb2), or DAPI staining for staining cell nucleus. As a result, green fluorescence shown by knocked-in CRX::Venus was observed more in the order of +BMP4 group, +Cyclopamine-KAAD group, Control group, +LDN193189 group, +SAG group (Fig. 2a, b, c, d, e). Observation of the immunostained sections with a fluorescence microscope revealed that CRX-positive cells, which are photoreceptor precursors, were clearly higher in the retinal tissue of the +BMP group than in the retinal tissue of the Control group. Slightly more CRX-positive cells were found in the retinal tissue of +Cyclopamine-KAAD group as compared to the retinal tissue of the control group. On the other hand, CRX-positive cells were less in +LDN193189 group, +SAG group than in the retinal tissue of the Control group, and lesser in +SAG group (Fig. 2f, g, h, i, j).

Also, the CRX-positive cells up to day 70 or day 75 from the start of suspension culture contained "cone photoreceptor precursor that emerges in initial stage of development" simultaneously expressing TRβ2. The "cone photoreceptor precursor that emerges in initial stage of development" was observed more in the retinal tissues of +BMP group, +Cyclopamine-KAAD group than in the retinal tissue of the Control group, similar to the photoreceptor precursor. On the other hand, "cone photoreceptor precursor that emerges in initial stage of development" was less in +LDN193189 group, +SAG group as compared to the retinal tissue of the Control group (Fig. 2p, q, r, s, t).

From these, it was found that the dorsalization signal transmitter has an action to increase the photoreceptor precursor in the retinal tissue on before and after day 70 from the start of suspension culture, and "cone photoreceptor precursor that emerges in initial stage of development". Conversely, it was found that the ventralization signal transmitter has an action to decrease the photoreceptor precursor in the retinal tissue on before and after day 70 from the start of suspension culture, or "cone photoreceptor precursor that emerges in initial stage of development".

### Example 3

In the method described in Example 1, a dorsalization signal transmitter (BMP4 or Cyclopamine-KAAD), or a ventralization signal transmitter (LDN193189 or SAG) was added as follows to prepare a retinal tissue in a differentiation stage with maturation to a level showing Muller cell.
[1] Control group (Fig. 3a, f, k, p); according to the method described in Example 1, cultured without addition up to day 191 from the start of suspension culture.
[2] +BMP group (Fig. 3b, g, l, q); 0.15 nM BMP4 was added from day 22 to day 100 from the start of suspension culture and cultured up to day 191 from the start of suspension culture. From day 100 to day 191 from the start of suspension culture, cultured in medium [3] described in Example 1.
[3] +Cyclopamine-KAAD group (Fig. 3c, h, m, r); 500 nM Cyclopamine-KAAD was added from day 22 to day 100 from the start of suspension culture, and cultured up to day 191 from the start of suspension culture. From day 100 to day 191 from the start of suspension culture, cultured in medium [3] described in Example 1.
[4] +LDN193189 group (Fig. 3d, i, n, s); 100 nM LDN193189 was added from day 22 to day 100 from the start of suspension culture, and cultured up to day 191 from the start of suspension culture. From day 100 to day 191 from the start of suspension culture, cultured in medium [3] described in Example 1.
[5] +SAG group (Fig. 3e, j, o, t); 100 nM SAG was added from day 22 to day 100 from the start of suspension culture, and cultured up to day 191 from the start of suspension culture. From day 100 to day 191 from the start of suspension culture, cultured in medium [3] described in Example 1.

After fixing cell aggregates containing retinal tissue cultured under the above conditions with 4% para-formaldehyde, cryosections were prepared and subjected to immunostaining using GFP antibody against fluorescence protein Venus knocked in at the CRX gene locus, antibody against NRL specifically expressed in rod photoreceptor, antibody against RXR-γ (RXRg) specifically expressed in cone photoreceptor among photoreceptors, or DAPI staining for staining the nucleus. The proportion of NRL-positive rod photoreceptor precursor among photoreceptor precursors was reduced in the +BMP4 group as compared to the Control group, and a region where rod photoreceptor precursor was almost absent was observed (Fig. 3g). Conversely, it was found that the proportion of the NRL-positive rod photoreceptor precursor among photoreceptor precursors increased in +LDN193189 group, +SAG group as compared to the Control group (Fig. 3i, j). Among these, the proportion of the RXR-γ-positive cone photoreceptor precursor among photoreceptor precursors was low in +SAG group as compared to the Control group, and the proportion of NRL-positive rod photoreceptor precursor increased in particular (Fig. 3j).

From these, it was found that the BMP signal transduction pathway agonist can increase the proportion of cone photoreceptor precursor among photoreceptor precursors, and a region almost free of rod photoreceptor precursor among photoreceptor precursors can be prepared. In addition, it was found that the +Cyclopamine-KAAD group can increase the proportion of cone photoreceptor precursor among photoreceptor precursors. In addition, it was found that the proportion of rod photoreceptor precursor among photoreceptor precursors can be decreased somewhat. Furthermore, it was found that CRX::Venus-positive photoreceptor precursor can be increased. Conversely, it was found that when a ventralization signal transmitter was added as in +LDN193189 group, +SAG group and the like, the proportion of rod photoreceptor precursor among photoreceptor precursors can be increased, and a retinal tissue with a particularly high proportion of NRL-positive rod photoreceptor precursor can be prepared in +SAG group.

### Example 4

The retinal tissues produced by the method described in Example 2 were fixed with 4% para-formaldehyde, cryosections were prepared, and immunostaining with CRX, TRβ2, or DAPI staining for staining of nucleus was performed. The retinal tissue used was one on day 70-75 from the start of suspension culture similar to Example 2. The number of CRX-positive cells and the numbers of TRβ2-positive cells and DAPI-positive cells in CRX-positive cells were measured using image analysis software (Image J). As a result, about 10.6% were CRX-positive cells in the Control (NUC) group free of addition, and about 17.0%, 14.1%, 6.8%, 5.1% were CRX-positive cells respectively in +BMP4 group, +Cyclopamine-KAAD group, +LDN193189 group, +SAG group. In the Control (NUC) group, about 6.8% were CRX-positive and TRβ2-positive cells, and about 10.8%, 8.1%, 4.5%, 3.2% were CRX-positive and TRβ2-positive cells respectively in +BMP4 group, +Cyclopamine-KAAD group, +LDN193189 group, +SAG group (Fig. 4). From these, it was found that culturing with addition of a dorsalization signal transmitter increases the cone photoreceptor precursor, and culturing with addition of a ventralization signal transmitter decreases the cone photoreceptor precursor.

### Example 5

The retinal tissues produced by the method described in Example 2 were fixed with 4% para-formaldehyde, cryosections were prepared, and immunostaining for OTX2, OC2, TRβ2, or DAPI staining for staining the nucleus was performed. The retinal tissue used was one on day 70-75 from the start of suspension culture similar to Example 2. As a result of measurement using an image analysis software (Image J), the number of OTX2-positive cells was higher in +BMP4 group, +Cyclopamine-KAAD group than in the Control (NUC) group free of addition, and the number of OTX2-positive cells was lower in +LDN193189 group, +SAG group than in the Control group (Fig. 5, image and graph). The number of OC2-positive cells decreased in +BMP4 group, +Cyclopamine-KAAD group as compared to the Control (NUC) group, and increased in +LDN193189 group. To be specific, about 26.4% were OC2-positive cells in the Control (NUC) group, and about 16.0%, 16.7%, 35.1%, 27.3% were OC2-positive cells respectively in +BMP4 group, +Cyclopamine-KAAD group, +LDN193189 group, +SAG group (Fig. 5, image and graph). In addition, as compared to the Control (NUC) group, the expression level (intensity of immunostained fluorescence) of OC2 was about 36% on average in +BMP4 group, about 64% on average in +Cyclopamine-KAAD group, and it was found that the expression level of OC2 decreases in a retinal tissue cultured with addition of a dorsalization signal transmitter was added (Fig. 5, image, graph).

### Example 6

The retinal tissues produced by the method described in Example 3 were fixed with 4% para-formaldehyde, cryosections were prepared, and immunostaining for GFP (fluorescence of GFP variant incorporated in CRX gene locus can be identified. That is, cells similar to CRX-positive cell can be identified), RXR-γ, NRL, or DAPI staining for staining the nucleus was performed. The retinal tissue used was one on day 191 - 192 from the start of suspension culture similar to Example 3. Using image analysis software (Image J), among GFP-positive cells (CRX::Venus-positive cell) and GFP-positive cells, the number of RXR-γ-positive and NRL-negative cone photoreceptor precursors (Cone), the number of NRL-positive rod photoreceptor precursor (Rod-like), and the number of DAPI-positive cells were measured using image analysis software (Image J). As a result, the proportions of cone photoreceptor precursor (Cone)/rod photoreceptor precursor (Rod-like) were respectively about 22.7%/about 12.8% in the Control (NUC) group free of addition, and cone photoreceptor precursor (Cone)/rod photoreceptor precursor (Rod-like) was respectively about 31.3%/about 6.1%, about 30.2%/about 11.5%, about 20.0%/about 14.0%, about 12.6%/about 17.2% in +BMP4 group, +Cyclopamine-KAAD group, +LDN193189 group, +SAG group. The proportion of cone photoreceptor precursor (Cone) increases in +BMP4 group, +Cyclopamine-KAAD group as compared to the Control (NUC) group, whereas the proportion of rod photoreceptor precursor (Rod-like) increases in +LDN193189 group, +SAG group as compared to the Control (NUC) group (Fig. 6).

### Example 7

The retinal tissues produced by the method described in Example 2 were fixed with 4% para-formaldehyde, cryosections were prepared, and immunostaining for GFP (fluorescence of GFP variant incorporated in CRX gene locus can be identified. That is, cells similar to CRX-positive cell can be identified), ALDH1A3, or DAPI staining for staining the nucleus was performed. The retinal tissue used was one on day 75 from the start of suspension culture similar to Example 2. As a result, an ALDH1A3-positive region considered to be a ventral region in the Control (NUC) group was found in the retinal tissue, and in such region, particularly GFP-positive cells, i.e., CRX::Venus-positive cells expressing CRX, were less. In +BMP4 group, overall expression of ALDH1A3 was suppressed as compared to the Control (NUC) group, and CRX::Venus-positive cells increased along therewith. In +LDN193189 group, +SAG group, ALDH1A3-positive region increased than the Control (NUC) group, and CRX::Venus-positive cells also decreased along therewith (Fig. 7, image). From these, it was found that differentiation of CRX-positive or CRX::Venus-positive photoreceptor precursor and cone photoreceptor precursor is suppressed in ALDH1A3-positive region, i.e., ventralized region, and promoted in ALDH1A3-negative region, i.e., relatively dorsalized region.

RNA was extracted from retinal tissue on about day 70-75 from the start of suspension culture and prepared under the same conditions, cDNA was synthesized, and the ALDH1A3 gene expression level was analyzed by quantitative PCR. As a result, the expression level of the ALDH1A3 gene was suppressed in +BMP4 group as compared to the Control (NUC) group, and the expression was promoted in +LDN193189 group, +SAG group (Fig. 7, white bar (d70) in graph), as similarly observed by immunostaining. In addition, RNA was extracted from retinal tissue on about day 40 - 44 from the start of suspension culture and cultured under the same conditions except the culture period, cDNA was synthesized, and the ALDH1A3 gene expression level was analyzed by quantitative PCR. As a result, the expression level scarcely changed on about day 40 from the start of suspension culture as compared to on about day 70 from the start of suspension culture and difference was not found (Fig. 7, black bar (d40) in graph). Thus, it was suggested that such ALDH1A3 gene expression was continuously expressed from about day 40 to day 70 from the start of suspension culture.

### Example 8

The retinal tissue produced by the methods described in Example 2 and Example 7 was fixed with 4% para-formaldehyde, cryosections were prepared, and immunostaining for ALDH1A1 was performed. The retinal tissue used was one on day 75 from the start of suspension culture similar to Example 2 and one on about day 40 from the start of suspension culture and cultured under the same conditions except the culture period as in Example 7. As a result, a clear ALDH1A1-positive region (i.e., dorsal region) was found only in +BMP4 group on day 40 from the start of suspension culture; however, such clear ALDH1A1-positive region was not observed under any conditions on day 75 from the start of suspension culture (Fig. 8-1, image).

The expression level of ALDH1A1 gene was analyzed by quantitative PCR using the same cDNA as that used in Example 7. As a result, similar to that observed by immunostaining, the expression level of ALDH1A1 gene was promoted in +BMP4 group as compared to the Control (NUC) group on about day 40 from the start of suspension culture (Fig. 8-2, black bar (d40) in graph). However, on about day 70 from the start of suspension culture, ALDH1A1 gene expression showed a tendency of decrease under all conditions including +BMP4 group, which correlates to the results of immunostaining (Fig. 8-2, white bar (d70) in graph). Therefore, it was clarified that the gene expression of ALDH1A1 gene decreased under conditions that promote differentiation of photoreceptor precursor and cone photoreceptor precursor, that is, culture with addition of 0.15 nM BMP4, on about day 40 to about day 70 from the start of suspension culture, and expression of ALDH1A1 is not clearly observed on about day 70 from the start of suspension culture.

On the other hand, using cDNA on about day 70 from the start of suspension culture, the expression level of CYP26A1 gene was analyzed. As a result, a pattern similar to the expression level of ALDH1A1 gene was shown, and it was clarified that addition of 0.15 nM BMP4 promotes expression of CYP26A1 gene, and addition of LDN-193189 suppresses expression of CYP26A1 gene (Fig. 8-3).

### Example 9

In the method described in Example 1, a cell aggregate containing a retinal tissue was prepared by adding a dorsalization signal transmitter (BMP4) as described below.
[1] +BMP4 (0.15 nM) group; 0.15 nM BMP4 was added from day 22 from the start of suspension culture to completion of suspension culture, and cultured up to day 70- 75 from the start of suspension culture.
[2] +BMP4 (0.45 nM) group; 0.45 nM BMP4 was added from day 22 from the start of suspension culture to completion of suspension culture, and cultured up to day 70- 75 from the start of suspension culture.
[3] +BMP4 (1.35 nM) group; 1.35 nM BMP4 was added from day 22 from the start of suspension culture to completion of suspension culture, and cultured up to day 70- 75 from the start of suspension culture.

After extracting RNA from retinal tissue prepared in this way, cDNA was synthesized and the expression level of ALDH1A1 gene was analyzed by quantitative PCR. As a result, it was found that CRX::Venus-positive cells became less in a group added with high concentration BMP4 (0.45 nM group and 1.35 nM group) as compared with a group added with 0.15 nM BMP4 (0.15 nM group) (Fig. 9, image, upper panel). In addition, it was found that ALDH1A1 gene expression was significantly increased in the group added with high concentration BMP4 (1.35 nM group) (Fig. 9, graph). Furthermore, a retinal tissue prepared using 1.35 nM BMP4 was fixed with 4% para-formaldehyde, cryosections were prepared, and immunostaining using an antibody against GFP (fluorescence of GFP variant incorporated in CRX gene locus can be identified. That is, cells similar to CRX-positive cell can be identified), ALDH1A1, or DAPI staining for staining the nucleus was performed. As a result, it was found that the number of CRX::Venus was small in a further dorsalized region where the expression of ALDH1A1 was relatively high, and in such region, differentiation of photoreceptor precursor was relatively suppressed similar to the case of ALDH1A3-positive ventral region (Fig. 9, image, lower panel). The brackets in the image show the region where the expression of ALDH1A1 was relatively low and, in the region that is relatively close to the ventral region in the dorsal region as compared to a further dorsalized region, comparatively many CRX::Venus were found. That is, it was found that addition of an excessive concentration of BMP4 rather suppresses differentiation of photoreceptor precursor, and thus it is desirable to induce with BMP4 at a relatively low concentration (e.g., 0.05 nM - 0.25 nM) to promote differentiation of photoreceptor precursor.

Considering the results of Fig. 9 and the results shown in Figs. 8-1, 8-2 and 8-3 together, suppression of ALDH1A3 and induction of CYP26A1 are preferable for promotion of differentiation of photoreceptor precursor up to the stage shown in the Figure. On the other hand, it was found that higher expression of ALDH1A1 than when BMP4 was added may be induced, but dorsalization of the level causing excess expression of ALDH1A1 by high concentration (e.g., 0.45 nM - 1.35 nM) of BMP4 is not preferable, that is, it rather suppresses differentiation of photoreceptor precursor. From these, it was found that, for differentiation of photoreceptor precursor and photoreceptor using BMP4, induction with BMP4 at a comparatively low concentration (e.g., 0.05 nM - 0.25 nM) is desirable, and the concentration of BMP4 can be set using expression of ALDH1A1 as an index such that the expression of ALDH1A1 is not excessive or not strong positive. When dorsalization is induced with BMP4, it was found that, after confirming that the expression of ALDH1A3 is suppressed, an appropriate concentration (e.g., 0.05 nM - 0.25 nM) can be set such that the expression of a dorsal marker other than ALDH1A1 (e.g., CYP26A1) is induced. The photoreceptor precursor or photoreceptor that emerges up to day 70 - day 75 from the start of suspension culture is cone photoreceptor precursor or cone photoreceptor.

### Example 10

By a method similar to the methods described in Example 1 and Example 3, a cell aggregate containing a retinal tissue in a differentiation stage matured to the level that Muller cell was observed was prepared. To the serum-containing medium used was not added a dorsalization signal transmitter (BMP4 or Cyclopamine-KAAD), or a ventralization signal transmitter (LDN193189 or SAG), and culture was performed up to day 270 via a retinal tissue in a differentiation stage matured to the level that Muller cell was observed (e.g., day 190 from the start of culture). After fixing the cell aggregate containing the obtained retinal tissue with 4% para-formaldehyde, frozen sections were prepared, and subjected to immunostaining for S-Opsin (S-OPN), LM-Opsin (LM-OPN), Rhodopsin (RET-P1), Cone arrestin (ARR3), cone photoreceptor specific cyclic nucleotide-gated channel (CNGA3), which are expressed along with further maturation of photoreceptors. As a result, S-opsin expression was observed somewhat, but almost all photoreceptor precursors did not express S-opsin (S-OPN), LM-Opsin (LM-OPN), Rhodopsin (RET-P1), Cone arrestin (ARR3), and it was found that final maturation was not observed unlike in vivo (Fig. 10).

### Example 11

By a method similar to the methods described in Example 1 and Example 3, a cell aggregate containing a retinal tissue in a differentiation stage matured to the level that Muller cell was observed was prepared. To the serum-containing medium used was added a ventralization signal transmitter (LDN193189) as in Example 3, and culture was performed up to about day 190 to give a retinal tissue in a differentiation stage matured to the level that Muller cell was observed. Thereafter, culture was performed up to day 260 - 338 from the start of culture in a medium obtained by adding 1% N2 supplement and 100 µM taurine but not adding 10% fetal calf serum to DMEM/F12 medium containing glutamic acid as a component. After fixing the cell aggregate containing the obtained retinal tissue with 4% para-formaldehyde, a frozen section or a cell aggregate sample containing a retinal tissue for multiangle light sheet fluorescence microscope was prepared, and subjected to immunostaining for S-opsin (S-OPN), Rhodopsin (RET-P1), Cone arrestin (ARR3), Rod Arrestin (SAG), cone photoreceptor specific cyclic nucleotide-gated channel (CNGA3), rod specific cyclic nucleotide-gated channel (CNGA1), cone photoreceptor specific transducin alpha subunit (Gat2), rod photoreceptor specific transducin alpha subunit (Gat1), cone photoreceptor specific Phosphodiesterase 6C (PDE6c), rod photoreceptor specific Phosphodiesterase 6A (PDE6a), which are expressed along with further maturation of photoreceptors (Fig. 11). As a result, it was found that these functional molecules that are expressed along with further maturation were expressed in a cone photoreceptor (Fig. 11, upper left panel) or a rod photoreceptor (Fig. 11, lower left panel), and final maturation was promoted. By observation with a multiangle light sheet fluorescence microscope, it was found that matured photoreceptor was found in the whole retinal tissue (Fig. 11, right).

### Example 12

By a method similar to the methods described in Example 1 and Example 3, a cell aggregate containing a retinal tissue in a differentiation stage matured to the level that Muller cell was observed was prepared. To the serum-containing medium used was added a dorsalization signal transmitter (BMP4) as in Example 3, and culture was performed up to about day 190 to give a retinal tissue in a differentiation stage matured to the level that Muller cell was observed. Thereafter, 0.15 nM BMP4 and 2 - 4 nM T3 were added to a medium obtained by adding 1% N2 supplement and 100 µM taurine but not adding 10% fetal calf serum to DMEM/F12 medium containing glutamic acid as a component, and culture was performed up to day 341 - 344 from the start of culture. After fixing the cell aggregate containing the obtained retinal tissue with 4% para-formaldehyde, a frozen section or a cell aggregate sample containing a retinal tissue for multiangle light sheet fluorescence microscope was prepared, and subjected to immunostaining for LM-opsin (LM-OPN), Cone arrestin (ARR3), cone photoreceptor specific cyclic nucleotide-gated channel (CNGA3), cone photoreceptor specific transducin alpha subunit (Gat2), cone photoreceptor specific Phosphodiesterase 6C (PDE6c), which are expressed along with further maturation of photoreceptors. As a result, it was found that these functional molecules that are expressed along with further maturation were expressed in a cone photoreceptor, and final maturation was promoted (Fig. 12, upper left panel and lower panel). By observation with a multiangle light sheet fluorescence microscope, it was found that the expression of LM-opsin was promoted when BMP4 and T3 were added (Fig. 12, right, DMEM/F12 (without FBS) with BMP4/T3) as compared to when BMP4 and T3 were not added (Fig. 12, center, DMEM/F12 (without FBS)).

### Example 13

By a method similar to the methods described in Example 1 and Example 3, a cell aggregate containing a retinal tissue in a differentiation stage matured to the level that Muller cell was observed was prepared and culture was performed, in the serum-containing medium used, up to about day 190 to give a retinal tissue in a differentiation stage matured to the level that Muller cell was observed. Thereafter, by a method similar to the methods described in Example 11 and 12, further maturation was performed. That is, 0 - 1.35 nM BMP4 and 0 - 8 nM T3 were added to a medium obtained by adding 1% N2 supplement and 100 µM taurine but not adding 10% fetal calf serum to DMEM/F12 medium containing glutamic acid as a component, and culture was performed for not less than 30 days from the start of culture for further maturation. A cell aggregate containing a retinal tissue was recovered, quantitatively PCR was performed according to a conventional method, and the expression levels of S-opsin, M-opsin, L-opsin were confirmed. As a result, irrespective of the presence or absence of BMP4, the expression of S-opsin was promoted when T3 was absent. Thus, it was found that further maturation into S cone photoreceptor, i.e., final maturation, was promoted. On the other hand, when T3 was added, the expression of S-opsin was suppressed, and it was found that T3 acts suppressively on the final maturation into S cone photoreceptor. When BMP4 was added in combination with T3, the expression of L and M-opsin was promoted, and it was found that further maturation into L or M cone photoreceptor, i.e., final maturation, was promoted (Fig. 13).

### [Industrial Applicability]

According to the production method of the present invention, it is possible to produce a retinal tissue rich in cone photoreceptors and provide same as a retinal tissue for transplantation to the macula of the eye of patients with age-related macular degeneration and the like, and to produce a retinal tissue rich in rod photoreceptors and provide same as a retinal tissue for transplantation to the periphery of the macula or the outside thereof of the eye of patients with age-related macular degeneration and the like. In addition, the retinal tissue rich in cone photoreceptors and the retinal tissue rich in rod photoreceptors of the present invention are useful as pharmaceutical compositions.

This application is based on a patent application No. 2017-177187 filed in Japan (filing date: September 14, 2017), the contents of which are incorporated in full herein.

## Claims

1. A method for increasing a proportion of a cone photoreceptor precursor and a cone photoreceptor in a photoreceptor precursor and a photoreceptor comprised in a retinal tissue, the method comprising a step of culturing a retinal tissue in an initial developmental stage to a stage where an emergence rate of a cone photoreceptor precursor reaches maximum in a medium comprising a dorsalization signal transmitter at a concentration sufficient to suppress expression of a ventral marker.

2. The method according to claim 1, wherein the cone photoreceptor precursor and the cone photoreceptor are CRX-positive and RXR-γ-positive, or CRX-positive and TRβ2-positive; and NRL-negative cells.

3. The method according to claim 1 or 2, wherein the ventral marker is ALDH1A3 and/or COUP-TF I.

4. The method according to any one of claims 1 to 3, wherein the concentration of the dorsalization signal transmitter is such that it does not induce expression of a most dorsal marker.

5. The method according to any one of claims 1 to 3, wherein the concentration of the dorsalization signal transmitter is such that it promotes expression of the dorsal marker.

6. The method according to any one of claims 1 to 3, wherein the concentration of the dorsalization signal transmitter is such that it does not induce expression of the most dorsal marker and promotes expression of other dorsal markers.

7. The method according to claim 5 or 6, wherein the dorsal marker is CYP26A1 and/or CYP26C1.

8. The method according to claim 4 or 6, wherein the most dorsal marker is COUP-TF II.

9. The method according to claim 5 or 6, wherein the dorsal marker is ALDH1A1.

10. The method according to claim 9, wherein the concentration of the dorsalization signal transmitter is sufficient to induce expression of not less than 0.1% and not more than 30% of the expression level of ALDH1A1 promoted by 1.35 nM BMP4.

11. The method according to any one of claims 1 to 10, wherein the retinal tissue in an initial developmental stage comprises a ciliary marginal zone-like structure.

12. The method according to any one of claims 1 to 10, wherein the retinal tissue in an initial developmental stage comprises a cell that can differentiate into a photoreceptor and a ganglion cell.

13. The method according to any one of claims 1 to 12, wherein the retinal tissue in an initial developmental stage is derived from a pluripotent stem cell.

14. The method according to any one of claims 1 to 12, wherein the retinal tissue in an initial developmental stage is derived from a neuroepithelial cell obtained from an adult tissue.

15. The method according to any one of claims 1 to 14, wherein the retinal tissue in an initial developmental stage comprises a PAX6-positive and RX-positive cell.

16. The method according to any one of claims 1 to 15, wherein the retinal tissue in an initial developmental stage comprises a PAX6-positive, RX-positive and CHX10-positive cell.

17. The method according to any one of claims 1 to 16, wherein the step of culturing in the presence of a dorsalization signal transmitter is continued for 4 days to 170 days.

18. The method according to claim 17, wherein the step of culturing in the presence of a dorsalization signal transmitter is continued until a period when a rod photoreceptor precursor emerges when cultured in the absence of a dorsalization signal transmitter.

19. The method according to any one of claims 1 to 18, wherein the dorsalization signal transmitter is a BMP signal transduction pathway agonist or a Wnt signal transduction pathway agonist, or a SHH signal transduction pathway inhibitor which is capable of inducing a BMP signal corresponding to 0.01 nM - 0.90 nM of BMP4.

20. The method according to any one of claims 1 to 19, wherein the dorsalization signal transmitter is BMP4.

21. The method according to claim 20, wherein the concentration of BMP4 is 0.05 nM - 0.45 nM.

22. The method according to any one of claims 1 to 19, wherein the dorsalization signal transmitter is Cyclopamine-KAAD.

23. The method according to claim 22, wherein the concentration of Cyclopamine-KAAD is 0.01 µM - 5 µM.

24. The method according to claim 23, wherein the concentration of Cyclopamine-KAAD is 0.2 µM - 1 µM.

25. The method according to any one of claims 1 to 24, wherein the method is performed in a medium free of 9-cisretinoic acid.

26. A retinal tissue comprising a photoreceptor precursor rich in a cone photoreceptor precursor and/or a photoreceptor rich in a cone photoreceptor, wherein the retinal tissue is obtained by the method according to any one of claims 1 to 25.

27. A retinal tissue comprising a photoreceptor precursor rich in a cone photoreceptor precursor and/or a photoreceptor rich in a cone photoreceptor, and a ganglion cell, wherein the number of the cone photoreceptor precursor and cone photoreceptor is not less than 2 times, preferably not less than 4 times, the number of the rod photoreceptor precursor and rod photoreceptor, in the photoreceptor precursor and photoreceptor.

28. The retinal tissue according to claim 26 or 27, wherein the whole photoreceptor precursor and the whole photoreceptor comprises the cone photoreceptor precursor and cone photoreceptor in not less than 70%, preferably not less than 80%.

29. A retinal tissue that is able to mature into the retinal tissue according to claim 26 or 27 by culturing.

30. The retinal tissue according to any one of claims 26 to 29, wherein not less than 50% of the layer structure of the retinal tissue forms a continuous epithelial structure.

31. The retinal tissue according to claim 30, wherein the retinal tissue has a diameter in the major axis direction of not less than 0.6 mm.

32. A pharmaceutical composition for transplantation to a retinal tissue of a retina disease patient in need of transplantation, comprising a retinal tissue section cut out from the retinal tissue according to any one of claims 26 to 31.

33. The pharmaceutical composition according to claim 32, wherein the retinal tissue requiring transplantation is a tissue of a region comprising Rod-free zone.

34. The pharmaceutical composition according to claim 33, wherein the region comprising the Rod-free zone has a macular-like structure.

35. A method for increasing a proportion of a rod photoreceptor precursor and a rod photoreceptor in a photoreceptor precursor and a photoreceptor comprised in a retinal tissue, comprising a step of culturing a retinal tissue, in an initial developmental stage to a stage where an emergence rate of a cone photoreceptor precursor reaches maximum, for at least one day in the presence of a ventralization signal transmitter at a concentration sufficient to promote expression of a ventral marker.

36. The method according to claim 35, wherein the rod photoreceptor precursor and rod photoreceptor are NRL-positive and CRX-positive cells.

37. The method according to claim 35 or 36, wherein the ventral marker is ALDH1A3 and/or COUP-TF I.

38. The method according to any one of claims 35 to 37, wherein the retinal tissue in an initial developmental stage comprises a ciliary marginal zone-like structure.

39. The method according to any one of claims 35 to 38, wherein the retinal tissue in an initial developmental stage comprises a cell that can differentiate into a photoreceptor or a ganglion cell.

40. The method according to any one of claims 35 to 39, wherein the retinal tissue in an initial developmental stage is derived from a pluripotent stem cell.

41. The method according to any one of claims 35 to 39, wherein the retinal tissue in an initial developmental stage is derived from a neuroepithelial cell obtained from an adult tissue.

42. The method according to any one of claims 35 to 41, wherein the retinal tissue in an initial developmental stage comprises a PAX6-positive and RX-positive cell.

43. The method according to claim 42, wherein the retinal tissue in an initial developmental stage comprises a PAX6-positive, RX-positive and CHX10-positive cell.

44. The method according to any one of claims 35 to 43, wherein the step of culturing in the presence of a ventralization signal transmitter is continued for 4 days to 170 days.

45. The method according to claim 44, wherein the step of culturing in the presence of a ventralization signal transmitter is continued until a period when a rod photoreceptor precursor emerges.

46. The method according to any one of claims 35 to 45, wherein the ventralization signal transmitter is a substance having an SHH signal transduction pathway promoting activity corresponding to 1 nM - 10 µM SAG, or a substance having a BMP signal transduction pathway inhibitory activity corresponding to 0.1 nM - 20 µM LDN193189.

47. The method according to claim 46, wherein the ventralization signal transmitter is SAG.

48. The method according to claim 47, wherein the concentration of SAG is 1 nM - 10 µM.

49. The method according to claim 48, wherein the concentration of SAG is 10 nM - 500 nM.

50. The method according to claim 46, wherein the ventralization signal transmitter is LDN193189.

51. The method according to claim 50, wherein the concentration of LDN193189 is 0.1 nM - 20 µM.

52. The method according to claim 51, wherein the concentration of LDN193189 is 30 nM - 1 µM.

53. The method according to any one of claims 35 to 52, wherein the method is performed in a medium free of 9-cisretinoic acid.

54. A retinal tissue comprising a photoreceptor precursor rich in a rod photoreceptor precursor and/or a photoreceptor rich in a rod photoreceptor, wherein the retinal tissue is obtained by the method according to any one of claims 35 to 53.

55. A retinal tissue comprising a photoreceptor precursor rich in a rod photoreceptor precursor and/or a photoreceptor rich in a rod photoreceptor, and a ganglion cell, wherein not less than 40%, preferably not less than 55%, of the number of the cells of the photoreceptor precursor and photoreceptor are rod photoreceptor precursors and rod photoreceptors.

56. A retinal tissue that is able to mature into the retinal tissue according to claim 55 by culturing.

57. The retinal tissue according to any one of claims 54 to 56, wherein not less than 50% of the layer structure of the retinal tissue forms a continuous epithelial structure.

58. The retinal tissue according to claim 57, wherein the retinal tissue has a diameter in the major axis direction of not less than 0.6 mm.

59. A pharmaceutical composition for transplantation to a retinal tissue of a retina disease patient in need of transplantation, comprising a retinal tissue section cut out from the retinal tissue according to any one of claims 54 to 58.

60. The pharmaceutical composition according to claim 59, wherein the retinal tissue requiring transplantation is a region including the periphery of the macula and the outside thereof having a high proportion of rod photoreceptor precursor (Rod precursor) and/or rod photoreceptor.

61. A method for treating a disease based on a disorder of a retinal cell or retinal tissue, comprising transplanting an effective amount of the retinal tissue according to any one of claims 26 to 31, or any according to claim 54 to 58, to a subject in need of transplantation.

62. Use of the retinal tissue according to any one of claims 26 to 31, or any according to claim 54 to 58, as a reagent for evaluating toxicity or efficacy.

63. A method for producing a completely matured retinal tissue that expresses S-opsin, L-opsin and/or M-opsin, comprising a step of culturing a retinal tissue comprising a cone photoreceptor precursor and a cone photoreceptor in a serum-free medium.

64. The method according to claim 63, wherein the retinal tissue comprising the cone photoreceptor precursor and cone photoreceptor is the retinal tissue according to any one of claims 26 to 31.

65. The method according to claim 63 or 64, wherein the serum-free medium is a medium comprising a dorsalization signal transmitter.

66. The method according to claim 65, wherein the dorsalization signal transmitter is BMP.

67. The method according to any one of claims 63 to 66, wherein the serum-free medium further comprises a thyroid gland hormone signal transmitter.
